# EUROPEAN PATENT APPLICATION

(11) **EP 4 487 807 A1**
(43) Date of publication of application: **08.01.2025**
(21) Application number: 23760446.7
(22) Date of filing: 28.02.2023
(51) Int. Cl.: A61B 34/30, A61B 34/00, A61B 17/29, A61B 17/00

(54) **SURGICAL INSTRUMENT AND SURGICAL ROBOT COMPRISING SAME**

(30) Priority: 28.02.2022 KR 20220026415
(71) Applicant: Livsmed Inc., Seongnam-si, Gyeonggi-do 13516 (KR)
(72) Inventor: SONG, Young Jae, Seongnam-si, Gyeonggi-do 13516 (KR); LEE, Jung Joo, Seongnam-si, Gyeonggi-do 13516 (KR); KIM, Hee Jin, Seongnam-si, Gyeonggi-do 13516 (KR); JANG, Dong Kyu, Seongnam-si, Gyeonggi-do 13516 (KR)
(74) Representative: AWA Sweden AB
(86) International application number: PCT/KR2023/002818
(87) International publication number: WO 2023/163572

(57) **Abstract**

The present disclosure relates to a surgical instrument, and more particularly, to a surgical instrument that is mountable on a robotic arm or manually operable for use in laparoscopic surgery or various other surgeries.

## Description

### Technical Field

The present disclosure relates to a surgical instrument and a surgical robot including the same, and more particularly, to a surgical instrument that is mountable on a robotic arm or manually operable for use in laparoscopic surgery or various other surgeries, and a surgical robot including the surgical instrument.

### BACKGROUND ART

Medically, surgery refers to the treatment of diseases by cutting, slitting, or manipulating the skin, mucous membranes, or other tissues using medical devices In particular, open surgery in which the skin of the surgical site is incised and opened to treat, shape, remove organs or the like therein and the like cause problems such as bleeding, side effects, patient pain, scarring. Accordingly, recently, surgery performed by inserting only a medical device, for example, laparoscopic surgical instrument, microsurgical microscope, and the like by forming a predetermined hole in the skin or surgery using a robot has been spotlighted as an alternative.

Here, a surgical robot refers to a robot that has a function of replacing a surgical action performed by a surgeon. Advantageously, the surgical robot may operate more accurately and precisely as compared with a human and enable remote operation.

Surgical robots that are currently being developed worldwide may include a bone surgical robot, a laparoscopic surgical robot, a stereotactic surgical robot, and the like. Here, the laparoscopic surgical robot is a robot that performs minimum invasive surgery using a laparoscope and small surgical instruments.

Laparoscopic surgery is a cutting-edge surgery technique that involves perforating one or more small holes in the abdomen and inserting a laparoscope, which is an endoscope for looking inside the abdomen to perform the surgery, and is a field that is expected to advance in the future. Today's laparoscopes are mounted with computer chips and have been developed to the extent that magnified images, which are clearer than images seen with the naked eye, can be obtained and when used with specially-designed laparoscopic surgical tools while looking at a monitor screen, any type of surgery is possible.

Moreover, laparoscopic surgery offers the same range of surgical procedures as open surgery, but with several advantages including fewer complications, the ability to initiate treatment shortly after the procedure, and the capability to maintain the patient's stamina and immune functions. As a result, laparoscopic surgery is becoming increasingly recognized as the standard surgery for treating colorectal cancer or the like in places such as the United States and Europe.

Meanwhile, a surgical robot is generally composed of a master robot and a surgical robot. When a surgical operator manipulates a control lever (e.g., a handle) equipped on the master robot, a surgical tool coupled to or held by a robot arm on the surgical robot may be manipulated to perform surgery.

### DESCRIPTION OF EMBODIMENTS

### TECHNICAL PROBLEM

The present disclosure is directed to providing a multi-joint type surgical device capable of being mounted on a robot arm or operated manually for use in laparoscopic surgery or various surgeries, the multi-joint type surgical device capable of independently and smoothly performing a pitch motion and a yaw motion/actuation motion by compensating for jaw wire movement that occurs during the pitch motion.

### TECHNICAL SOLUTION TO PROBLEM

One aspect of the present disclosure provides a surgical instrument comprising: an end tool comprising one or more jaws and an end tool jaw pulley, which is coupled to the jaw, and formed to be rotatable together with the one or more jaws around a first shaft, the end tool being formed to be able to perform at least pitch rotation and yaw rotation; a jaw wire that is coupled to the end tool jaw pulley and moves according to rotation of the end tool jaw pulley; a connection part extending in one direction, through which the jaw wire passes, and having one end to which the end tool is coupled; a driving part that is coupled to another end of the connection part and is configured to control the pitch rotation and the yaw rotation of the end tool, wherein the driving part comprises: a driving part jaw pulley that is formed to be rotatable around a second shaft, and is formed to wind at least a portion of the jaw wire; and a driving part jaw rotation shaft that is formed to be rotatable around a third shaft different from the second shaft, is relatively movable while maintaining a preset distance from a center of the driving part jaw pulley, revolves around the center of the driving part jaw pulley, and is coupled to the jaw wire, the jaw wire moves through rotation of the driving part jaw rotation shaft to cause the end tool jaw pulley and the jaw to rotate, two strands of the jaw wire, which emerge while being wound around the driving part jaw pulley, extend toward the end tool after being sequentially wound around the driving part jaw pulley, the driving part jaw rotation shaft, and the driving part jaw pulley, and when the driving part jaw rotation shaft moves relative to the driving part jaw pulley, the end tool performs the pitch rotation while an overall length of the jaw wire within the driving part is changed.

In an embodiment of the present disclosure, a surgical instrument further comprises a driving part pitch pulley arranged adjacent to the driving part jaw pulley and formed to be rotatable around the second shaft, wherein the driving part jaw rotation shaft is formed to be movable relative to the driving part pitch pulley, such that, when the driving part pitch pulley rotates, a relative position of the driving part jaw rotation shaft with respect to the second shaft is changed.

In an embodiment of the present disclosure, wherein a relative distance between a rotation center of the driving part pitch pulley and a rotation center of the driving part jaw rotation shaft is kept constant.

In an embodiment of the present disclosure, wherein, when the driving part pitch pulley rotates, the driving part jaw rotation shaft moves in conjunction with the driving part pitch pulley.

In an embodiment of the present disclosure, wherein, when the driving part pitch pulley rotates around the second shaft, the driving part jaw rotation shaft moves relative to the driving part pitch pulley such that the overall length of the jaw wire within the driving part is changed.

In an embodiment of the present disclosure, wherein, as the overall length of the jaw wire within the driving part is changed due to rotation of the driving part pitch pulley, an overall length of the jaw wire within the end tool is also changed.

In an embodiment of the present disclosure, wherein, even when the overall length of the jaw wire within the driving part is changed due to rotation of the driving part pitch pulley, an overall length of the jaw wire is kept constant.

In an embodiment of the present disclosure, a surgical instrument further comprises an end tool jaw pitch main pulley formed adjacent to the end tool jaw pulley and formed to be rotatable around a fourth shaft different from the first shaft, and an end tool jaw pitch sub-pulley formed adjacent to the end tool jaw pitch main pulley and formed to be rotatable around a fifth shaft different from the first shaft.

In an embodiment of the present disclosure, wherein, during the pitch rotation of the end tool, the two strands of the jaw wire, which emerge while being wound around the end tool jaw pulley and pass through the end tool jaw pitch main pulley and the end tool jaw pitch sub-pulley, simultaneously move in the same direction.

In an embodiment of the present disclosure, wherein, with respect to one plane perpendicular to the first shaft and comprising the fourth shaft, the two strands of the jaw wire, which emerge while being wound around the end tool jaw pulley, are arranged on the same side with respect to the one plane.

In an embodiment of the present disclosure, wherein the jaw comprises a first jaw and a second jaw, the end tool jaw pulley comprises an end tool first jaw pulley coupled to the first jaw, and an end tool second jaw pulley coupled to the second jaw, and
the jaw wire comprises a first jaw wire coupled to the end tool first jaw pulley and a second jaw wire coupled to the end tool second jaw pulley.

In an embodiment of the present disclosure, wherein, with respect to a plane perpendicular to the first shaft and comprising the fourth shaft, two strands of the first jaw wire, which emerge while being wound around the end tool first jaw pulley, are arranged on one side with respect to the plane, and two strands of the second jaw wire, which emerge while being wound around the end tool second jaw pulley, are arranged on another side with respect to the plane.

In an embodiment of the present disclosure, wherein the jaw wire is formed to be sequentially in contact with the end tool jaw pulley, the end tool jaw pitch main pulley, and the end tool jaw pitch sub-pulley.

In an embodiment of the present disclosure, the surgical instrument further comprises an end tool pitch pulley arranged adjacent to the end tool jaw pulley and formed to be rotatable around the fourth shaft or the fifth shaft; and a pitch wire coupled to each of the end tool pitch pulley and the driving part pitch pulley to connect the end tool pitch pulley to the driving part pitch pulley.

In an embodiment of the present disclosure, wherein a rotation amount of the driving part pitch pulley and a rotation amount of the end tool pitch pulley are substantially equal to each other.

In an embodiment of the present disclosure, wherein, when the driving part pitch pulley rotates by a first angle, the driving part jaw rotation shaft revolves by the first angle, and when the driving part pitch pulley rotates by the first angle, the end tool pitch pulley and the end tool jaw pitch main pulley rotate by a second angle.

In an embodiment of the present disclosure, the surgical instrument further comprises at least one driving part sub-relay pulley that is arranged adjacent to the driving part jaw pulley and transfers the jaw wire from the end tool to the driving part jaw pulley, wherein the jaw wire sequentially passes through the driving part sub-relay pulley, the driving part jaw pulley, and the driving part jaw rotation shaft.

In an embodiment of the present disclosure, wherein, when the driving part pitch pulley rotates, the driving part jaw rotation shaft rotates together such that a path length of the jaw wire, from an entry point to the driving part sub-relay pulley, through the driving part jaw pulley, and to an exit point from the driving part jaw rotation shaft, is changed.

In an embodiment of the present disclosure, wherein, when the driving part pitch pulley rotates, a path length of the jaw wire, from a point at which the jaw wire first contacts the driving part jaw pulley, to a point at which the jaw wire last contacts the driving part jaw pulley, on an arrangement path of the jaw wire connecting the end tool jaw pulley to the driving part jaw rotation shaft, is changed.

In an embodiment of the present disclosure, wherein the driving part jaw pulley is formed to be rotatable around the second shaft, and the driving part jaw rotation shaft is formed to be revolvable around the second shaft.

In an embodiment of the present disclosure, wherein, when the driving part pitch pulley rotates around the second shaft, the driving part jaw rotation shaft connected to the driving part pitch pulley revolves around the second shaft, such that the overall length of the jaw wire within the driving part is changed.

In an embodiment of the present disclosure, wherein, when the driving part pitch pulley rotates around the second shaft, the driving part jaw rotation shaft rotates around the second shaft while maintaining a certain distance from the second shaft, in a state in which the driving part jaw rotation shaft is spaced apart from the second shaft by a certain extent.

In an embodiment of the present disclosure, the surgical instrument further comprises a base plate formed to rotate together with the driving part pitch pulley around the second shaft, wherein the driving part jaw rotation shaft is formed on the base plate.

In an embodiment of the present disclosure, wherein two or more holes are formed on the base plate, and the driving part jaw rotation shaft is arranged in at least one of the holes.

In an embodiment of the present disclosure, wherein the base plate rigidly connects the driving part pitch pulley to the driving part jaw rotation shaft, such that, when the driving part pitch pulley rotates around the second shaft, the driving part jaw rotation shaft revolves around the second shaft.

In an embodiment of the present disclosure, wherein, when the driving part jaw rotation shaft rotates around the second shaft, a length of the jaw wire by which the jaw wire is wound around the driving part jaw pulley is changed.

In an embodiment of the present disclosure, wherein, even when the driving part jaw pulley rotates, the overall length of the jaw wire within the driving part is kept constant.

In an embodiment of the present disclosure, wherein the jaw wire is combined with each of the end tool jaw pulley and the driving part jaw pulley to form a closed loop as a whole.

In an embodiment of the present disclosure, wherein the yaw rotation is a motion in which the end tool jaw pulley rotates around the first shaft, and the pitch rotation is a motion in which the end tool jaw pulley revolves around a fourth shaft different from the first shaft.

One aspect of the present disclosure provides a surgical robot comprising: one or more robotic arm units configured to perform a motion by handle manipulation by an operator; and a surgical instrument coupled to the robotic arm unit, wherein the surgical instrument comprises: an end tool comprising one or more jaws and an end tool jaw pulley, which is coupled to the jaw, and formed to be rotatable together with the jaw around a first shaft, the end tool being formed to be able to perform at least pitch rotation and yaw rotation; a jaw wire that is coupled to the end tool jaw pulley and moves according to rotation of the end tool jaw pulley; a connection part extending in one direction, through which the jaw wire passes, and having one end to which the end tool is coupled; and a driving part that is coupled to another end of the connection part and is configured to control the pitch rotation and the yaw rotation of the end tool, wherein the driving part comprises: a driving part jaw pulley that is formed to be rotatable around a second shaft, and is formed to wind at least a portion of the jaw wire; and a driving part jaw rotation shaft that is formed to be rotatable around a third shaft different from the second shaft, is relatively movable while maintaining a preset distance from a center of the driving part jaw pulley, revolves around the center of the driving part jaw pulley, and is coupled to the jaw wire, the jaw wire moves through rotation of the driving part jaw rotation shaft to cause the end tool jaw pulley and the jaw to rotate, two strands of the jaw wire, which emerge while being wound around the driving part jaw pulley, extend toward the end tool after being sequentially wound around the driving part jaw pulley, the driving part jaw rotation shaft, and the driving part jaw pulley, and when the driving part jaw rotation shaft moves relative to the driving part jaw pulley, the end tool performs the pitch rotation while an overall length of the jaw wire within the driving part is changed.

### ADVANTAGEOUS EFFECTS OF DISCLOSURE

According to the present disclosure, a pitch motion and a yaw motion/actuation motion can be smoothly performed independently by compensating for jaw wire movement occurring during the pitch motion.

### Brief Description of Drawings

FIG. 1 is a conceptual diagram illustrating a surgical robotic system on which a surgical instrument is mounted, according to an embodiment of the present disclosure.
FIG. 2 is a block diagram illustrating an internal configuration of the surgical robotic system of FIG. 1.
FIG. 3 is a perspective view illustrating a surgical robot of the surgical robotic system of FIG. 1, and a surgical instrument mounted on the surgical robot.
FIG. 4 is a diagram illustrating a state in which an instrument case in FIG. 3 is removed.
FIG. 5 is a diagram illustrating a state in which the surgical instrument is removed from the surgical robot of FIG. 3.
FIG. 6 is a perspective view illustrating a surgical instrument according to an embodiment of the present disclosure.
FIGS. 7 and 8 are perspective views of an end tool of the surgical instrument of FIG. 6.
FIGS. 9 and 10 are plan views of the end tool of the surgical instrument of FIG. 6.
FIG. 11 is a diagram illustrating a neutral state in a related-art surgical instrument.
FIG. 12 is a diagram illustrating the surgical instrument of FIG. 11 performing pitch compensation.
FIG. 13 is a conceptual diagram of pitch motion compensation of the surgical instrument of FIG. 11.
FIG. 14 is a conceptual diagram of pitch motion compensation of the surgical instrument of FIG. 6.
FIGS. 15 to 18 are perspective views illustrating a driving part of the surgical instrument of FIG. 6.
FIGS. 19 and 20 are plan views illustrating the driving part of the surgical instrument of FIG. 6.
FIG. 21 is a side view illustrating the driving part of the surgical instrument of FIG. 6.
FIG. 22 is a bottom perspective view illustrating the driving part of FIG. 15.
FIG. 23 is a diagram partially illustrating components related to a first jaw in the driving part of FIG. 15.
FIG. 24 is a diagram partially illustrating components related to a second jaw in the driving part of FIG. 15.
FIG. 25 is a plan view illustrating the driving part of the surgical instrument of FIG. 6.
FIG. 26 is a diagram illustrating an end tool of the surgical instrument of FIG. 25.
FIG. 27 is a diagram illustrating a driving part and an end tool during a pitch motion of the surgical instrument of FIGS. 25 and 26.
FIG. 28 is a diagram illustrating the driving part and the end tool of FIGS. 25 and 26 during a pitch motion in the opposite direction to that in FIG. 27.
FIG. 29 is a plan view illustrating the driving part and the end tool of the surgical instrument of FIG. 6.
FIG. 30 is a diagram illustrating the driving part and the end tool during a yaw motion of the surgical instrument of FIG. 6.
FIGS. 31 to 33 are perspective views illustrating a motor pack according to an embodiment of the present disclosure.
FIG. 34 is a bottom perspective view illustrating the motor pack of FIG. 31.
FIG. 35 is a plan view illustrating the motor pack of FIG. 31.
FIG. 36 is a diagram illustrating rotation by a pitch driving motor in FIG. 31.
FIG. 37 is a diagram illustrating rotation by a second jaw driving motor in FIG. 31.
FIG. 38 is a diagram illustrating rotation by a pitch driving motor and a second jaw driving motor in FIG. 31.
FIGS. 39 and 40 are perspective views illustrating a driving part according to another embodiment of the present disclosure.
FIG. 41 is a plan view illustrating a driving part according to another embodiment of the present disclosure.
FIG. 42 is a bottom perspective view illustrating a driving part according to another embodiment of the present disclosure.
FIGS. 43 and 44 are perspective views illustrating a driving part according to another embodiment of the present disclosure.
FIG. 45 is a plan view illustrating a driving part according to another embodiment of the present disclosure.
FIG. 46 is a bottom perspective view illustrating a driving part according to another embodiment of the present disclosure.
FIGS. 47 to 51 are perspective views illustrating a motor pack according to another embodiment of the present disclosure.
FIG. 52 is a plan view illustrating a motor pack according to another embodiment of the present disclosure.
FIG. 53 is a diagram illustrating rotation by a pitch driving motor in FIG. 51.
FIGS. 54 to 56 are enlarged views sequentially illustrating rotation by a pitch driving motor.
FIG. 57 is a diagram illustrating rotation by a second jaw driving motor in FIG. 51.
FIG. 58 is a diagram illustrating rotation by a pitch driving motor and a second jaw driving motor in FIG. 51.

### Best Mode

While the present disclosure is susceptible to various modifications and alternative forms, specific embodiments thereof are shown by way of example in the drawings and will herein be described in detail. However, it should be understood that there is no intent to limit the present disclosure to the particular forms disclosed herein, rather, the present disclosure should be construed to cover various modifications, equivalents, and alternatives of embodiments of the present disclosure. In describing the present disclosure, a detailed description of known related arts will be omitted when it is determined that the gist of the present disclosure may be unnecessarily obscured

Although terms such as "first", "second", and the like may be used to describe various components, such components should not be limited to the above terms The terms are only used to distinguish one component from another.

The terms used herein are for the purpose of describing particular embodiments only and are not intended to be limiting to the present disclosure. Singular forms are intended to include plural forms as well, unless the context clearly indicates otherwise. In the present application, it will be further understood that the terms "comprise", "comprising", "include", and/or "including", when used in this specification, specify the presence of stated features, integers, steps, operations, elements, components and/or groups thereof but do not preclude the presence or addition of one or more other features, integers, steps, operations, elements, components and/or groups thereof.

Hereinafter, the embodiments of the present disclosure will be described below in detail with reference to the accompanying drawings, and when the embodiments of the present disclosure are described with reference to the drawings, the same or corresponding components are given the same reference numerals, and repetitive descriptions thereof will be omitted.

Further, in describing the various embodiments of the present disclosure, it is to be understood that each embodiment is not intended to be interpreted or implemented independently, and that the technical ideas described in each embodiment may be interpreted or implemented in combination with other embodiments described separately.

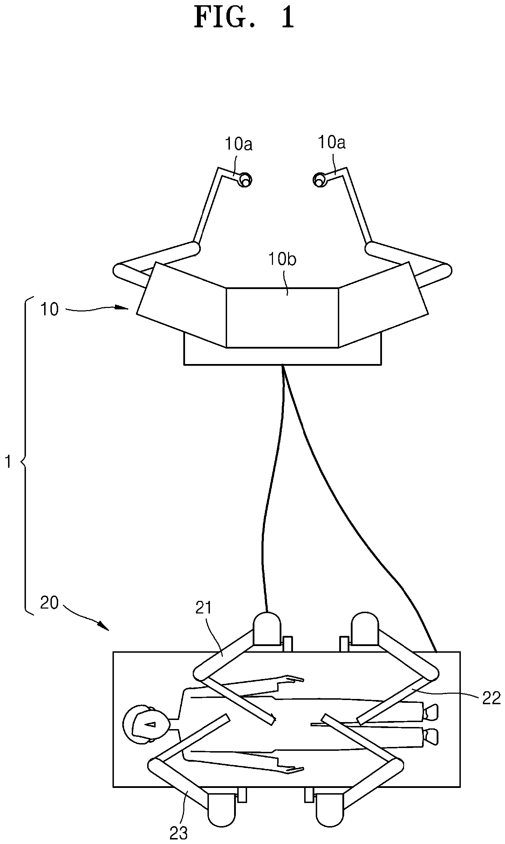

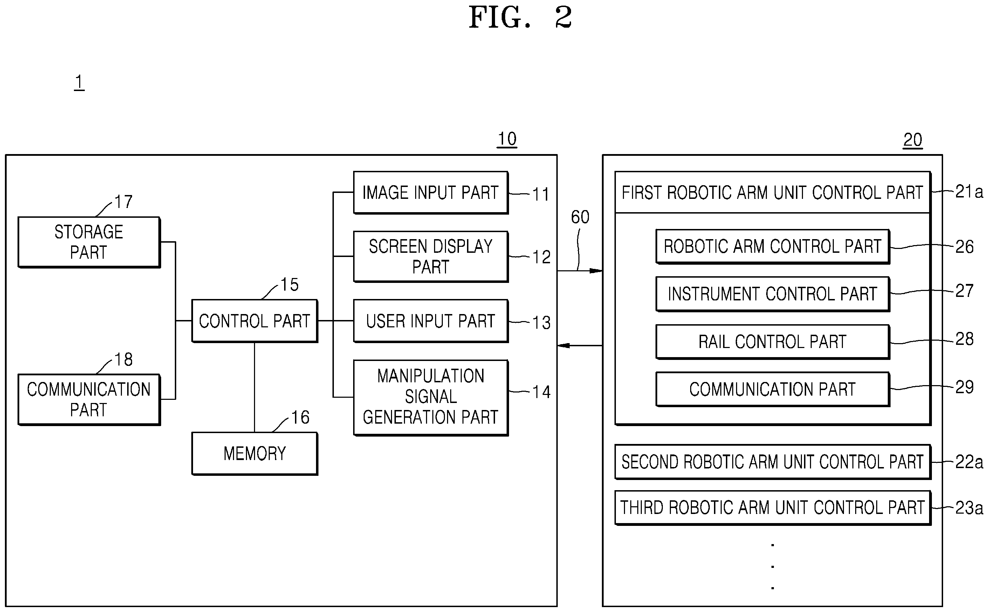

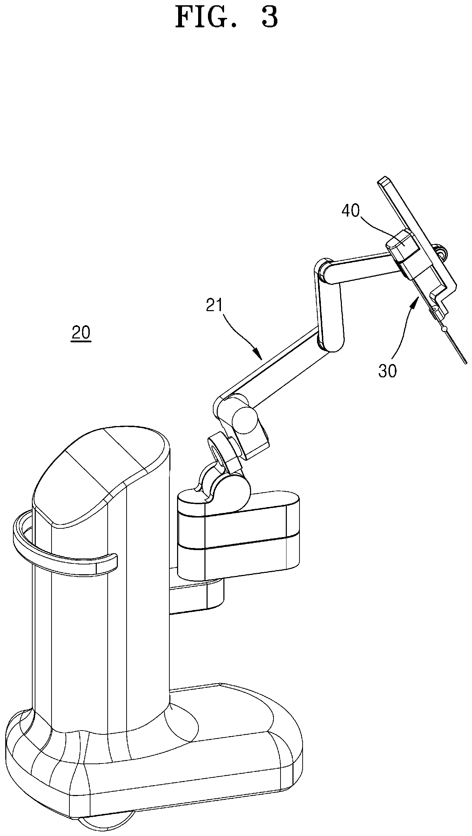

FIG. 1 is a conceptual diagram illustrating a surgical robotic system on which a surgical instrument is mounted, according to an embodiment of the present disclosure. FIG. 2 is a block diagram illustrating an internal configuration of the surgical robotic system of FIG. 1. FIG. 3 is a perspective view illustrating a surgical robot of the surgical robotic system of FIG. 1, and a surgical instrument mounted on the surgical robot.

Referring to FIGS. 1 to 3, a surgical robot system 1 includes a master robot 10, a surgical robot 20, and a surgical instrument 30.

The master robot 10 includes manipulating members 10a and a display member 10b, and the surgical robot 20 includes one or more robot arm units 21, 22, and 23.

In detail, the master robot 10 includes the manipulating members 10a so that a surgical operator can grip and manipulate them respectively with both hands. The manipulating members 10a may be implemented as two or more handles as illustrated in FIG. 1, and manipulation signals according to the handle manipulation of the surgical operator are transmitted to the surgical robot 20 through a wired or wireless communication network so that the robot arm units 21, 22, and 23 are controlled. That is, surgical motions such as positioning, rotation, and cutting operations of the robot arm units 21, 22, and 23 may be performed by the handle manipulation of the surgical operator.

For example, the surgical operator may manipulate the robot arm units 21, 22, and 23 using manipulation levers in the form of a handle. The manipulation lever as described above may have various mechanical configurations according to the manipulate method thereof, and may be provided in various configurations for operating the robot arm units 21, 22, and 23 of the surgical robot 20 and/or other surgical instruments, such as a master handle manipulating the motion of each of the robot arm units 21, 22, and 23 and various input tools added to the master robot 10 for manipulating the functions of the entire system such as joystick, keypad, trackball, foot pedal, and touch screen. Here, the manipulating member 10a is not limited to the shape of a handle and can be applied without any limitation as long as it can control motions of the robot arm units 21, 22, and 23 through a network such as a wired or wireless communication network.

Alternatively, a voice input or a motion input may also be applied for user input. That is, a user may wear, on the head thereof, glasses or a head mount display (HMD), to which a sensor is attached , and a laparoscope may move according to a direction in which the user's gaze. Alternatively, when the user issues a command with voice, such as "left", "right", "first arm", "second arm", and the like, the voice command may be recognized and the motion may be performed.

An image captured through the laparoscope to be described later is displayed as a screen image on the display member 10b of the master robot 10. In addition, a predetermined virtual manipulation plate may be displayed independently or displayed together with the image captured by the laparoscope on the display member 10b. A detailed description of the arrangement, configuration, and the like of such a virtual manipulation plate will be omitted.

Here, the display member 10b may include one or more monitors, each of which may individually display information necessary for surgery. The quantity of monitors may be variously determined depending on the type or kind of information that needs to be displayed.

Meanwhile, the surgical robot 20 may include one or more robot arm units 21, 22, and 23. Here, each of the robot arm units 21, 22, and 23 may be provided in the form of a module that can operate independently of each other, and in this case, an algorithm for preventing a collision between the robot arm units 21, 22, and 23 may be applied to the surgical robot system 1.

In general, a robot arm refers to a device having a function similar to that of the arm and/or the wrist of a human being and having a wrist portion to which a predetermined tool may be attached. In the present specification, the robot arm units 21, 22, and 23 may each be defined as a concept encompassing all of the components such as an upper arm, a lower arm, a wrist, and an elbow, a multi-joint type surgical device coupled to the wrist portion, and the like. Alternatively, the robot arm unit may also be defined as a concept that includes only components for driving the multi-joint type surgical device, excluding the multi-joint type surgical device coupled to the wrist portion.

The robot arm units 21, 22, and 23 of the surgical robot 20 described above may be implemented to be driven with multiple degrees of freedom. The robot arm units 21, 22, and 23 may include, for example, a surgical instrument inserted into a surgical site of a patient, a yaw driving part for rotating the surgical instrument in a yaw direction according to a surgical position, a pitch driving part for rotating the surgical instrument in a pitch direction perpendicular to a rotational driving of the yaw driving part, a transfer driving part for moving the surgical instrument in a length direction, a rotation driving part for rotating the surgical instrument, and a surgical instrument driving part for incising or cutting the surgical lesion by driving an end effector at an end of the surgical instrument. However, the configuration of the robot arm units 21, 22, and 23 is not limited thereto, and it should be understood that this example does not limit the scope of the present disclosure. Here, a detailed description of the actual control process, such as rotation and movement of the robot arm units 21, 22, and 23 in a corresponding direction by the surgical operator manipulating the manipulating member 10a will be omitted.

Here, two of the robot arm units 21, 22, and 23 may have the surgical instrument 30 attached thereto, and one of the robot arm units 21, 22, and 23 may have the laparoscope attached thereto. In addition, the surgical operator may select the robot arm unit 21, 22, or 23 to be controlled via the master robot 10. As described above, by directly controlling a total of three or more surgical instruments through the master robot 10, the surgical operator may accurately and freely control various tools according to the intention of the surgical operator without a surgical assistant.

Meanwhile, one or more surgical robots 20 may be provided to operate the patient, and the laparoscope for allowing a surgical site to be displayed as a screen image through the display member 10b may be implemented as an independent surgical robot 20. In addition, as described above, the embodiments of the present disclosure can be used universally for surgeries in which various surgical endoscopes other than laparoscopes (e.g., thoracoscopic, arthroscopic, rhinoscopic, and the like) are used.

Referring to FIG. 2, in an embodiment of the present disclosure, the master robot 10 may include an image input part 11, a screen display part 12, a user input part 13, a manipulation signal generation part 14, a control part 15, a memory 16, a storage part 17, and a communication part 18.

The image input part 11 may receive an image captured by a camera provided in the laparoscope of the surgical robot 20 through a wired or wireless communication network.

The screen display part 12 outputs a screen image corresponding to the image received through the image input part 11 as visual information. In addition, the screen display part 12 may further output information corresponding to biometric information of a subject to be treated, when the biometric information is input. In addition, the screen display part 12 may further output image data (e.g., an X-ray image, a CT image, an MRI image, or the like) associated with a patient for a surgical site. Here, the screen display part 12 may be implemented in the form of a display member (see 10b of FIG. 1), and an image processing process for allowing the received image to be output as a screen image through the screen display part 12 may be performed by the control part 15.

In the embodiment illustrated in FIG. 2, the image input part and the screen display part are illustrated as being included in the master robot 10, but the present disclosure is not limited thereto. That is, the display member may be provided as a separate member spaced apart from the master robot 10. Alternatively, the display member may be provided as one component of the master robot 10. In addition, in another embodiment, a plurality of display members may be provided, one of which may be disposed adjacent to the master robot 10, and others thereof may be disposed at some distance from the master robot 10.

Here, the screen display part 12 (that is, the display member 10b of FIG. 1) may be provided as a three-dimensional display device. In detail, the three-dimensional display device refers to an image display device in which depth information is added to a two-dimensional image by applying a stereoscopic technique, and this depth information is used to enable an observer to feel a three-dimensional living feeling and a sense of reality. The surgical robot system 1 according to an embodiment of the present disclosure may provide a more realistic virtual environment to a user by including a three-dimensional display device as the screen display part 12.

The user input part 13 is a member for allowing the surgical operator to manipulate the positions and functions of the robot arm units 21, 22, and 23 of the surgical robot 20. The user input part 13 may be formed in the form of a handle-shaped manipulation member (see 10a of FIG. 1) as illustrated in FIG. 1, but the shape thereof is not limited thereto and may be implemented by being modified in various shapes to achieve the same purpose. In addition, for example, some of the user input part 13 may be formed in the shape of a handle, and the others may be formed in a different shape, such as a clutch button. In addition, a finger insertion tube or insertion ring may be further formed so as to allow the surgical operator's finger to be inserted therethrough and fixed to facilitate manipulation of the surgical tool.

When the surgical operator manipulates the user input part 13 to move the positions of robot arm units 21, 22, and 23 or manipulate surgical operations thereof, the manipulation signal generation part 14 may generate a corresponding manipulation signal, and transmit the manipulation signal to the surgical robot 20 through the communication part 18. The manipulation signal may be transmitted and received via a wired or wireless communication network.

The control part 15 is a kind of central processing device, and controls the operation of each component so that the above-described functions can be performed. In an example, the control part 15 may perform a function of converting an image input through the image input part 11 into a screen image to be displayed through the screen display part 12.

The memory 16 may perform a function of temporarily or permanently storing data processed by the control part 15. Here, the memory 16 may include a magnetic storage medium or a flash storage medium, but the scope of the present disclosure is not limited thereto.

The storage part 17 may store data received from the surgical robot 20. In addition, the storage part 17 may store various pieces of input data (e.g., patient data, device data, surgery data, and the like).

The communication part 18 interworks with a communication network 60 to provide a communication interface necessary for transmitting and receiving image data transmitted from the surgical robot 20 and control data transmitted from the master robot 10.

The surgical robot 20 includes a plurality of robot arm unit control parts 21a, 22a, and 23a. In addition, the robot arm unit control part 21a includes a robot arm control part 26, an instrument control part 27, and a communication part 29. In addition, the robot arm unit control part 21a may further include a rail control part 28.

The robot arm control part 26 may receive a manipulation signal generated by the manipulation signal generation part 14 of the master robot 10, and may serve to control the robot arm units 21, 22, and 23 so as to operate according to the manipulation signal.

The instrument control part 27 may receive a manipulation signal generated by the manipulation signal generation part 14 of the master robot 10, and may serve to control the surgical instrument 30 so as to operate according to the manipulation signal.

The communication part 29 interworks with the communication network 60 to provide a communication interface necessary for transmitting and receiving image data transmitted from the surgical robot 20 and control data transmitted from the master robot 10.

Meanwhile, the communication network 60 serves to connect the master robot 10 and the surgical robot 20. That is, the communication network 60 refers to a communication network for providing an access path so that data can be transmitted and received between the master robot 10 and the surgical robot 20 after the master robot 10 and the surgical robot 20 are connected. The communication network 60 may be, for example, a wired network such as local area networks (LANs), wired area networks (WANs), metropolitan area networks (MANs), and integrated service digital networks (ISDNs), or a wireless network such as wireless LANs, code division multiple access (CDMA), Bluetooth, and satellite communication, but the scope of the present disclosure is not limited thereto.

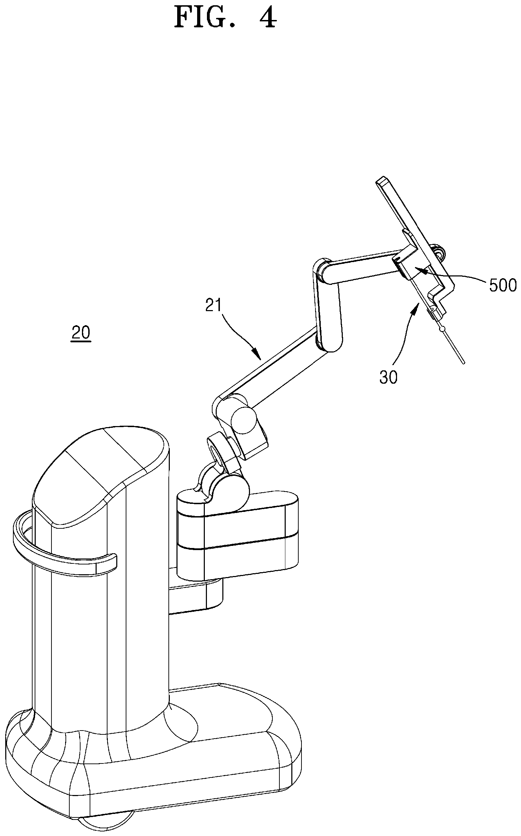

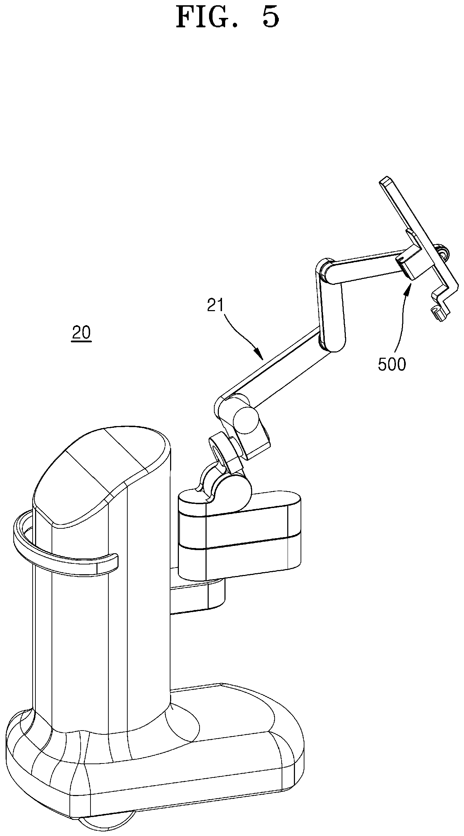

FIG. 3 is a perspective view illustrating a surgical robot of the surgical robotic system of FIG. 1, and a surgical instrument mounted on the surgical robot. FIG. 4 is a diagram illustrating a state in which an instrument case in FIG. 3 is removed. FIG. 5 is a diagram illustrating a state in which the surgical instrument is removed from the surgical robot of FIG. 3.

The surgical instrument 30, which will be described below, may be connected to and installed in the robotic arm unit 21, 22, or 23. Referring to FIG. 3, an instrument case 40 may cover the surgical instrument 30, and may be connected to the robotic arm unit 21. The instrument case 40 may cover one side of the surgical instrument 30 exposed to the outside, so as to prevent external foreign substances from reaching the surgical instrument 30, and protect the surgical instrument 30 from being damaged due to external shock.

FIG. 3 illustrates that only one robotic arm unit 21 among the robotic arm units 21, 22, and 23 is combined with the surgical instrument 30, but the present disclosure is not limited thereto, and as described above, the surgical instruments 30 may be attached to two of the robotic arm units 21, 22, and 23, and a laparoscope may be attached to the other one.

Referring to FIGS. 3 to 5, a motor pack 500 is connectable to the surgical instrument 30, and may be coupled to the surgical robot 20, specifically, the robotic arm unit 21, and fixed in position.

The instrument case 40 may be connected to one side of the surgical instrument 30, and the motor pack 500 may be connected to the opposite side. The motor pack 500 is for generating power by receiving power from the outside, and may transmit the power generated by the motor pack 500 to the surgical instrument 30, and accordingly, the surgical instrument 30 may perform a pitch motion, a yaw motion, an actuation motion, and a roll motion.

### (Multi-joint type surgical device)

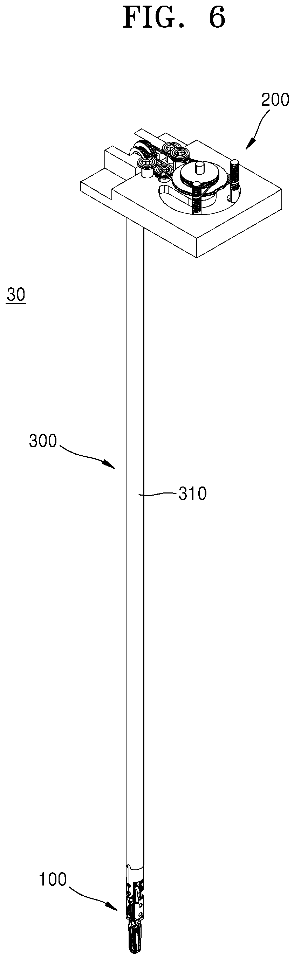

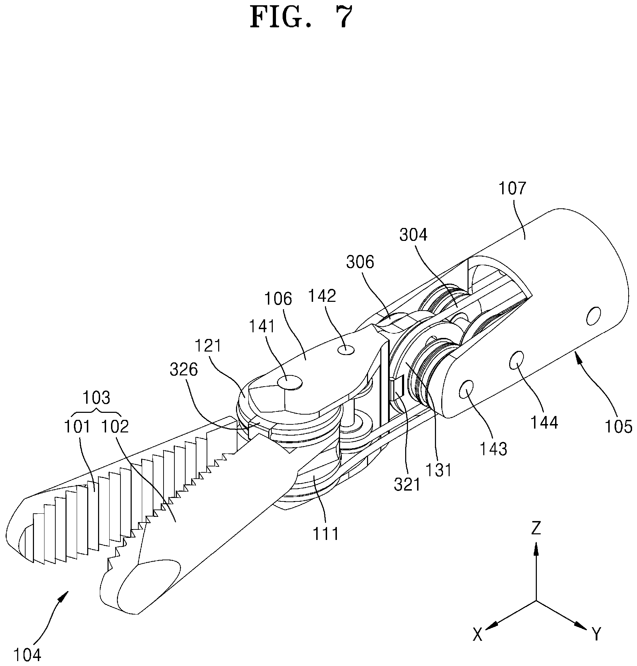

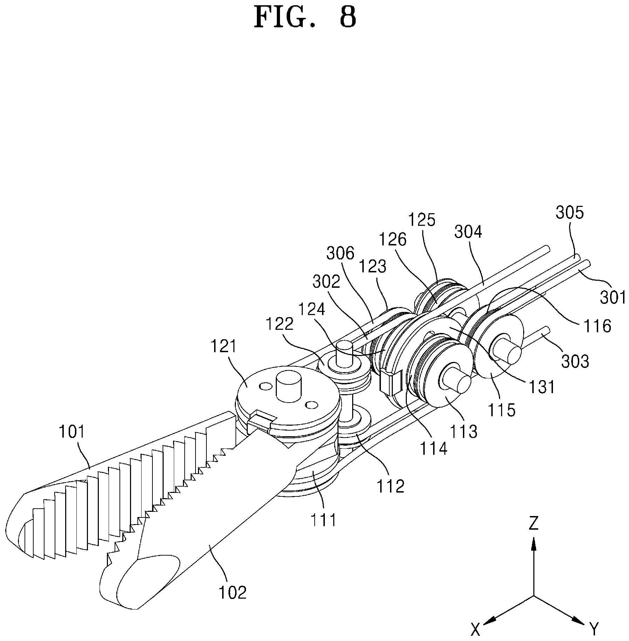

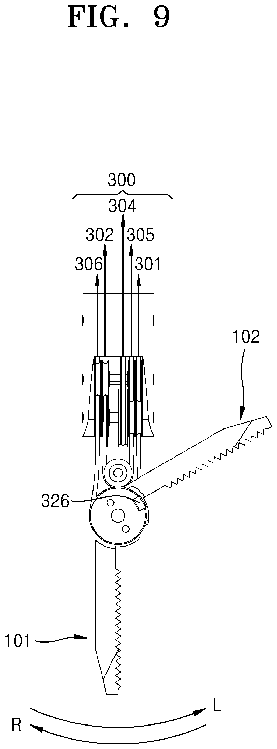

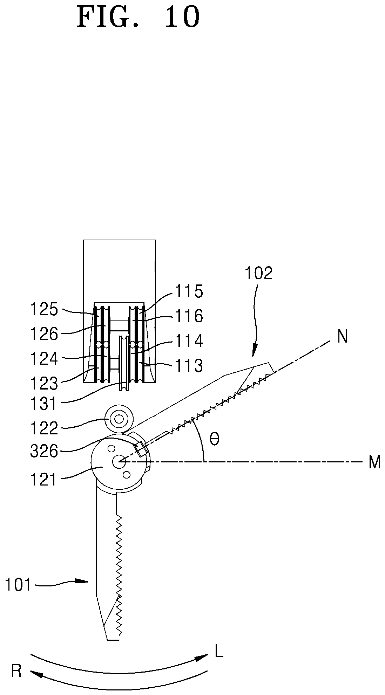

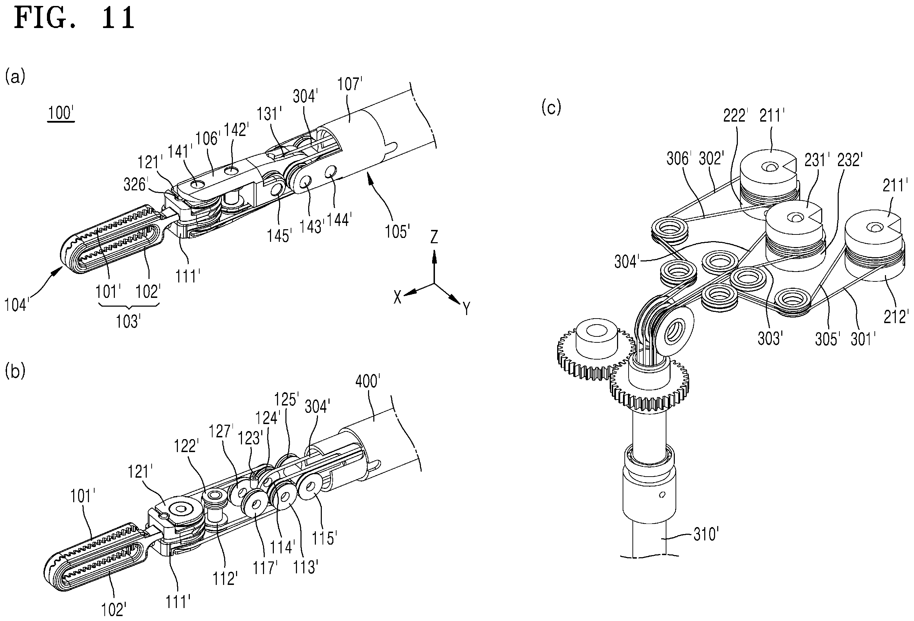

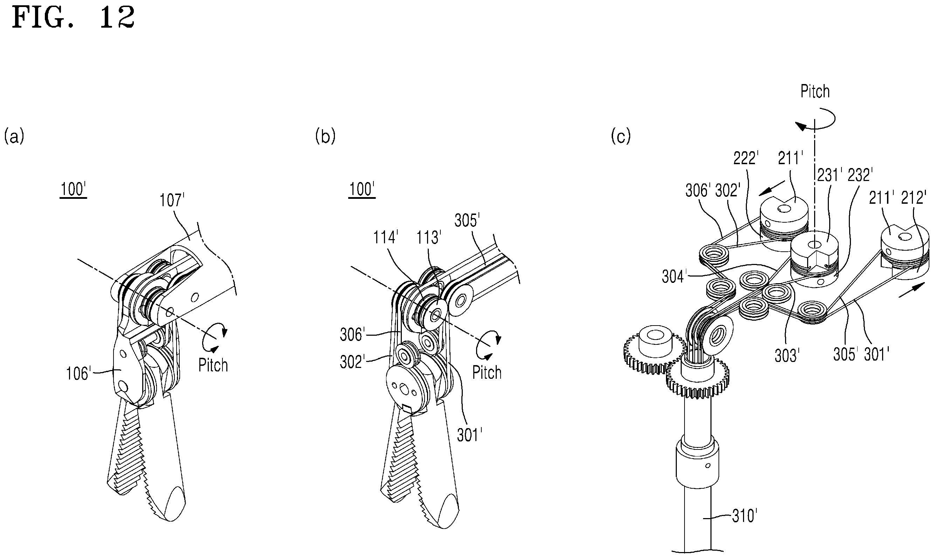

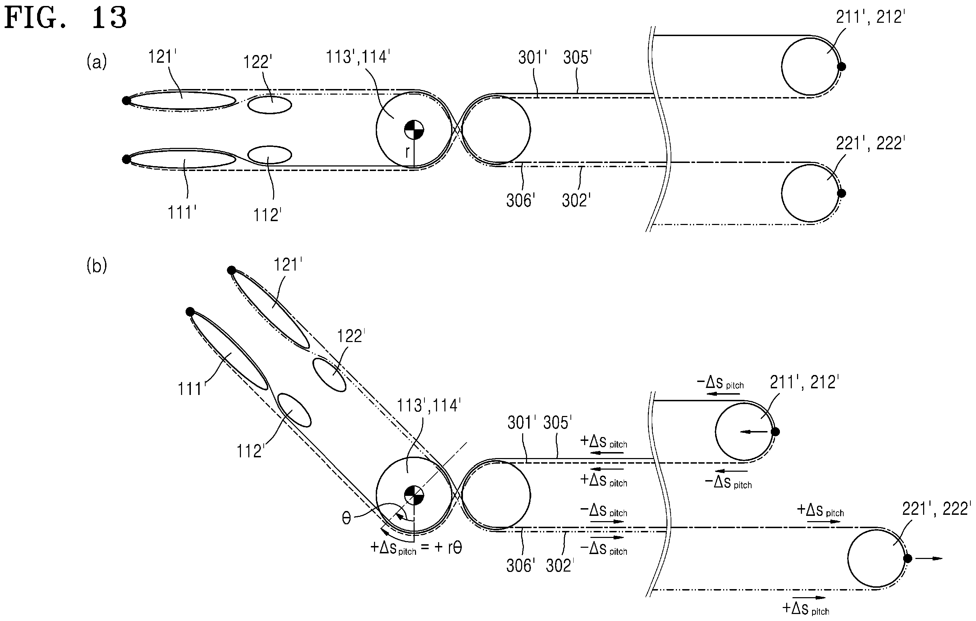

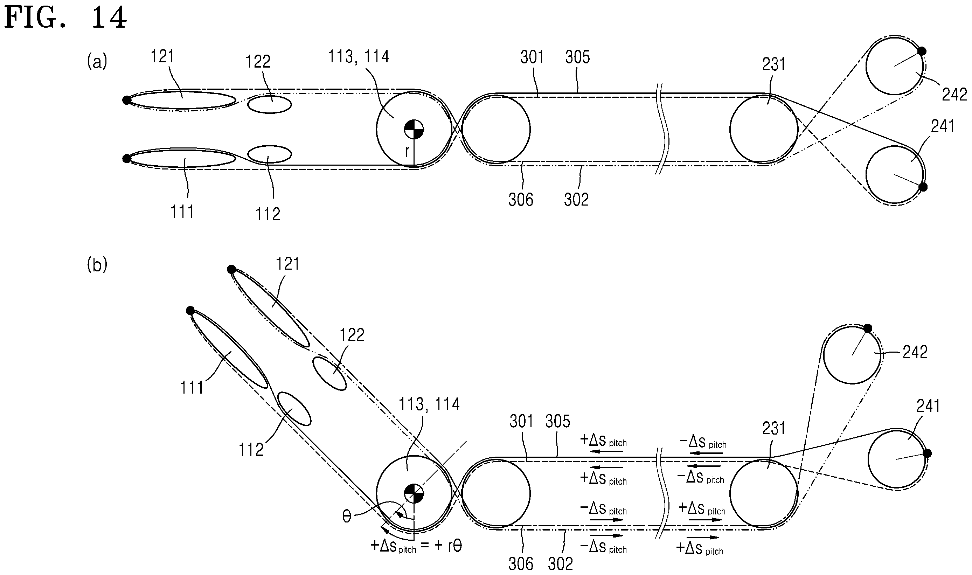

FIG. 6 is a perspective view illustrating a surgical instrument according to an embodiment of the present disclosure. FIGS. 7 and 8 are perspective views of an end tool of the surgical instrument of FIG. 6. FIGS. 9 and 10 are plan views of the end tool of the surgical instrument of FIG. 6. FIG. 11 is a diagram illustrating a neutral state in a related-art surgical instrument. FIG. 12 is a diagram illustrating the surgical instrument of FIG. 11 performing pitch compensation. FIG. 13 is a conceptual diagram of pitch motion compensation of the surgical instrument of FIG. 11. FIG. 14 is a conceptual diagram of pitch motion compensation of the surgical instrument of FIG. 6.

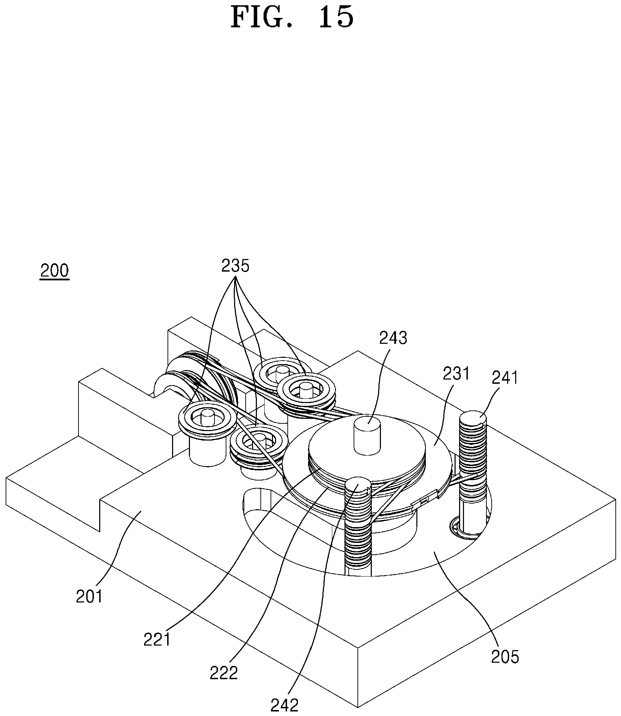

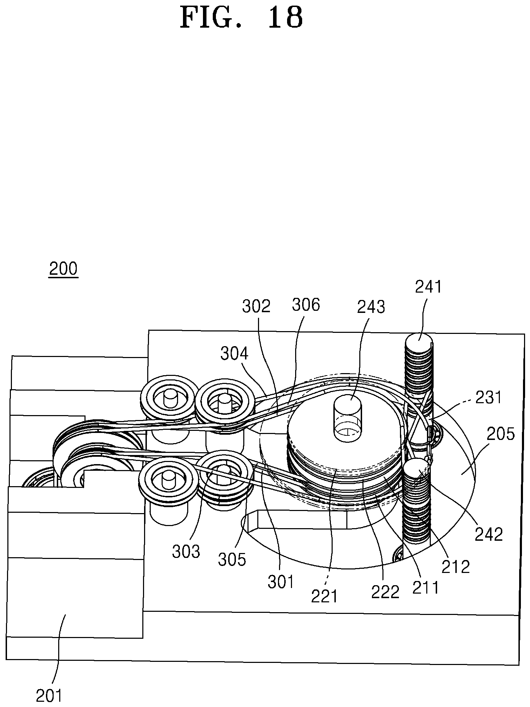

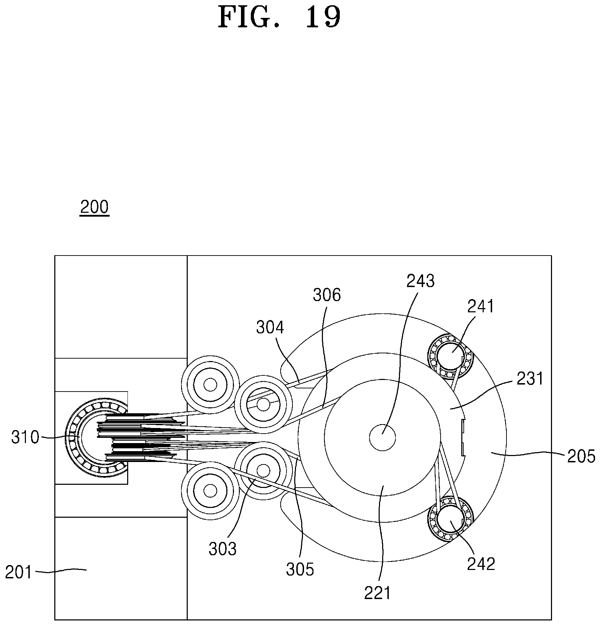

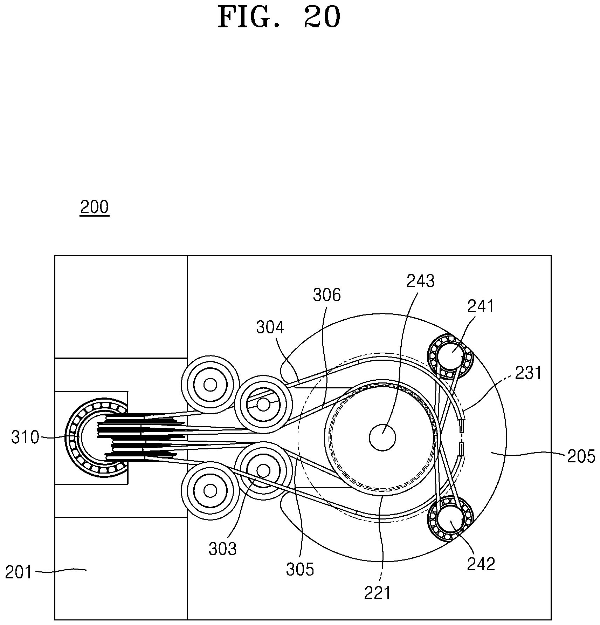

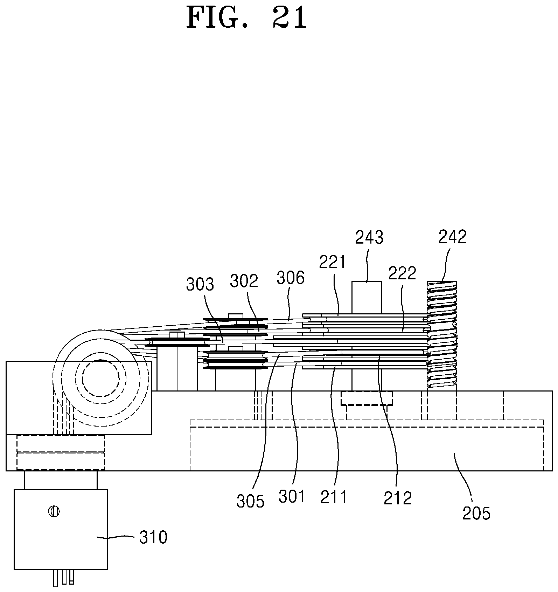

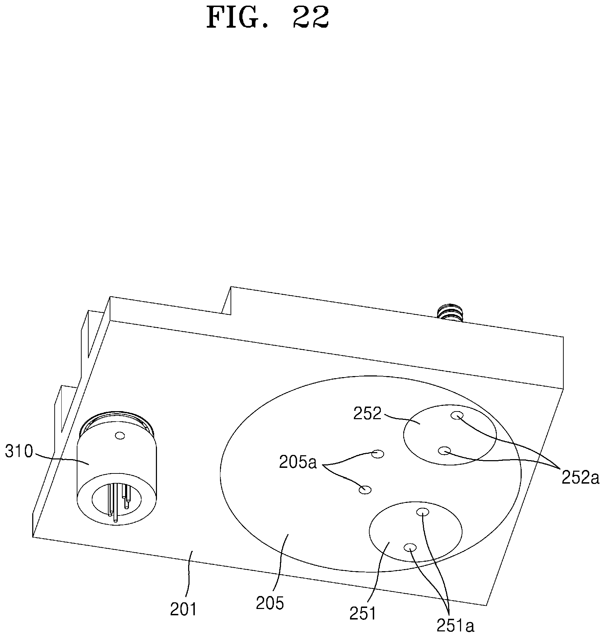

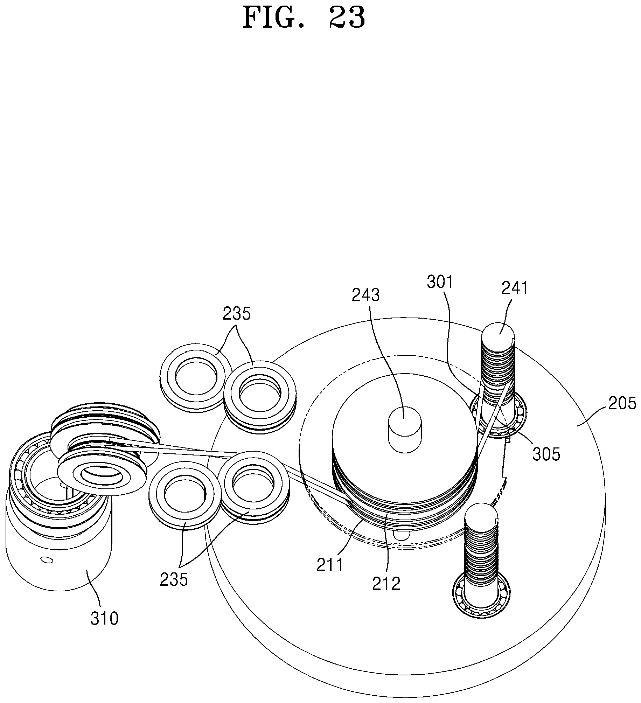

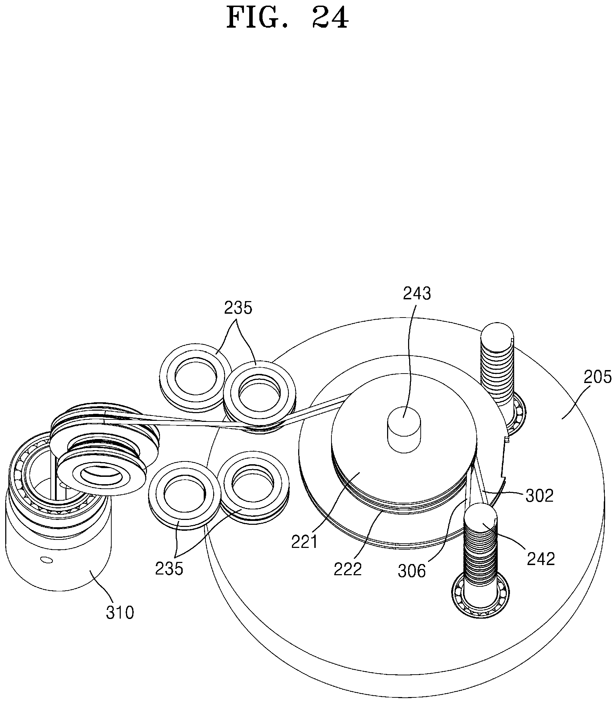

FIGS. 15 to 18 are perspective views illustrating a driving part of the surgical instrument of FIG. 6. FIGS. 19 and 20 are plan views illustrating the driving part of the surgical instrument of FIG. 6. FIG. 21 is a side view illustrating the driving part of the surgical instrument of FIG. 6. FIG. 22 is a bottom perspective view illustrating the driving part of FIG. 15. FIG. 23 is a diagram partially illustrating components related to a first jaw in the driving part of FIG. 15. FIG. 24 is a diagram partially illustrating components related to a second jaw in the driving part of FIG. 15.

Referring first to FIG. 6, a surgical instrument 30 according to a first embodiment of the present disclosure may include an end tool 100, a driving part 200, and a power transmission part 300, and the power transmission part 300 may include a connection part 310.

Here, the connection part 310 is formed in the shape of a hollow shaft, in which one or more wires (to be described later) may be accommodated, and may have one end portion to which the driving part 200 is coupled and the other end portion to which the end tool 100 is coupled and serve to connect the driving part 200 and the end tool 100.

The driving part 200 is formed at one end portion of the connection part 310 and provides an interface capable of being coupled to the robot arm unit (see 21 or the like in FIG. 1). Accordingly, when a user operates the master robot (see 10 in FIG. 1), a motor pack(500) of the robot arm unit (see 21 or the like in FIG. 1) is operated so that the end tool 100 of the surgical instrument 30 can perform a motion corresponding thereto, and a driving force of the motor pack(500) is transmitted to the end tool 100 through the driving part 200. Viewed from another perspective, it may be described that the driving part 200 itself becomes an interface between the surgical instrument 30 and the surgical robot 20.

The end tool 100 is formed on the other end portion of the connection part 310, and performs necessary motions for surgery by being inserted into a surgical site. In an example of the above-described end tool 100, as shown in FIG. 5, a pair of jaws 101 and 102 for performing a grip motion may be used. However, the concept of the present disclosure is not limited thereto, and various devices for performing surgery may be used as the end tool 100. For example, a configuration such as a cantilever cautery may also be used as the end tool. The above-described end tool 100 is connected to the driving part 200 by the power transmission part 300 and receives a driving force through the power transmission part 300 to perform a motion necessary for surgery, such as a gripping motion, a cutting motion, a suturing motion, or the like.

Here, the end tool 100 of the surgical instrument 30 according to the first embodiment of the present disclosure is formed to be rotatable in at least two directions, for example, the end tool 100 may be formed to perform a pitch motion around a rotation shaft 143 of FIG. 5 and simultaneously perform a yaw motion and an actuation motion around a rotation shaft 141 of FIG. 5.

Here, each of the pitch, yaw, and actuation motions used in the present disclosure are defined as follows.

First, the pitch motion means a motion of the end tool 100 rotating in a vertical direction with respect to an extension direction of the connection part 310 (an X-axis direction of FIG. 4), that is, a motion rotating around the Y-axis of FIG. 4. In other words, the pitch motion means a motion of the end tool 100, which is formed to extend from the connection part 310 in the extension direction of the connection part 310 (the X-axis direction of FIG. 4), rotating vertically around the Y-axis with respect to the connection part 310.

Next, the yaw motion means a motion of the end tool 100 rotating in left and right directions, that is, a motion rotating around the Z-axis of FIG. 4, with respect to the extension direction of the connection part 310 (the X-axis direction of FIG. 4). In other words, the yaw motion means a motion of the end tool 100, which is formed to extend from the connection part 310 in the extension direction of the connection part 310 (the X-axis direction of FIG. 4), rotating horizontally around the Z-axis with respect to the connection part 310. That is, the yaw motion means a motion of two jaws 101 and 102, which are formed on the end tool 100, rotating around the Z-axis in the same direction.

Meanwhile, the actuation motion means a motion of the end tool 100 rotating around the same axis of rotation as that of the yaw motion, while the two jaws 101 and 102 rotate in the opposite directions so as to be closed or opened. That is, the actuation motion means rotating motions of the two jaws 101 and 102, which are formed on the end tool 100, in the opposite directions around the Z-axis.

Defining this from another perspective, the yaw rotation may be defined as a motion in which an end tool jaw pulley to be described later is rotated around the rotation shaft 141, which is an end tool jaw pulley rotation shaft, and the pitch rotation may be defined as a motion in which the end tool jaw pulley is revolved around the rotation shaft 143, which is an end tool pitch rotation shaft.

The power transmission part 300 may connect the driving part 200 and the end tool 100, transmit the driving force from the driving part 200 to the end tool 100, and include a plurality of wires, pulleys, links, sections, gears, or the like.

Hereinafter, the end tool 100, the driving part 200, the power transmission part 300, and the like of the surgical instrument 30 of FIG. 4 will be described in more detail.

### (Power transmission part)

Hereinafter, the power transmission part 300 of the surgical instrument 30 of FIG. 6 will be described in more detail.

Referring to FIGS. 7 to 24, the power transmission part 300 of the surgical instrument 30 according to an embodiment of the present disclosure may include a wire 301, a wire 302, a wire 303, a wire 304, a wire 305, and a wire 306.

Here, the wires 301 and 305 may be paired to serve as first jaw wires. The wires 302 and 306 may be paired to serve as second jaw wires. Here, the components encompassing the wires 301 and 305, which are first jaw wires, and the wires 302 and 306, which are second jaw wires, may be referred to as jaw wires. In addition, the wires 303 and 304 may be paired to serve as pitch wires.

Here, in the drawings, a pair of wires are illustrated as being associated with a rotational motion of a first jaw 101, and a pair of wires are illustrated as being associated with a rotational motion of a second jaw 102, but the concept of the present disclosure is not limited thereto. For example, a pair of wires may be associated with a yaw motion, and a pair of wires may be associated with an actuation motion.

In addition, the power transmission part 300 of the surgical instrument 30 according to an embodiment of the present disclosure may include a coupling member 321, a coupling member 326, and the like, which are coupled to respective end portions of the wires in order to couple the wires and the pulleys. Here, each of the coupling members may have various shapes as necessary, such as a ball shape, a tube shape, and the like.

Here, the coupling member 321, which is a pitch wire coupling member, is coupled to the end portions of the wires 303 and 304, which are pitch wires, at the end tool 100 side to serve as a pitch wire-end tool coupling member. Meanwhile, although not illustrated in the drawings, a pitch wire-driving part coupling member (not shown) may be coupled to the end portions of the wires 303 and 304, which are pitch wires, at the driving part 200 side.

Meanwhile, the coupling member 326, which is a second jaw wire coupling member, is coupled to the end portions of the wires 302 and 306, which are second jaw wires, at the end tool 100 side to serve as a second jaw wire-end tool coupling member. Meanwhile, although not illustrated in the drawings, a second jaw wire-driving part coupling member (not shown) may be coupled to the end portions of the wires 302 and 306, which are second jaw wires, at the driving part 200 side.

Meanwhile, although not illustrated in the drawings, a coupling member (not shown) having the same shape as the coupling member 326 may be coupled to the end portions of the wires 301 and 305, which are first jaw wires, at the end tool 100 side to serve as a first jaw wire-end tool coupling member. Meanwhile, although not illustrated in the drawings, a first jaw wire-driving part coupling member (not shown) may be coupled to the end portions of the wires 301 and 305, which are first jaw wires, at the driving part 200 side.

Here, each of the coupling members is classified as being included in the power transmission part 300, but the coupling members may be classified such that the coupling member at the end tool 100 side may be included in the end tool 100, and the coupling member at the driving part 200 side may be included in the driving part 200.

The coupling relationship between the wires, the coupling members, and the respectively pulleys will be described in detail as follows.

First, the wires 302 and 306, which are second jaw wires, may be a single wire. The coupling member 326, which is a first jaw wire-end tool coupling member, is inserted at an intermediate point of the second jaw wire, which is a single wire, and the coupling member 326 is crimped and fixed, and then, both strands of the second jaw wire centered on the coupling member 326 may be referred to as the wire 302 and the wire 306, respectively.

Alternatively, the wires 302 and 306, which are second jaw wires, may also be formed as separate wires, and connected by the coupling member 326.

In addition, by coupling the coupling member 326 to a pulley 121, the wires 302 and 306 may be fixedly coupled to the pulley 121. This allows the pulley 121 to rotate as the wires 302 and 306 are pulled and released.

Meanwhile, the second jaw wire-driving part coupling member (not shown) may be coupled to the end portions of the wires 302 and 306, which are opposite to the end portions to which the coupling member 326 is coupled. That is, the second jaw wire-driving part coupling member (not shown) may be fixed to each of the wires 302 and 306 by inserting the opposite end portions of the wires 302 and 306 into the second jaw wire-driving part coupling member (not shown) and crimping the coupling member (not shown).

In addition, by coupling the second jaw wire-driving part coupling member (not shown) coupled to the wires 302 and 306 to each of the pulley 221 and the pulley 222, the wire 302 and the wire 306 may be fixedly coupled to the pulley 221 and the pulley 222, respectively. As a result, when the pulley 221 and the pulley 222 are rotated by a motor or a human force, the pulley 121 of the end tool 100 may be rotated as the wire 302 and the wire 306 are pulled and released.

Here, a driving part second jaw pulley may include two pulleys of the pulley 221 and the pulley 222, and thus the second jaw wire-driving part coupling member may also include two coupling members. Alternatively, the driving part second jaw pulley includes one pulley, the second jaw wire-driving part coupling member also includes one coupling member, and the wires 302 and 306 may be coupled to one coupling member to be coupled to one driving part second jaw pulley.

In the same manner, the wire 301 and the wire 305, which are first jaw wires, are coupled to the first jaw wire-end tool coupling member (not shown) and the first jaw wire-driving part coupling member (not shown), respectively. In addition, the first jaw wire-end tool coupling member (not shown) is coupled to a pulley 111, and the first jaw wire-driving part coupling member (not shown) is coupled to a pulley 211 and a pulley 212. As a result, when the pulleys 211 and 212 are rotated by a motor or a human force, the pulley 111 of the end tool 100 may be rotated as the wire 301 and the wire 305 are pulled and released.

In the same manner, each of one end portions of the wires 303 and 304, which are pitch wires, is coupled to the coupling member 321, which is a pitch wire-end tool coupling member, and each of the other end portions of the wires 303 and 304 are coupled to the pitch wire-driving part coupling member (not shown). In addition, the coupling member 321 is coupled to a pulley 131, and the pitch wire-driving part coupling member (not shown) is coupled to a pulley 231. As a result, when the pulley 231 is rotated by a motor or a human force, the pulley 131 of the end tool 100 may be rotated as the wire 303 and the wire 304 are pulled and released.

As a result, the wire 301 and the wire 305, which are both strands of the first jaw wire, are coupled to a coupling member 323, which is a first jaw wire-end tool coupling member, and the first jaw wire-driving part coupling member (not shown) so as to form as a whole a closed loop. Similarly, the second jaw wire and the pitch wire may each be formed to form a closed loop.

### (End tool)

Hereinafter, the end tool 100 of the surgical instrument 30 of FIG. 6 will be described in more detail.

FIGS. 7 and 8 are perspective views of an end tool of the surgical instrument of FIG. 6. FIGS. 9 and 10 are plan views of the end tool of the surgical instrument of FIG. 6. Here, FIG. 7 illustrates a state in which an end tool hub 106 and a pitch hub 107 are coupled, and FIG. 8 illustrates a state in which the end tool hub 106 and the pitch hub 107 are removed.

Referring to FIGS. 7 to 10, the end tool 100 of the first embodiment of the present disclosure includes a pair of jaws for performing a grip motion, that is, the first jaw 101 and the second jaw 102. Here, each of the first jaw 101 and the second jaw 102, or a component encompassing the first jaw 101 and the second jaw 102 may be referred to as a jaw 103.

Further, the end tool 100 may include the pulley 111, a pulley 112, a pulley 113, a pulley 114, a pulley 115, and a pulley 116 that are related to a rotational motion of the first jaw 101. In addition, the end tool 100 may include the pulley 121, a pulley 122, a pulley 123, a pulley 124, a pulley 125, and a pulley 126 that are related to a rotational motion of the second jaw 102.

Here, in the drawings, one group of pulleys are illustrated as being associated with a rotational motion of the first jaw 101, and one group of pulleys are illustrated as being associated with a rotational motion of the second jaw 102, but the concept of the present disclosure is not limited thereto. For example, one group of pulleys in the end tool may be associated with a yaw motion, and one group of pulleys in the end tool may be associated with an actuation motion. Here, the pulleys included in the end tool 100, including the pulleys described above, may be collectively referred to as end tool pulleys.

Here, the pulleys facing each other are illustrated in the drawings as being formed parallel to each other, but the concept of the present disclosure is not limited thereto, and each of the pulleys may be variously formed with a position and a size suitable for the configuration of the end tool.

Further, the end tool 100 of the first embodiment of the present disclosure may include the end tool hub 106 and the pitch hub 107.

The rotation shaft 141 and a rotation shaft 142, which will be described later, may be inserted through the end tool hub 106, and the end tool hub 106 may internally accommodate at least some of the first jaw 101 and the second jaw 102, which are axially coupled to the rotation shaft 141. In addition, the end tool hub 106 may internally accommodate at least some of the pulley 112 and the pulley 122 that are axially coupled to the rotation shaft 142.

In addition, the pulley 131 serving as an end tool pitch pulley may be formed at one end portion of the end tool hub 106. As shown in FIG. 5, the pulley 131 may be formed as a separate member from the end tool hub 106 and coupled to the end tool hub 106. Alternatively, although not illustrated in the drawings, the pulley 131 may be integrally formed with the end tool hub 106 as one body. That is, one end portion of the end tool hub 106 is formed in a disk shape or a semi-circular shape such as a pulley, and a groove around which a wire can be wound may be formed on an outer circumferential surface thereof. The wires 303 and 304 described above are coupled to the pulley 131 serving as an end tool pitch pulley, and a pitch motion is performed as the pulley 131 is rotated around the rotation shaft 143.

The rotation shaft 143 and a rotation shaft 144, which will be described later, may be inserted through the pitch hub 107, and the pitch hub 107 may be axially coupled to the end tool hub 106 and the pulley 131 by the rotation shaft 143. Thus, the end tool hub 106 and the pulley 131 (coupled thereto) may be formed to be rotatable around the rotation shaft 143 with respect to the pitch hub 107.

Further, the pitch hub 107 may internally accommodate at least some of the pulley 113, the pulley 114, the pulley 123, and the pulley 124 that are axially coupled to the rotation shaft 143. In addition, the pitch hub 107 may internally accommodate at least some of the pulley 115, the pulley 116, the pulley 125, and the pulley 126 that are axially coupled to the rotation shaft 144.

Further, the end tool 100 of the first embodiment of the present disclosure may include the rotation shaft 141, the rotation shaft 142, the rotation shaft 143, and the rotation shaft 144. As described above, the rotation shaft 141 and the rotation shaft 142 may be inserted through the end tool hub 106, and the rotation shaft 143 and the rotation shaft 144 may be inserted through the pitch hub 107.

The rotation shaft 141, the rotation shaft 142, the rotation shaft 143, and the rotation shaft 144 may be arranged sequentially from a distal end 104 of the end tool 100 toward a proximal end 105 thereof. Accordingly, starting from the distal end 104, the rotation shaft 141 may be referred to as a first pin, the rotation shaft 142 may be referred to as a second pin, the rotation shaft 143 may be referred to as a third pin, and the rotation shaft 144 may be referred to as a fourth pin.

Here, the rotation shaft 141 may function as an end tool jaw pulley rotation shaft, the rotation shaft 142 may function as an end tool jaw auxiliary pulley rotation shaft, the rotation shaft 143 may function as an end tool pitch rotation shaft, and the rotation shaft 144 may function as an end tool pitch auxiliary rotation shaft of the end tool 100.

Each of the rotation shafts 141, 142, 143, and 144 may be fitted into one or more pulleys, which will be described in detail below.

The pulley 111 functions as an end tool first jaw pulley, and the pulley 121 functions as an end tool second jaw pulley, and these two components may be collectively referred to as end tool jaw pulleys.

The pulley 111 and the pulley 121, which are end tool jaw pulleys, are formed to face each other, and are formed to be rotatable independently of each other around the rotation shaft 141, which is an end tool jaw pulley rotation shaft. Here, in the drawings, it is illustrated that the pulley 111 and the pulley 121 are formed to rotate around one rotation shaft 141, but it is of course possible that each end tool jaw pulley may be formed to be rotatable around a separate shaft. Here, the first jaw 101 may be fixedly coupled to the pulley 111 and rotated together with the pulley 111, and the second jaw 102 may be fixedly coupled to the pulley 121 and rotated together with the pulley 121. Yaw and actuation motions of the end tool 100 are performed according to the rotation of the pulley 111 and the pulley 121. That is, when the pulley 111 and the pulley 121 are rotated in the same direction around the rotation shaft 141, the yaw motion is performed, and when the pulley 111 and the pulley 121 are rotated in opposite directions around the rotation shaft 141, the actuation motion is performed.

Here, the first jaw 101 and the pulley 111 may be formed as separate members and coupled to each other, or the first jaw 101 and the pulley 111 may be integrally formed as one body. Similarly, the second jaw 102 and the pulley 121 may be formed as separate members and coupled to each other, or the second jaw 102 and the pulley 121 may be integrally formed as one body.

The pulley 112 functions as an end tool first jaw auxiliary pulley, and the pulley 122 functions as an end tool second jaw auxiliary pulley, and these two components may be collectively referred to as end tool jaw auxiliary pulleys.

In detail, the pulley 112 and the pulley 122, which are end tool jaw auxiliary pulleys, may be additionally provided on one side of the pulley 111 and one side of the pulley 121, respectively. In other words, the pulley 112, which is an auxiliary pulley, may be disposed between the pulley 111 and the pulley 113/pulley 114. In addition, the pulley 122, which is an auxiliary pulley, may be disposed between the pulley 121 and the pulley 123/pulley 124. The pulley 112 and the pulley 122 may be formed to be rotatable independently of each other around the rotation shaft 142. Here, in the drawings, it is illustrated that the pulley 112 and the pulley 122 are formed to rotate around one rotation shaft 142, but it is of course possible that each of the pulley 112 and the pulley 122 may be formed to be rotatable around a separate shaft. Such auxiliary pulleys will be described in more detail later.

The pulley 113 and the pulley 114 function as end tool first jaw pitch main pulleys, and the pulley 123 and the pulley 124 function as end tool second jaw pitch main pulleys, and these two components may be collectively referred to as end tool jaw pitch main pulleys.

The pulley 115 and the pulley 116 function as end tool first jaw pitch sub-pulleys, and the pulley 125 and the pulley 126 function as end tool second jaw pitch sub-pulleys, and these two components may be collectively referred to as end tool jaw pitch sub-pulleys.

Hereinafter, components related to the rotation of the pulley 111 will be described.

The pulley 113 and the pulley 114 function as end tool first jaw pitch main pulleys. That is, the pulley 113 and the pulley 114 function as main rotation pulleys for a pitch motion of the first jaw 101. Here, the wire 301, which is a first jaw wire, is wound around the pulley 113, and the wire 305, which is a first jaw wire, is wound around the pulley 114.

The pulley 115 and the pulley 116 function as end tool first jaw sub-pulleys. That is, the pulley 115 and the pulley 116 function as sub rotation pulleys for a pitch motion of the first jaw 101. Here, the wire 301, which is a first jaw wire, is wound around the pulley 115, and the wire 305, which is a first jaw wire, is wound around the pulley 116.

Here, the pulley 113 and the pulley 114 are disposed on one side of the pulley 111 and the pulley 112 to face each other. Here, the pulley 113 and the pulley 114 are formed to be rotatable independently of each other around the rotation shaft 143 that is an end tool pitch rotation shaft. In addition, the pulley 115 and the pulley 116 are disposed on one side of the pulley 113 and on one side of the pulley 114, respectively, to face each other. Here, the pulley 115 and the pulley 116 are formed to be rotatable independently of each other around the rotation shaft 144 that is an end tool pitch auxiliary rotation shaft. Here, in the drawings, it is illustrated that the pulley 113, the pulley 115, the pulley 114, and the pulley 116 are all formed to be rotatable around a Y-axis direction, but the concept of the present disclosure is not limited thereto, and the rotation axes of the respective pulleys may be formed in various directions according to configurations thereof.

The wire 301, which is a first jaw wire, is sequentially wound to make contact with at least portions of the pulley 115, the pulley 113, and the pulley 111. In addition, the wire 305 connected to the wire 301 by the coupling member 323 is sequentially wound to make contact with at least portions of the pulley 111, the pulley 112, the pulley 114, and the pulley 116 in turn.

Viewed from another perspective, the wires 301 and 305, which are first jaw wires, are sequentially wound to make contact with at least portions of the pulley 115, the pulley 113, the pulley 111, the pulley 112, the pulley 114, and the pulley 116 and are formed to move along the above pulleys while rotating the above pulleys.

Accordingly, when the wire 301 is pulled in the direction of an arrow 301 of FIG. 9, a coupling member (not shown) to which the wire 301 is coupled and the pulley 111 coupled to the coupling member (not shown) are rotated in an arrow L direction of FIG. 9. In contrast, when the wire 305 is pulled in the direction of an arrow 305 of FIG. 9, a coupling member (not shown) to which the wire 305 is coupled and the pulley 111 coupled to the coupling member (not shown) are rotated in an arrow R direction of FIG. 9.

Hereinafter, the pulley 112 and the pulley 122 serving as auxiliary pulleys will be described in more detail.

The pulley 112 and the pulley 122 may serve to increase rotation angles of the first jaw 101 and the second jaw 102, respectively, by coming into contact with the wire 305, which is a first jaw wire, and the wire 302, which is a second jaw wire, and changing the arrangement paths of the wires 305 and 302 to a certain extent.

That is, when the auxiliary pulleys are not disposed, each of the first jaw and the second jaw may be rotated up to a right angle, but in an embodiment of the present disclosure, the pulley 112 and the pulley 122, which are auxiliary pulleys, are additionally provided, so that the maximum rotation angle may be increased by θ as shown in FIG. 10. This enables a motion of the two jaws of the end tool 120 being opened for an actuation motion while the two jaws are yaw-rotated by 90° in the L direction. This is because the second jaw 102 is rotated by the additional angle θ as shown in FIG. 10. Similarly, an actuation motion is possible even when the two jaws are yaw-rotated in the R direction. In other words, a feature of increasing the range of yaw rotation in which an actuation motion is possible may be obtained through the pulley 112 and the pulley 122. FIGS. 7 and 8 are perspective views of an end tool of the surgical instrument of FIG. 6. FIGS. 9 and 10 are plan views of the end tool of the surgical instrument of FIG. 6.

This will be described below in more detail.

When the auxiliary pulleys are not disposed, since the first jaw wire is fixedly coupled to the end tool first jaw pulley, and the second jaw wire is fixedly coupled to the end tool second jaw pulley, each of the end tool first jaw pulley and the end tool second jaw pulley may be rotated up to 90°. In this case, when the actuation motion is performed while the first jaw and the second jaw are located at a 90° line, the first jaw may be opened, but the second jaw may not be rotated beyond 90°. Accordingly, when the first jaw and the second jaw perform a yaw motion over a certain angle, there was a problem that the actuation motion is not smoothly performed.

In order to address such a problem, in the surgical instrument 30 of the present disclosure, the pulley 112 and the pulley 122, which are auxiliary pulleys, are additionally disposed at one side of the pulley 111 and one side of the pulley 121, respectively. As described above, as the arrangement paths of the wire 305, which is a first jaw wire, and the wire 302, which is a second jaw wire, are changed to a certain extent by disposing the pulley 112 and the pulley 122, a tangential direction of the wires 305 and 302 is changed, and accordingly, the coupling member 326 for coupling the wire 302 and the pulley 121 may be rotated up to a line N of FIG. 7. That is, the coupling member 326, which is a coupling part of the wire 302 and the pulley 121, is rotatable until the coupling member 326 is located on a common internal tangent of the pulley 121 and the pulley 122. Similarly, the coupling member 323, which is a coupling part of the wire 305 and the pulley 111, is rotatable until the coupling member 323 is located on a common internal tangent of the pulley 111 and the pulley 112, so that the range of rotation in the L direction may be increased.

In other words, by the pulley 112, the wires 301 and 305, which are two strands of the first jaw wire wound around the pulley 111, are disposed at one side with respect to a plane perpendicular to the Y-axis and passing through the X-axis. Simultaneously, by the pulley 122, the wires 302 and 306, which are two strands of the second jaw wire wound around the pulley 121, are disposed at the other side with respect to the plane perpendicular to the Y-axis and passing through the X-axis.

In other words, the pulley 113 and the pulley 114 are disposed at one side with respect to the plane perpendicular to the Y-axis and passing through the X-axis, and the pulley 123 and the pulley 124 are disposed at the other side with respect to the plane perpendicular to the Y-axis and passing through the X-axis.

In other words, the wire 305 is located on the internal tangent of the pulley 111 and the pulley 112, and the rotation angle of the pulley 111 is increased by the pulley 112. In addition, the wire 302 is located on the internal tangent of the pulley 121 and the pulley 122, and the rotation angle of the pulley 121 is increased by the pulley 122.

According to the present disclosure, as the rotation radii of the jaw 101 and the jaw 102 increase, an effect of increasing a yaw motion range in which a normal opening/closing actuation motion is performed may be obtained.

Next, components related to the rotation of the pulley 121 will be described.

The pulley 123 and the pulley 124 function as end tool second jaw pitch main pulleys. That is, the pulley 123 and the pulley 124 function as main rotation pulleys for a pitch motion of the second jaw 102. Here, the wire 306, which is a second jaw wire, is wound around the pulley 123, and the wire 302, which is a second jaw wire, is wound around the pulley 124.

The pulley 125 and the pulley 126 function as end tool second jaw sub-pulleys. That is, the pulley 125 and the pulley 126 function as sub rotation pulleys for a pitch motion of the second jaw 102. Here, the wire 306, which is a second jaw wire, is wound around the pulley 125, and the wire 302, which is a second jaw wire, is wound around the pulley 126.

On one side of the pulley 121, the pulley 123 and the pulley 124 are disposed to face each other. Here, the pulley 123 and the pulley 124 are formed to be rotatable independently of each other around the rotation shaft 143 that is an end tool pitch rotation shaft. In addition, the pulley 125 and the pulley 126 are disposed on one side of the pulley 123 and one side of the pulley 124, respectively, to face each other. Here, the pulley 125 and the pulley 126 are formed to be rotatable independently of each other around the rotation shaft 144, which is an end tool pitch auxiliary rotation shaft. Here, in the drawings, it is illustrated that all of the pulley 123, the pulley 125, the pulley 124, and the pulley 126 are formed to be rotatable around the Y-axis direction, but the concept of the present disclosure is not limited thereto, and the rotation axes of the respective pulleys may be formed in various directions according to configurations thereof.

The wire 306, which is a second jaw wire, is sequentially wound to make contact with at least portions of the pulley 125, the pulley 123, and the pulley 121. In addition, the wire 302 connected to the wire 306 by the coupling member 326 is sequentially wound to make contact with at least portions of the pulley 121, the pulley 122, the pulley 124, and the pulley 126.

Viewed from another perspective, the wires 306 and 302, which are second jaw wires, are sequentially wound to make contact with at least portions of the pulley 125, the pulley 123, the pulley 121, the pulley 122, the pulley 124, and the pulley 126, and are formed to move along the above pulleys while rotating the above pulleys.

Accordingly, when the wire 306 is pulled in the direction of an arrow 306 of FIG. 9, the coupling member 326 to which the wire 306 is coupled and the pulley 121 coupled to the coupling member 326 are rotated in the arrow R direction of FIG. 9. In contrast, when the wire 302 is pulled in the direction of an arrow 302 of FIG. 6, the coupling member 326 to which the wire 302 is coupled and the pulley 121 coupled to the coupling member 326 are rotated in the arrow L direction of FIG. 9.

Hereinafter, a pitch motion of the present disclosure will be described in more detail.

First, for the pitch motion, at the end tool 100 side, the pulley 113, the pulley 114, the pulley 123, and the pulley 124, which are end tool jaw pitch main pulleys, are formed to be rotatable around the rotation shaft 143. Meanwhile, in a direction of the proximal end 105 of the end tool jaw pitch main pulley, the pulley 115, the pulley 116, the pulley 125, and the pulley 126, which are end tool jaw pitch sub-pulleys, are formed to be rotatable around the rotation shaft 144.

In addition, based on a plane perpendicular to the rotation shaft 141 and including the rotation shaft 143 (i.e., an XY plane), the wires 301 and 305, which are two strands of the first jaw wire, are located on the same side with respect to the XY plane That is, the wire 301 and the wire 305 are formed to pass through lower sides of the pulley 113 and the pulley 114, which are end tool jaw pitch main pulleys, and upper sides of the pulley 115 and the pulley 116, which are end tool jaw pitch sub-pulleys.

Similarly, the wires 302 and 306, which are two strands of the second jaw wire, are located on the same side with respect to the XY plane. That is, the wires 302 and 306 are formed to pass through upper sides of the pulley 123 and the pulley 124, which are end tool jaw pitch main pulleys, and lower sides of the pulley 125 and the pulley 126, which are end tool jaw pitch sub-pulleys.

In addition, in the wires 301 and 305 that are two strands of the first jaw wire, when the wire 301 is pulled toward the arrow 301 of FIG. 7 and simultaneously the wire 305 is pulled toward the arrow 305 of FIG. 7 (i.e., when both strands of the first jaw wire are pulled in the same direction), as shown in FIG. 5, since the wires 301 and 305 are wound around lower portions of the pulleys 113 and 114, which are rotatable around the rotation shaft 143 that is an end tool pitch rotation shaft, the pulley 111 to which the wire 301 and the wire 305 are fixedly coupled, and the end tool hub 106 to which the pulley 111 is coupled are rotated together as a whole in a counterclockwise direction around the rotation shaft 143, as a result, the end tool 100 performs the pitch motion while rotating downward. At this time, since the second jaw 102 and the wires 302 and 306 fixedly coupled thereto are wound around the upper portions of the pulleys 123 and 124 rotatable around the rotation shaft 143, the wires 302 and 306 are unwound in opposite directions of the arrows 302 and 306, respectively.

In contrast, in the wires 302 and 306 that are two strands of the second jaw wire, when the wire 302 is pulled toward the arrow 302 of FIG. 7 and simultaneously the wire 306 is pulled toward the arrow 306 of FIG. 7 (i.e., when both strands of the second jaw wire are pulled in the same direction), as shown in FIG. 5, since the wires 302 and 306 are wound around upper portions of the pulleys 123 and 124, vwhich are rotatable around the rotation shaft 143 that is an end tool pitch rotation shaft, the pulley 121 to which the wire 302 and the wire 306 are fixedly coupled, and the end tool hub 106 to which the pulley 121 is coupled are rotated together as a whole in a clockwise direction around the rotation shaft 143, as a result, the end tool 100 performs the pitch motion while rotating upward. At this time, since the first jaw 101 and the wires 301 and 305 fixedly coupled thereto are wound around the lower portions of the pulleys 113 and 114 rotatable around the rotation shaft 143, the wires 302 and 306 are moved in opposite directions of the arrows 301 and 305, respectively.

Viewed from another perspective, it may be also described that both strands of each jaw wire are moved simultaneously in the same direction when the end tool 100 is pitch-rotated.

Meanwhile, the end tool 100 of the surgical instrument 30 of the present disclosure may further include the pulley 131, which is an end tool pitch pulley, the driving part 200 may further include the pulley 231, which is a driving part pitch pulley, and the power transmission part 300 may further include the wire 303 and the wire 304 that are pitch wires. In detail, the pulley 131 of the end tool 100 is rotatable around the rotation shaft 143, which is an end tool pitch rotation shaft, and may be integrally formed with the end tool hub 106 (or fixedly coupled to the end tool hub 106) as one body. In addition, the wires 303 and 304 may serve to connect the pulley 131 of the end tool 100 and the pulley 231 of the driving part 200.

Thus, when the pulley 231 of the driving part 200 is rotated, the rotation of the pulley 231 is transmitted to the pulley 131 of the end tool 100 via the wires 303 and 304, which causes the pulley 131 to also be rotated, and as a result, the end tool 100 performs a pitch motion while rotating.

That is, in the surgical instrument 30 according to the first embodiment of the present disclosure, by providing the pulley 131 of the end tool 100, the pulley 231 of the driving part 200, and the wires 303 and 304 of the power transmission part 300 to transmit power for a pitch motion, the driving force for a pitch motion from the driving part 200 may be more completely transmitted to the end tool 100, thereby improving operation reliability.

Here, a diameter of each of the pulley 113, the pulley 114, the pulley 123, and the pulley 124, which are end tool jaw pitch main pulleys, and a diameter of the pulley 131, which is an end tool pitch pulley, may be the same as each other or different from each other. At this time, a ratio of the diameter of the end tool jaw pitch main pulley to the diameter of the end tool pitch pulley may be the same as a ratio of a diameter of a driving part relay pulley of the driving part 200, which will be described later, to a diameter of a driving part pitch pulley. This will be described in detail below.

### (Driving part)

Hereinafter, the driving part 200 of the surgical instrument 30 of FIG. 6 will be described in more detail.

Referring to FIGS. 15 to 24, the driving part 200 of the surgical instrument 30 according to the first embodiment of the present disclosure may include the pulley 211 and the pulley 212 both associated with a rotational motion of the first jaw 101. The driving part 200 may also include the pulley 221 and the pulley 222 both associated with a rotational motion of the second jaw 102.

Although FIG. 21 illustrates that the pulleys facing each other are arranged in parallel with each other, the present disclosure is not limited thereto, and the pulleys may be formed in various positions and sizes suitable for the configuration of the driving part 200.

The driving part 200 of the surgical instrument 30 according to the first embodiment of the present disclosure may further include the pulley 231 serving as a driving part pitch pulley.

The driving part 200 according to the first embodiment of the present disclosure may include a rotation shaft 241, a rotation shaft 242, and a rotation shaft 243. Here, the rotation shaft 241 may function as a driving part first jaw rotation shaft, and the rotation shaft 242 may function as a driving part second jaw rotation shaft. In addition, the rotation shaft 243 may function as a driving part pitch rotation shaft. One or more pulleys may be fit into each of the rotation shafts 241, 242, and 243, and this will be described in detail below.

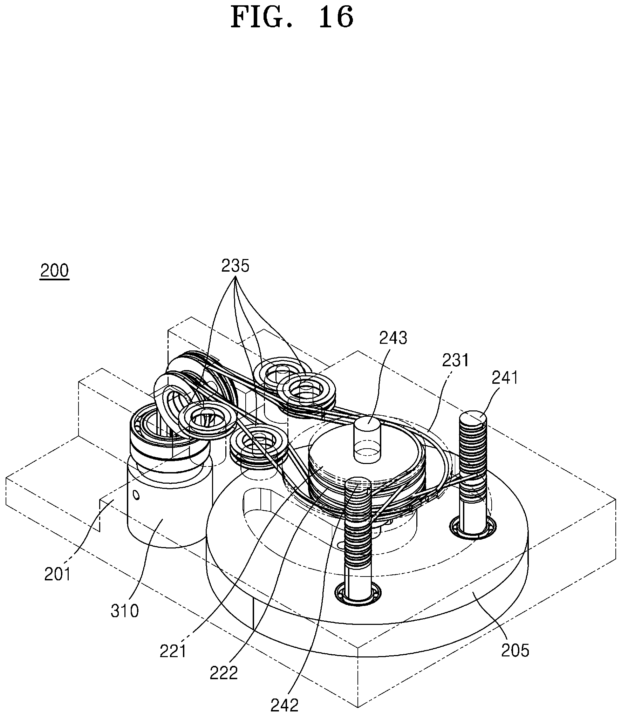

Referring to FIGS. 15 and 16, the driving part 200 according to the first embodiment of the present disclosure may include a base plate 205. The base plate 205 is coupled to the rotation shaft 243 to rotate together with the rotation shaft 243 and the pulley 231. The base plate 205 and the rotation shaft 243 may be variously modified, for example, may be formed as one body, or may be formed independently of each other and then combined with each other.

Referring to FIGS. 15 and 16, the rotation shaft 241, which is a driving part first jaw rotation shaft, and a rotation shaft 242, which is a driving part second jaw rotation shaft, may be coupled to the base plate 205. Accordingly, when the base plate 205 rotates around the rotation shaft 243, the rotation shaft 241 and the rotation shaft 242 are revolved around the rotation shaft 243.

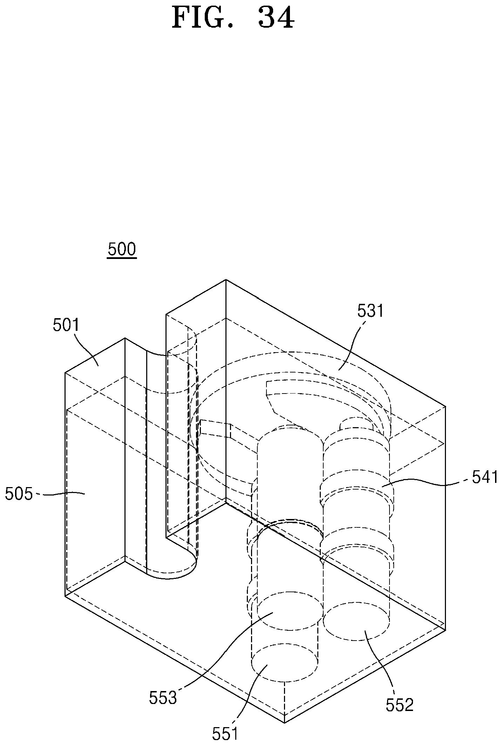

FIG. 22 is a bottom perspective view illustrating the driving part of FIG. 15, and at least one coupling hole 205a in the shape of a hole, to which a pitch driving motor (see 553 of FIG. 34) may be coupled, may be formed on the bottom surface of the base plate 205, and accordingly, the base plate 205 may connect the driving part 200 to the motor pack 500, and may specifically function as a pitch driving motor coupling part.

Referring to FIG. 22, the driving part 200 of the first embodiment of the present disclosure may include a motor coupling part 251 and a motor coupling part 252. Here, the motor coupling part 251 may function as a first jaw driving motor coupling part, and the motor coupling part 252 may function as a second jaw driving motor coupling part.

Referring to FIGS. 21 and 22, the motor coupling part 251 may be formed in the shape of a rotatable plate, and may have formed thereon at least one coupling hole 251a into which a first jaw driving motor (see 551 of FIG. 34) may be coupled. The motor coupling part 252 may be formed in the shape of a rotatable plate, and may have formed thereon at least one coupling hole 252a into which a second jaw driving motor (see 552 of FIG. 34) may be coupled.

Referring to FIGS. 21 and 22, pairs of coupling holes 205a, 251a, and 252a are formed. Accordingly, the base plate 205, the motor coupling part 251, and the motor coupling part 252, which are coupled to a pitch driving motor 553, a first jaw driving motor 551, and a second jaw driving motor 552, respectively, may receive power from the motor pack 500, specifically the pitch driving motor 553, the first jaw driving motor 551, and the second jaw driving motor 552.

That is, the base plate 205, the motor coupling part 251, and the motor coupling part 252 of the driving part 200 may receive power by driving of the motor pack 500 arranged in each of the robotic arm units 21, 22, and 23, and the driving part 200 may be operated.

Hereinafter, each component will be described in more detail.

Referring to FIGS. 15 and 21, the pulley 211 and the pulley 212 function as driving part first jaw pulleys, the pulley 221 and the pulley 222 function as driving part second jaw pulleys, and these components may be collectively referred to as driving part jaw pulleys.

Here, the drawings illustrate that the pulley 211 is associated with a rotational motion of the first jaw 101 of the end tool 100, and the pulley 221 is associated with a rotational motion of the second jaw 102 of the end tool 100, but the present disclosure is not limited thereto. For example, one group of pulleys within the driving part may be associated with a yaw motion, and another group of pulleys may be associated with an actuation motion. Thus, the pulley 211 and the pulley 212 may be collectively referred to as driving part driving pulleys. In addition, in the other pulleys, one group of pulleys may be associated with a yaw motion, and another group of pulleys may be associated with an actuation motion.

Referring to FIG. 15, a case 201 is arranged to face the motor pack 500, one side thereof (the upper side in FIG. 15) may be connected to the instrument case 40, and the opposite side (the lower side in FIG. 15) may be connected to the motor pack 500.

The base plate 205 may be arranged inside the case 201, and the base plate 205 inside the case 201 may rotate clockwise or counterclockwise around the rotation shaft 243.

A plurality of rotation shafts including the rotation shaft 241, the rotation shaft 242, and the rotation shaft 243 may be formed on a first surface of the base plate 205. In addition, a plurality of relay pulleys 235 may be formed on the first surface of the base plate 205, to serve to redirect, toward the pulley 231, the wires 301, 302, 303, 304, 305, and 306 entering the driving part 200 through the connection part 310.

In detail, the plurality of relay pulleys 235 may guide the wires 301, 302, 303, 304, 305, and 306 moving parallel to the longitudinal central axis of the connection part 310 inside the connection part 310 extending in a direction parallel to the rotation shaft 243, such that they may be introduced into the pulley 231, the pulley 211, the pulley 212, the pulley 221, and the pulley 222, which are formed on a plane perpendicular to the rotation shaft 243.

The plurality of relay pulleys 235 are arranged adjacent to the pulley 211, the pulley 212, the pulley 221, and the pulley 222, which are driving part jaw pulleys, and may transfer the jaw wire from the end tool 100 to the pulley 211, the pulley 212, the pulley 221, and the pulley 222, which are driving part jaw pulleys. The plurality of relay pulleys 235 may function as driving part sub-relay pulleys.

The plurality of driving part sub-relay pulleys may be formed to have a diameter equal to or different from the diameter of the driving part jaw pulleys.

The jaw wire may sequentially pass through the pulley 235, which is a driving part sub-relay pulley, and the pulley 211, the pulley 212, the pulley 221, and the pulley 222, which are driving part jaw pulleys, and the rotation shaft 241 and the rotation shaft 242, which are driving part jaw rotation shafts.

The plurality of relay pulleys 235 may be arranged at different heights. Accordingly, it is possible to prevent interference during movement of the wires 301, 302, 303, 304, 305, and 306, which are formed to be wound at least in part around the plurality of relay pulleys 235.

In addition, the connection part 310 in the shape of a shaft may be coupled to a second surface (the lower surface in FIG. 15) of the base plate 205, and A motor coupling part 252 and a motor coupling part 253 to which the motor pack 500 for driving the pulleys is coupled may be formed on the second surface. Here, the motor coupling parts 251 and 252 may be respectively connected to the rotation shafts 241 and 242 directly or indirectly through gears.

For example, by directly coupling the motor coupling part 251, which is a first jaw driving motor coupling part, to the rotation shaft 241, which is a driving part first jaw rotation shaft, when the motor coupling part 251 coupled to a first jaw driving motor (see 551 of FIG. 34) is rotated, the rotation shaft 241 directly coupled to the motor coupling part 251 may be rotated together. Similarly, by directly coupling the motor coupling part 252, which is a second jaw driving motor coupling part, to the rotation shaft 242, which is a driving part second jaw rotation shaft, when the motor coupling part 252 coupled to a second jaw driving motor (see 552 of FIG. 34) is rotated, the rotation shaft 242 directly coupled to the motor coupling part 252 may be rotated together.

By directly coupling the base plate 205, which is a pitch driving motor coupling part, to the rotation shaft 243, which is a driving part pitch rotation shaft, when the base plate 205 coupled to a pitch driving motor (see 553 of FIG. 34) is rotated, the rotation shaft 243 directly coupled to the base plate 205 may be rotated together.

Meanwhile, although not illustrated in the drawings, a motor coupling part and a rotation shaft may be arranged to be spaced apart from each other by a certain extent when viewed on a plane perpendicular to the rotation shaft. In addition, the motor coupling part and the rotation shaft may be connected to each other by a driving gear.

As such, some motor coupling parts are configured to be directly connected to the rotation shafts, respectively, and the other motor coupling parts are configured to be indirectly connected to the rotation shafts, respectively, because the coupling position and direction between the surgical instrument 30 and the surgical robot 20 need to be considered. That is, the rotation shaft that is not affected by the coupling position with the surgical robot 20 may be directly connected to the motor coupling part, whereas the rotation shaft that may cause interference with the coupling position with the surgical robot 20 may be indirectly connected to the motor coupling part.

The rotation shaft 241 is coupled to the motor pack 500, specifically the first jaw driving motor (see 551 of FIG. 34) by the motor coupling part 251, and thus, when the first jaw driving motor (see 551 of FIG. 34) rotates for driving of the first jaw 101, the rotation shaft 241 rotates such that the wire 301 and the wire 305, which constitute the first jaw wire, are pulled or released.

As described above, the rotation shaft 242 is coupled to the motor pack 500, specifically the second jaw driving motor (see 552 of FIG. 34) by the motor coupling part 252, and thus, when the second jaw driving motor (see 552 of FIG. 34) rotates for driving of the second jaw 102, the rotation shaft 242 rotates such that the wire 302 and the wire 306, which constitute the second jaw wire, are pulled or released.

The pulley 231, which is a driving part pitch pulley, may be coupled to the rotation shaft 243 that is a driving part pitch rotation shaft. Here, the pulley 231 may be formed to rotate together with the rotation shaft 243.

As described above, the rotation shaft 243 is coupled to the motor pack 500, specifically the pitch driving motor (see 553 of FIG. 34) by the base plate 205, and thus, when the pitch driving motor (see 553 of FIG. 34) rotates for a pitch motion, the pulley 231, which is a driving part pitch pulley, rotates around the rotation shaft 243 such that the wire 303 and the wire 304, which are pitch wires, are pulled or released.

Meanwhile, the pulley 211, the pulley 212, the pulley 221, and the pulley 222, which are driving part jaw pulleys, may be rotatably coupled to the rotation shaft 243. Here, the pulley 211 and the pulley 212, which are driving part first jaw pulley, may be arranged on one surface side of the pulley 231 that is a driving part pitch pulley, and the pulley 221 and the pulley 222, which are driving part second jaw pulley, may be arranged on the other surface side of the pulley 231 that is the driving part pitch pulley.

In order words, the pulley 221 and the pulley 222, which are driving part second jaw pulleys, the pulley 231, which is a driving part pitch pulley, and the pulley 212 and the pulley 211, which are driving part first jaw pulleys, may be sequentially stacked and formed along the rotation shaft 243.

When the base plate 205 and the pulley 231, which is a driving part pitch pulley, rotate together with the rotation shaft 243, the rotation shaft 241 and the rotation shaft 242 coupled to the base plate 205 revolve around the rotation shaft 243.

In other words, it may also be described that, in a state in which the rotation shaft 241 and the rotation shaft 242 are spaced apart from the rotation shaft 243 by a certain extent, the rotation shaft 241 and the rotation shaft 242 rotate as a whole around the rotation shaft 243 while the rotation shaft 241 and the rotation shaft 242 maintain a constant distance from the rotation shaft 243.

That is, the rotation shaft 241 and the rotation shaft 242 are formed to be relatively movable with respect to the pulley 231, which is a driving part pitch pulley, such that the relative positions of the rotation shaft 241 and the rotation shaft 242 with respect to the pulley 231, which is a driving part pitch pulley, may be changed. On the contrary, the relative positions of the driving part pitch pulley 231 and the driving part jaw pulleys 211, 212, 221, and 222 are kept constant.

In addition, when the pulley 231, which is a driving part pitch pulley, rotates around the rotation shaft 243, the rotation shaft 241 and the rotation shaft 242 move relative to the pulley 231, which is a driving part pitch pulley, and thus, the overall lengths of the wire 301, the wire 302, the wire 305, and the wire 306, which are jaw wires, within the driving part 200 are changed.

Referring to FIGS. 21 and 23, the wire 301, which constitutes the first jaw wire, is connected to the end tool 100 through the connection part 310 after being wound to come into contact with the pulley 211 at least in part, in a state in which one end of the wire 301 is coupled to the rotation shaft 241 by a coupling member (not shown).

In other words, the wire 301, which constitute the first jaw wire, enters the driving part 200 after passing through the end tool 100 and the connection part 310, then is wound around the pulley 211, and then fixedly coupled to the rotation shaft 241.

Meanwhile, the wire 305, which constitutes the first jaw wire, is connected to the end tool 100 through the connection part 310 after being wound to come into contact with the pulley 212 at least in part, in a state in which one end of the wire 305 is coupled to the rotation shaft 241 by a coupling member (not shown).

Referring to FIGS. 21 and 24, the wire 302, which constitutes the second jaw wire, is connected to the end tool 100 through the connection part 310 after being wound to come into contact with the pulley 222 at least in part, in a state in which one end of the wire 302 is coupled to the rotation shaft 242 by a coupling member (not shown).

Meanwhile, the wire 306, which constitutes the second jaw wire, is connected to the end tool 100 through the connection part 310 after being wound to come into contact with the pulley 221 at least in part, in a state in which one end of the wire 306 is coupled to the rotation shaft 242 by a coupling member (not shown).

### (Pitch motion)

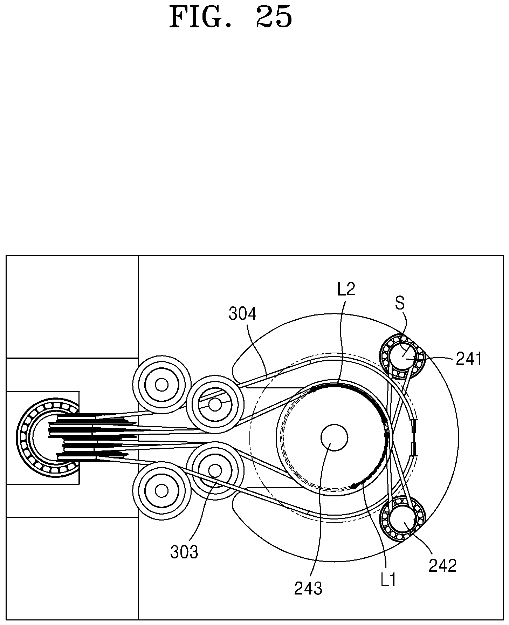

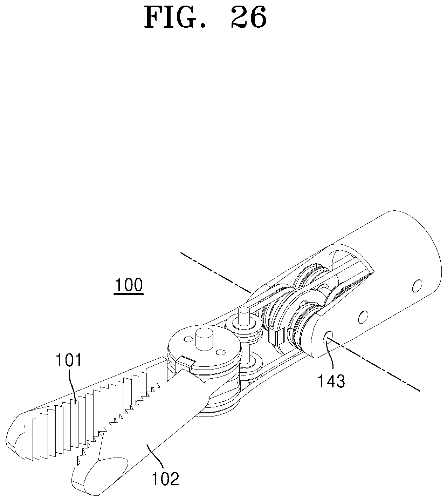

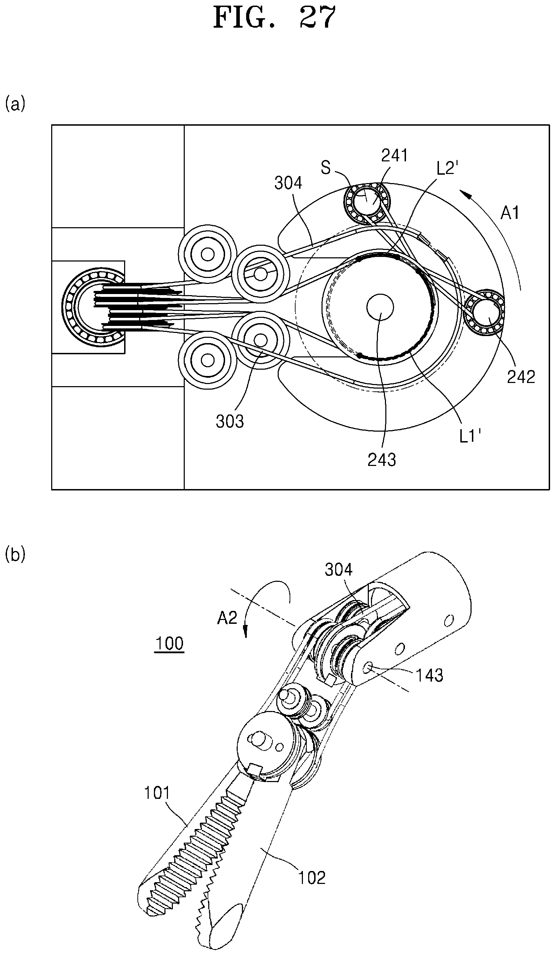

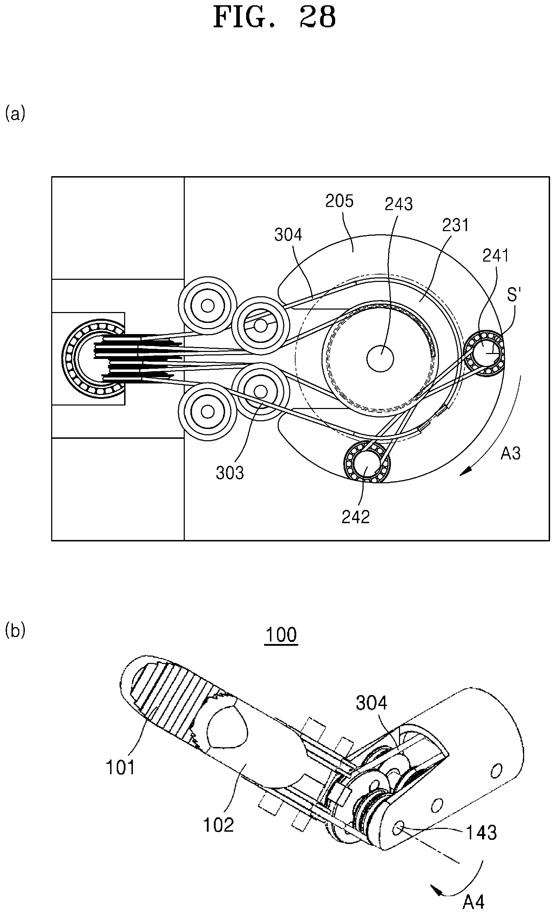

FIG. 25 is a plan view illustrating the driving part of the surgical instrument of FIG. 6. FIG. 26 is a diagram illustrating an end tool of the surgical instrument of FIG. 25. FIG. 27 is a diagram illustrating a driving part and an end tool during a pitch motion of the surgical instrument of FIGS. 25 and 26. FIG. 28 is a diagram illustrating the driving part and the end tool of FIGS. 25 and 26 during a pitch motion in the opposite direction to that in FIG. 27.

Here, in the surgical instrument 30 according to an embodiment of the present disclosure, when the rotation shaft 241 and the rotation shaft 242 revolve around the rotation shaft 243, the overall length of the jaw wire within the driving part 200 is changed, and thus, a pitch motion of the end tool 100 is performed.

In particular, in the surgical instrument 30 according to an embodiment of the present disclosure, when the pulley 231, which is a driving part pitch pulley, rotates, the rotation shaft 241 and the rotation shaft 242 revolve around the rotation shaft 243 of the pulley 231, which is the driving part pitch pulley, such that a path length of the jaw wire wound around the driving part relay pulley and the driving part jaw pulley is changed, and thus, a pitch motion of the end tool is performed.

In detail, when the base plate 205 rotates for a pitch motion, both strands of any one jaw wire are pulled simultaneously, and both strands of the other jaw wire are released simultaneously, such that the overall length of each jaw wire within the driving part 200 is changed, the overall length of each jaw wire within the end tool 100 is also changed accordingly, and thus, the end tool 100 rotates around the rotation shaft 143 to perform a pitch motion.

In other words, when the end tool performs a pitch motion by the rotation of the driving part pitch pulley, the jaw wire (responsible for a yaw motion and an actuation motion) is also moved by the pitch motion. That is, as the pitch rotation is performed around the rotation shaft 143 of the end tool 100, both strands of the jaw wire coupled to one jaw are pulled, and both strands thereof coupled to the other jaw are released.

Accordingly, it may also be described that, in the present disclosure, in order to compensate for the movement of the jaw wire, when the pitch motion of the end tool is performed, the overall length of the jaw wire within the driving part is changed as the rotation shaft 241 and the rotation shaft 242 revolve around the rotation shaft of the driving part pitch pulley, such that the jaw wire is released (or pulled) on the side of the end tool as much as the jaw wire is pulled (or released) on the side of the driving part, thereby compensating for the movement of the jaw wire when the pitch motion of the end tool is performed.

FIG. 11 is a diagram illustrating a neutral state in a related-art surgical instrument. (a) and (b) of FIG. 11 are perspective views of an end tool corresponding to FIGS. 7 and 8, and (c) of FIG. 11 is a diagram illustrating a neutral state of a driving part 200' for compensating for a movement of a jaw wire when performing a pitch motion of the end tool in a manner different from the present disclosure.

Referring to (a) and (b) of FIG. 11, a pulley 131' of an end tool 100' is rotatable around a rotation shaft 143', which is a pitch main rotation shaft, and may be formed integrally with an end tool hub 106' (or to be fixedly coupled to the end tool hub 106).

In addition, a wire 303' and a wire 304' may serve to connect the pulley 131' of the end tool 100' to a pulley 231'/pulley 232', which are driving part pitch pulleys.

Thus, when the pulley 231'/pulley 232', which are driving part pitch pulleys, rotate, the rotation of the pulley 231 '/pulley 232', which are driving part pitch pulleys, is transmitted to the pulley 131' of the end tool 100' through the wire 303' and the wire 304' such that the pulley 131' is rotated together therewith, and accordingly, the end tool 100' performs a pitch motion while rotating.

However, there was a problem that, when the pulley 231'/pulley 232', which are driving part pitch pulleys, rotate to perform the pitch motion as described above, and the driving part does not perform separate pitch compensation for the jaw wires, the pitch motion itself is impossible. That is, two strands of wires wound around any one jaw pulley are arranged above a pitch shaft, and when performing a pitch motion, these two strands of wires are pulled or released simultaneously. Similarly, two strands of wires wound around another jaw pulley are arranged below the pitch shaft, and when performing a pitch motion, these two strands of wires are released or pulled simultaneously. Thus, in this structure, when separate pitch compensation for a jaw wire is not performed by the driving part, the pitch motion itself is impossible.

FIG. 12 is a diagram illustrating the surgical instrument of FIG. 11 performing pitch compensation.

Referring to (c) of FIG. 12, the pulley 231'/pulley 232', which are driving part pitch pulleys, rotate clockwise (in FIG. 12) around a central rotation axis in order to perform a pitch motion of the end tool, a wire 302' and a wire 306' are pulled from the driving part 200' toward the end tool 100', and a wire 301' and a wire 305' are pulled from the driving part 200' toward the end tool 100'.

As such, when the wire 302' and the wire 306' move in the same direction, compensation for the pitch motion cannot be performed simply through rotation of a pulley 221'/pulley 222', which are driving part second jaw pulleys.

Thus, as illustrated in (c) of FIG. 12, pitch compensation may be performed by changing the positions of the pulley 221'/pulley 222', which are driving part second jaw pulleys, to change the path length of the second jaw wire within the driving part 200', and simultaneously changing the positions of a pulley 211'/pulley 212', which are driving part first jaw pulleys, to change the path length of the first jaw wire within the driving part 200'.

That is, in order to compensate for the movement of the jaw wires during pitch drive, the pulley 221'/pulley 222', which are driving part second jaw pulleys, move toward the end tool 100', and the pulley 211'/pulley 212', which are driving part first jaw pulleys, move in the opposite direction.

There is a problem that a separate space and structure are required for moving the pulley 221'/pulley 222', which are driving part second jaw pulleys, and the pulley 211'/pulley 212', which are driving part first jaw pulleys, in straight directions in an internal space of the driving part 200'.

FIG. 13 is a conceptual diagram of pitch motion compensation of the surgical instrument of FIG. 11. (a) of FIG. 13 is a conceptual diagram illustrating a neutral state of the driving part 200' of the surgical instrument, and (b) of FIG. 13 is a conceptual diagram illustrating pitch motion compensation in (a) of FIG. 13.

(b) of FIG. 13 illustrates a situation in which a pitch motion and pitch compensation are performed in a situation opposite to FIG. 12, and the pulley 231'/pulley 232', which are driving part pitch pulleys rotate counterclockwise (in FIG. 12) around the central rotation axis in order to perform a pitch motion of the end tool, the wire 301' and the wire 305' are pulled from the driving part 200' toward the end tool 100', and the wire 302' and the wire 306' are pulled from the end tool 100' toward the driving part 200'.

As such, when the wire 301' and the wire 305' move in the same direction, compensation for the pitch motion cannot be performed simply through rotation of a pulley 211'/pulley 212', which are driving part first jaw pulleys.

Thus, as illustrated in (b) of FIG. 13, pitch compensation may be performed by changing, by -△S_{pitch}, the positions of the pulley 211'/pulley 212', which are driving part first jaw pulleys, to change the path length of the first jaw wire within the driving part 200', and simultaneously changing the positions of a pulley 221'/pulley 222', which are driving part second jaw pulleys, to change the path length of the second jaw wire within the driving part 200'.

As described above, also in this case, there is a problem that a separate space and structure are required for moving the pulley 221'/pulley 222', which are driving part second jaw pulleys, and the pulley 211'/pulley 212', which are driving part first jaw pulleys, in straight directions in an internal space of the driving part 200'.

FIG. 14 is a conceptual diagram of pitch motion compensation of the surgical instrument of FIG. 6.

(a) of FIG. 14 illustrates a neutral state of the surgical instrument 30, and the rotation shaft 241 connected to the first jaw wire, and the rotation shaft 242 connected to the second jaw wire may be formed to maintain a constant distance from the driving part pitch pulley 231.

Referring to (b) of FIG. 14, when the pulley 231, which is a driving part pitch pulley, rotates counterclockwise (in FIG. 14), the wire 301 and the wire 305, which constitute the first jaw wire, are pulled from the driving part 200 toward the end tool 100, and the wire 302 and the wire 306, which constitute the second jaw wire, are pulled from the end tool 100 toward the driving part 200.

Referring to (b) of FIG. 14, in the driving part 200 according to the first embodiment of the present disclosure, when the pulley 231, which is a driving part pitch pulley, rotates, the rotation shaft 241, which is a driving part first jaw rotation shaft coupled to the first jaw wire, and the rotation shaft 242, which is a driving part second jaw rotation shaft coupled to the second jaw wire, revolve around the pulley 231, which is a driving part pitch pulley.

As the rotation shaft 241 and the rotation shaft 242 revolve around the pulley 231, which is a driving part pitch pulley, the overall lengths of the first jaw wire and the second jaw wire within the driving part 200 are changed, and thus compensation for the pitch motion is performed.

That is, in order to perform motion compensation for the pitch motion as described above, in the surgical instrument 30 according to an embodiment of the present disclosure, the rotation shaft 241 and the rotation shaft 242 revolve simultaneously as the driving part pitch pulley 231 rotates, such that the movement of the jaw wires by the rotation of the driving part pitch pulley is compensated for.

That is, the jaw wire wound on the side of the end tool 100 by rotation of the pulley 231, which is a driving part pitch pulley, is released by the same amount on the side of the driving part 200, and the jaw wire unwound on the side of the end tool 100 is wound by the same amount on the side of the driving part 200, such that the pitch motion does not affect a yaw motion.

In other words, when the end tool performs a pitch motion by the rotation of the driving part pitch pulley, the jaw wire (responsible for a yaw motion and an actuation motion) is also moved by the pitch motion. That is, as the pitch rotation is performed around the rotation shaft 143 of the end tool 100, both strands of the jaw wire coupled to one jaw are pulled, and both strands thereof coupled to the other jaw are released.

Thus, it may be described that, in the present disclosure, in order to compensate for the movement of the jaw wire, when the pitch motion of the end tool is performed, the overall length of the jaw wire within the driving part is changed while the driving part jaw rotation shaft is moved relative to the driving part pitch pulley, such that the jaw wire is released (or pulled) on the side of the end tool as much as the jaw wire is pulled (or released) on the side of the driving part, thereby compensating for the movement of the jaw wire when the pitch motion of the end tool is performed.

Hereinafter, the pitch motion will be described in more detail.

Referring to FIGS. 25 and 27, when the base plate 205 and the pulley 231, which is a driving part pitch pulley, rotate in the direction of an arrow A1 for a pitch motion, the rotation shaft 241 and the rotation shaft 242 revolve as a whole around the rotation shaft 243 in the direction of A1.

In order words, it may also be described that, when the pulley 231, which is a driving part pitch pulley, rotates, the rotation shaft 241 and the rotation shaft 242 move in conjunction with the pulley 231, which is the driving part pitch pulley.

Meanwhile, at this time, the positions of the pulley 211, the pulley 212, the pulley 221, and the pulley 222, which are driving part jaw pulleys coupled to the rotation shaft 243, are not changed.

In addition, as the rotation shaft 241 and the rotation shaft 242 revolve in this way, the relative positions of the rotation shaft 241 and the rotation shaft 242 with respect to the driving part jaw pulleys are changed, and accordingly, the length of each wire wound around the driving part jaw pulley, that is, the path length, is changed.

That is, as compared to a path length L1 by which the wire 301 and the wire 305, which constitute the first jaw wire, are wound around the driving part jaw pulleys at the position in FIG. 25, a path length L1' by which the first jaw wire is wound around the driving part jaw pulleys at the position in FIG. 27 increases, and the first jaw wire is further wound on the side of the driving part 200 as much as the path length increases (L1' - L2). That is, the overall lengths of the wire 301 and the wire 305, which constitute the first jaw wire, within the driving part 200 increases. In addition, as the overall length of the first jaw wire within the driving part 200 increases, the overall length of the first jaw wire within the end tool 100 decreases as much as the first jaw wire is pulled.

On the contrary, when the pulley 231, which is a driving part pitch pulley, rotates in the direction of the arrow A1, as compared to a path length L2 by which the second jaw wire is wound around the driving part relay pulley at the position in FIG. 25, a path length by which the second jaw wire is wound around the driving part relay pulley at the position in FIG. 26 decreases, and the second jaw wire is further unwound on the side of the driving part 200 as much as the path length decreases (L2 - L2'). That is, the overall lengths of the wire 302 and the wire 306, which constitute the second jaw wire, within the driving part 200 decreases. In addition, as the overall length of the second jaw wire within the driving part 200 decreases, the overall length of the second jaw wire within the end tool 100 increases as much as the second jaw wire is pulled.

As such, when the pulley 231, which is a driving part pitch pulley, rotates in the direction of the arrow A1 for a pitch motion, the rotation shaft 241 and the rotation shaft 242 move relative to the driving part pitch pulley and the driving part jaw pulley, and thus, their relative positions are changed. In addition, due to the relative movement of the rotation shaft 241 and the rotation shaft 242, the overall length of the first jaw wire within the driving part 200 decreases, and the overall length of the first jaw wire within the end tool 100 increases. At the same time, due to the relative movement of the rotation shaft 241 and the rotation shaft 242, the overall length of the second jaw wire within the driving part 200 increases, and the overall length of the second jaw wire within the end tool 100 decreases.

Accordingly, when viewed from the side of the end tool 100, when the pulley 231, which is a driving part pitch pulley, rotates in the direction of the arrow A1, the wire 301 and the wire 305, which are two strands of the first jaw wire, are pulled and the wire 302 and the wire 306, which are two strands of the second jaw wire, are released, such that the end tool 100 performs a pitch motion in the direction of an arrow A2 around the rotation shaft 143.

Meanwhile, as the above-described path lengths are changed as the rotation shaft 241 and the rotation shaft 242 move relative to the driving part jaw pulley, the overall length of the jaw wire within the driving part 200 is also changed. In addition, as the overall length of the jaw wire within the driving part 200 is changed, the overall length of the jaw wire within the end tool 100 is also changed.

However, it may be described that, because the overall length of the jaw wire within the end tool 100 also decreases (or increases) as much as the overall length of the jaw wire within the driving part 200 increases (decreases), the overall length of the jaw wire is not changed (assuming that elastic deformation or the like is not considered).

Accordingly, when the pulley 231, which is a driving part pitch pulley, rotates, the wire 301/wire 305, which constitute the first jaw wire, are released on the side of the driving part 200 as much as the wire 301/wire 305, which constitute the first jaw wire, are pulled on the side of the end tool 100, and as a result, a pitch motion is enabled.

Referring to FIG. 28, when the pulley 231, which is a driving part pitch pulley, rotates in the direction of an arrow A3, the wire 301 and the wire 305, which are two strands of the first jaw wire, are released, and the wire 302 and the wire 306, which are two strands of the second jaw wire, are pulled, such that the end tool 100 perform a pitch motion in the direction of an arrow A4 (in (b) of FIG. 28) around the rotation shaft 143.

Meanwhile, as described above, the end tool 100 of the surgical instrument 30 of the present disclosure may further include the pulley 131, which is an end tool pitch pulley, the driving part 200 may further include the pulley 231, which is a driving part pitch pulley, and the power transmission part 300 may further include the wire 303 and the wire 304, which are pitch wires.

Thus, when the pulley 231, which is a driving part pitch pulley, rotates in the direction of the arrow A1, the wire 304 is unwound from the pulley 231 and the wire 303 is wound around the pulley 231, by the rotation of the pulley 231. Accordingly, as the pulley 131, which is an end tool pitch pulley connected to the opposite side of the wire 303 and the wire 304, rotates in the direction of the arrow A2 around the rotation shaft 143, the pitch motion may be more surely and reliably performed.

### (Size ratio of pulleys)

Here, among the pulleys that rotates around the rotation shaft 143, which is an end tool pitch rotation shaft, the pulley 131, which is an end tool pitch pulley in contact with the wire 303 and the wire 304, which are pitch wires, may be formed to have a diameter different from those of the pulley 113, the pulley 114, the pulley 123, and the pulley 124, which are end tool jaw pitch main pulleys in contact with the wire 301, the wire 305, the wire 302, and the wire 306, which are jaw wires.

In this case, when the rotation shaft 143 rotates, the lengths by which the respective wires are wound around or unwound from the respective pulleys are different from each other. For example, when the diameter of the end tool pitch pulley is 6ϕ, the diameter of the end tool jaw pitch main pulley is 4ϕ, and the rotation shaft 143 is rotated by 90°, the length by which the pitch wire is wound around the end tool pitch pulley may be 1.5π, whereas the length by which the jaw wire is wound around the end tool jaw pitch main pulley may be 1π.

From this perspective, the 'length' by which the wire is wound around or unwound from the pulley may be defined as a 'rotation amount'. The rotation amount is a concept different from a rotation angle, and may be calculated as (diameter*rotation angle/360°*π).

In this case, because basically the pulley 231, which is a driving part pitch pulley, is directly connected to the pulley 131, which is an end tool pitch pulley, by the wire 303 and the wire 304, which are pitch wires, the rotation amount of the driving part pitch pulley is equal to that of the end tool pitch pulley. That is, the pitch wire is released from or wound around the end tool pitch pulley as much as the pitch wire is wound around or unwound from the driving part pitch pulley.

Meanwhile, the relationship of (diameter of end tool pitch pulley:diameter of end tool jaw pitch main pulley) = (rotation amount of wire wound around end tool pitch pulley:rotation amount of wire wound around end tool jaw pitch main pulley) may be satisfied.

As described above, when, in the end tool 100, the length by the pitch wire is wound around the end tool pitch pulley is different from the length by which the jaw wire is wound around the end tool jaw pitch main pulley, in the driving part 200, the length by which the pitch wire is to be unwound needs to be different from the length by which the jaw wire is to be unwound, by the same proportion.

To this end, the relationship of (diameter of end tool pitch pulley:diameter of end tool jaw pitch main pulley)=(diameter of driving part pitch pulley:diameter of driving part jaw pulley) may be satisfied.

For example, when the ratio of (diameter of end tool pitch pulley:diameter of end tool jaw pitch main pulley) is 6:4, the ratio of (diameter of driving part pitch pulley:diameter of driving part jaw pulley) may also be 6:4. According to this ratio, the diameter of the driving part pitch pulley may be 9ϕ, and the diameter of the driving part jaw pulley may be 6ϕ.

The final pitch motion process will be described again as follows.

Hereinafter, an example will be described in which the diameter of the end tool pitch pulley is 6ϕ, the diameter of the end tool jaw pitch main pulley is 4ϕ, the diameter of the driving part pitch pulley is 9ϕ, and the diameter of the driving part jaw pulley is 6ϕ.

First, for a pitch motion, the pulley 231, which is a driving part pitch pulley of the driving part 200, rotates by 60° to wind the wire 304, which is a pitch wire, while unwinding the wire 303. At this time, the lengths by which the wire 303/wire 304 are respectively wound and unwound is 1.5π.

Accordingly, as the wire 304 is pulled by 1.5π and the wire 303 is released by 1.5π in the end tool 100, the pulley 131, which is an end tool pitch pulley, rotates by 90° corresponding to 1.5π.

Meanwhile, when the pulley 131 pitch-rotates around the rotation shaft 143, the jaws 101 and 102 and the pulley 111/pulley 112 also pitch-rotate around the rotation shaft 143. Accordingly, both the wire 301 and the wire 305, which constitute the first jaw wire coupled to the pulley 111, are pulled, and both the wire 302 and the wire 306, which constitute the second jaw wire coupled to the pulley 121, are released. At this time, the angles by which the end tool pitch pulley and the end tool jaw pitch main pulley rotate are equal to each other, i.e., 90°, and thus, the lengths by which the jaw wires are wound around or unwound from the end tool jaw pitch main pulley are 1 rr.

That is, the wire 301 and the wire 305, which constitute the first jaw wire, are released on the side of the driving part 200 as much as the wire 301 and the wire 305 are pulled on the side of the end tool 100, and thus, the movement of the jaw wire due to the pitch motion is compensated for. Similarly, the wire 302 and the wire 306, which constitute the second jaw wire, are released on the side of the driving part 200 as much as the wire 302 and the wire 306 are pulled on the side of the end tool 100, and thus, the movement of the jaw wire due to the pitch motion is compensated for.

Accordingly, by releasing (or pulling) the jaw wires on the side of the driving part 200 by a length equal to the length by which the jaw wires are wound (or unwound) on the side of the end tool 100 according to the pitch motion, the pitch motion may be performed independently without affecting rotation of the jaw around the yaw shaft.

That is, the pulley 231, which is a driving part pitch pulley, is rotatable along the rotation shaft 243 together with the base plate 205, the rotation shaft 241 and the rotation shaft 242 are coupled and rigidly connected to the base plate 205, and when the driving part pitch pulley rotates around the rotation shaft 243, the rotation shaft 241 and the rotation shaft 242 revolve around the rotation shaft 243, and thus change the path length of the jaw wire wound around the driving part jaw pulley. In addition, the change in the path length of the jaw wire compensates for the movement of the jaw wires on the side of the end tool due to the pitch motion, and as a result, the pitch motion may be independently performed.

### (Yaw motion)

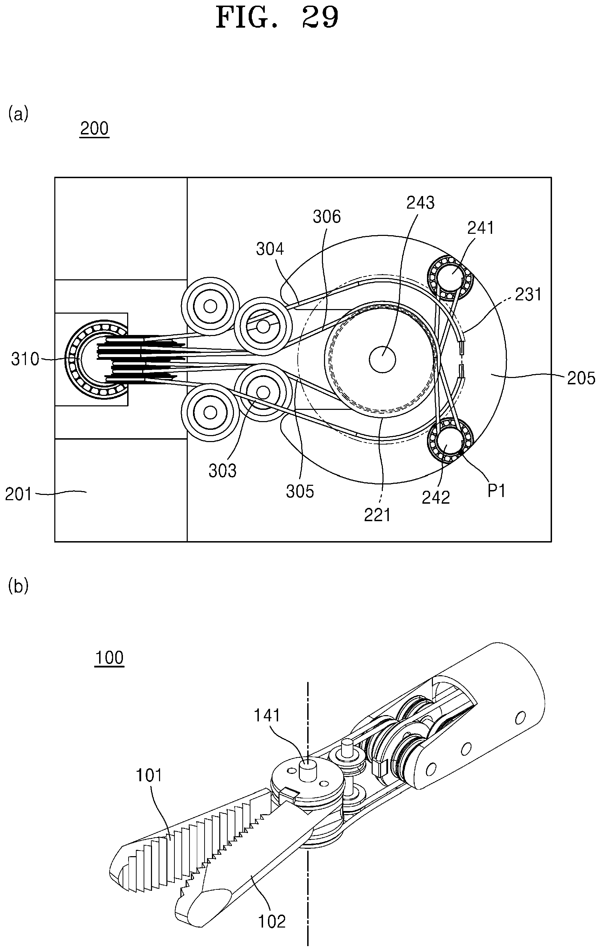

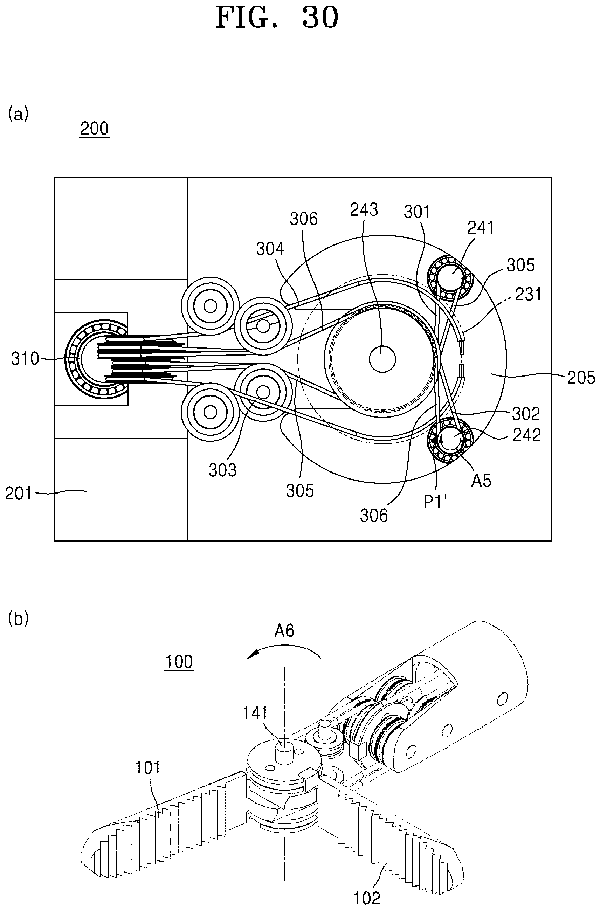

FIG. 29 is a plan view illustrating the driving part and the end tool of the surgical instrument of FIG. 6. FIG. 30 is a diagram illustrating the driving part and the end tool during a yaw motion of the surgical instrument of FIG. 6. FIGS. 29 and 30 are diagrams illustrating a yaw motion of the surgical instrument illustrated in FIG. 6

Referring to FIGS. 29 and 30, when the rotation shaft 242 rotates in the direction of an arrow A5 for a yaw motion, any one of the wire 302 and the wire 306, which constitute the second jaw wire, is wound around the rotation shaft 242, and the other one is unwound from the rotation shaft 242, according to the rotation of the rotation shaft 242. Accordingly, as the pulley 121, which is an end tool first jaw pulley connected to the opposite side of the wire 302 and the wire 306, rotates in the direction of an arrow A6, the yaw motion is performed.

At this time, the positions of the rotation shaft 242, and the pulley 221 and the pulley 222, which are driving part jaw pulleys, are not changed, but only a motion of winding or unwinding the wire 302 and the wire 306 around or from the driving part jaw pulleys 221 and 222 occurs.

Thus, the pulley 231, which is a driving part pitch pulley, does not rotate, and the wire 303 and the wire 304, which are pitch wires, also maintain their positions without being wound or unwound.

Similarly, when the rotation shaft 241 rotates for a yaw motion, any one of the wire 301 and the wire 305, which constitute the first jaw wire, is wound around the rotation shaft 241 and the other one is unwound from the rotation shaft 241, according to the rotation of the rotation shaft 241. Accordingly, as the pulley 111, which is an end tool first jaw pulley connected to the opposite side of the wire 301 and the wire 305, rotates in any one direction, the yaw motion is performed.

At this time, the positions of the rotation shaft 241, and the pulley 211 and the pulley 212, which are driving part jaw pulleys, are not changed, but only a motion of winding or unwinding the wire 301 and the wire 305 around or from the driving part jaw pulleys 211 and 212 occurs.

Thus, the pulley 231, which is a driving part pitch pulley, does not rotate, and the wire 303 and the wire 304, which are pitch wires, also maintain their positions without being wound or unwound.

Accordingly, even when the rotation shaft 241 or the rotation shaft 242 rotate for a yaw motion or an actuation motion, the overall lengths of the wire 301, the wire 302, the wire 305, and the wire 306, which are jaw wires, within the driving part 200 are kept constant.

As described above, in the surgical instrument 30 according to an embodiment of the present disclosure, when the driving part pitch pulley rotates, the rotation shaft 241 and the rotation shaft 242 revolve around the rotation shaft of the driving part pitch pulley to change the path length of the jaw wire wound around the driving part jaw pulley, and thus, the jaw wire is wound or unwound in response to the rotation of the driving part pitch pulley, such that the movement of the jaw wire due to the pitch drive may be offset or compensated for, and as a result, the effect of separating the pitch motion and the yaw motion from each other may be obtained.

### <Motor pack - first embodiment>

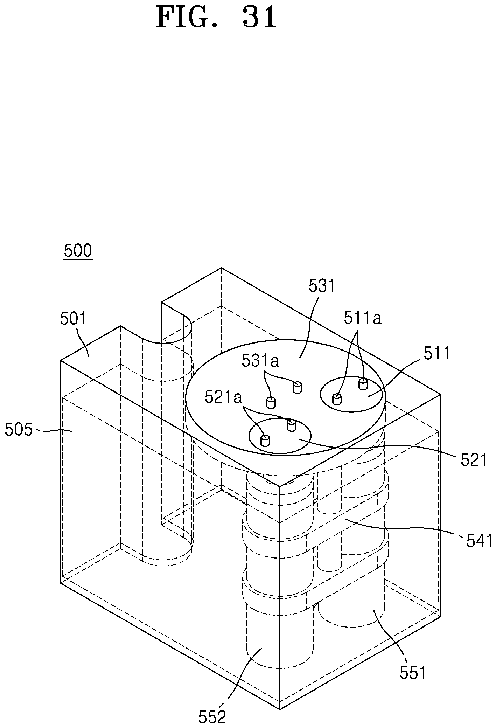

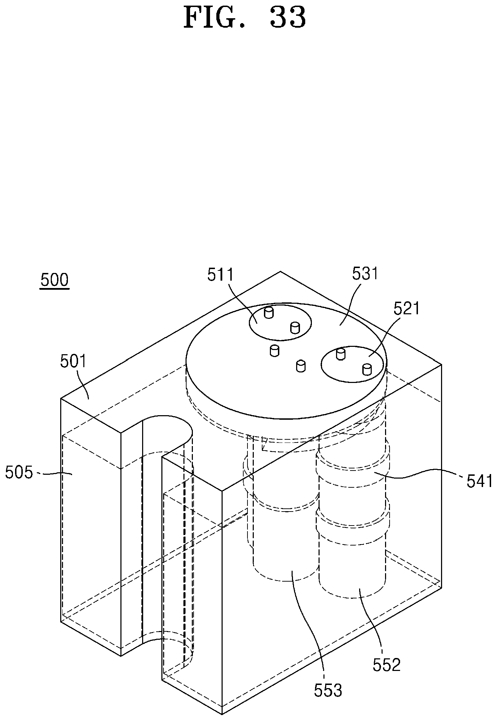

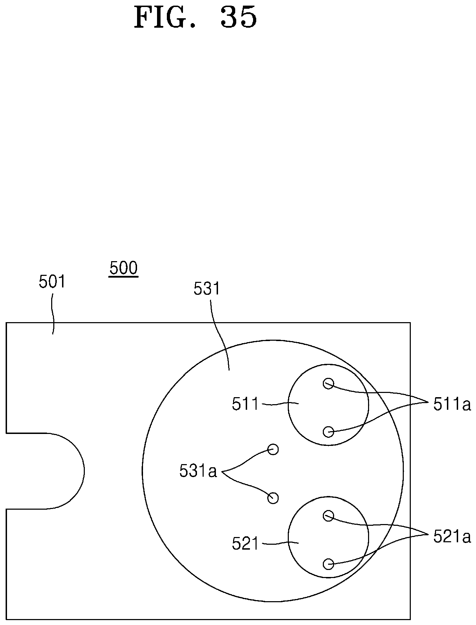

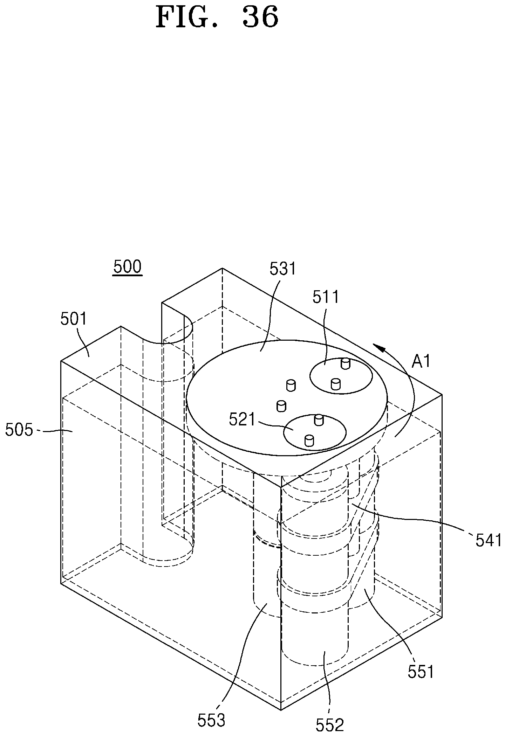

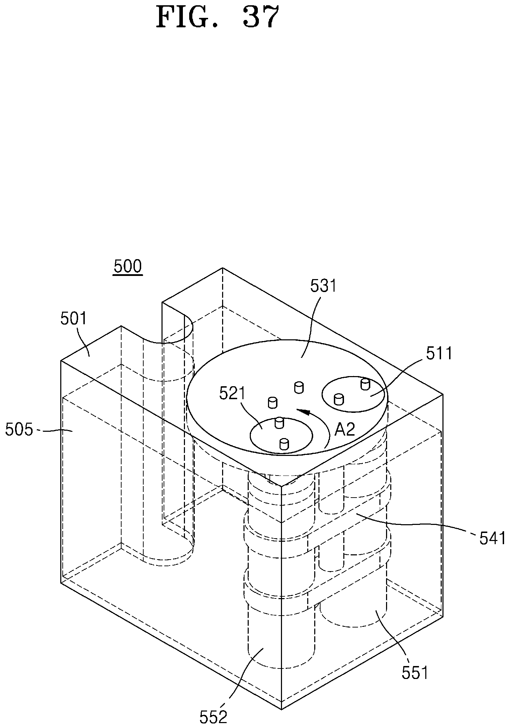

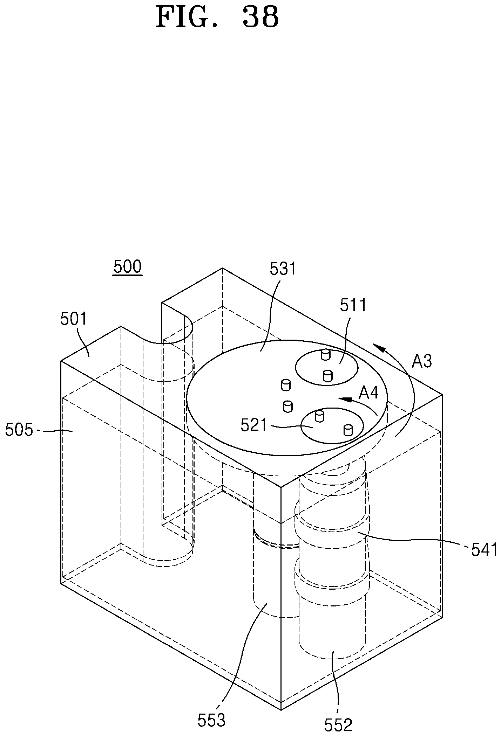

FIGS. 31 to 33 are perspective views illustrating a motor pack according to an embodiment of the present disclosure. FIG. 34 is a bottom perspective view illustrating the motor pack of FIG. 31. FIG. 35 is a plan view illustrating the motor pack of FIG. 31. FIG. 36 is a diagram illustrating rotation by a pitch driving motor in FIG. 31. FIG. 37 is a diagram illustrating rotation by a second jaw driving motor in FIG. 31. FIG. 38 is a diagram illustrating rotation by a pitch driving motor and a second jaw driving motor in FIG. 31.

Referring to FIGS. 31 to 38, the motor pack 500 according to the first embodiment of the present disclosure is provided in the surgical robot 20 and may be coupled to the robotic arm unit 21, 22, or 23. The motor pack 500 may receive power supply from an external source and generate power. However, the present disclosure is not limited thereto, and various modifications are possible, such as generating power by using a built-in battery.

The motor pack 500 is connected to the driving part 200 of the surgical instrument 30, and may transmit power to the driving part 200. The motor pack 500 may further include a first case 501, a second case 505, a pitch plate 531, a first jaw plate 511, a second jaw plate 521, the first jaw driving motor 551, the second jaw driving motor 552, a third jaw driving motor 553, and a motor connection member 541.

The first case 501 and the second case 505 form the exterior of the motor pack 500, and may have a hollow interior. The first jaw driving motor 551, the second jaw driving motor 552, and the pitch driving motor 553 may be arranged in internal spaces of the first case 501 and the second case 505.

Referring to FIGS. 31 to 38, the first case 501 and the second case 505 may be formed independently of each other, and then combined with each other. However, the present disclosure is not limited thereto, and various modifications are possible, for example, they may be formed as one body.

The pitch plate 531 is arranged on the first case 501 and may be rotatably arranged. The pitch plate 531 is connected to the pitch driving motor 553 and may receive power from the pitch driving motor 553.

The pitch driving motor 553 may be connected to one surface of the pitch plate 531 (the lower surface in FIG. 31), and protrusions 531a may be formed on the opposite surface (the upper surface in FIG. 31) to protrude toward the driving part 200.

One or more protrusions 531a may be formed and may be inserted into the coupling hole 205a formed on one surface of the driving part 200 facing the protrusions 531a. Accordingly, the driving part 200 and the motor pack 500 may be connected to each other, and power generated by the motor pack 500, specifically the pitch driving motor 553, may be transmitted to the driving part 200.

Referring to FIGS. 31 to 35, the first jaw plate 511 and the second jaw plate 521 may be arranged on the pitch plate 531. Holes may be formed on the pitch plate 531, and the first jaw plate 511 and the second jaw plate 521 may be arranged in these holes, respectively.

The first jaw plate 511 and the second jaw plate 521 may be arranged to be rotatable inside the pitch plate 531. The first jaw plate 511 and the second jaw plate 521 may be combined with the first jaw driving motor 551 and the second jaw driving motor 552, respectively.

One surface of the first jaw plate 511 (the lower surface in FIG. 31) may be connected to the first jaw driving motor 551, and the other side (the upper surface in FIG. 31) may be connected to the driving part 200, specifically the motor coupling part 251.

Referring to FIG. 35, protrusions 511a may be formed on the first jaw plate 511 and may be connected to the coupling hole 251a formed in the motor coupling part 251. Accordingly, the motor coupling part 251 may rotate by receiving power generated by the first jaw driving motor 551, and as the motor coupling part 251 rotates, the rotation shaft 241 of the motor coupling part 251 rotates, and the wires 301 and 305, which constitute the first jaw wire connected to the rotation shaft 241, may be wound or unwound.

One surface of the second jaw plate 521 (the lower surface in FIG. 31) may be connected to the second jaw driving motor 552, and the other side (the upper surface in FIG. 31) may be connected to the driving part 200, specifically the motor coupling part 252.

Referring to FIG. 35, protrusions 521a may be formed on the second jaw plate 521 and may be connected to the coupling hole 252a formed in the motor coupling part 252. Accordingly, the motor coupling part 252 may rotate by receiving power generated by the second jaw driving motor 552, and as the motor coupling part 252 rotates, the rotation shaft 242 of the motor coupling part 252 rotates, and the wires 302 and 306, which constitute the second jaw wire connected to the rotation shaft 242, may be wound or unwound.

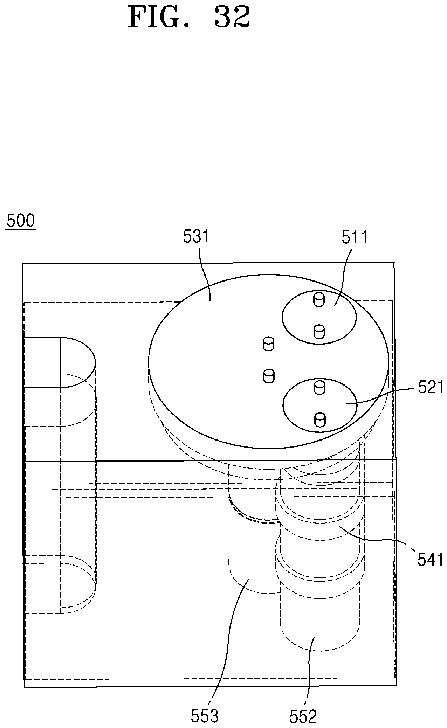

Referring to FIGS. 32 to 34, because the first jaw plate 511 and the second jaw plate 521 are arranged in a preset area on the pitch plate 531, the distances between the rotation centers of the first jaw plate 511 and the second jaw plate 512, and the rotation center of the pitch plate 531 may be kept constant.

Referring to FIGS. 31 to 34, the motor pack 500 may further include the motor connection member 541. The motor connection member 541 is connected to the pitch plate 531, is connected to each of the first jaw driving motor 551 and the second jaw driving motor 552, and may fix the positions of the first jaw driving motor 551 and the second jaw driving motor 552.

That is, the motor connection member 541 is connected to the pitch plate 531 and may rotate when the pitch plate 531 rotates, and accordingly, rotate the first jaw driving motor 551 and the second jaw driving motor 552.

In addition, when the pitch plate 531 rotates by receiving power generated by the pitch driving motor 553 due to the motor connection member 541, the first jaw driving motor 551 and the second jaw driving motor 552 respectively connected to the first jaw plate 511 and the second jaw plate 521 both arranged on the pitch plate 531 may move. In addition, power generated by the first jaw driving motor 551 and the second jaw driving motor 552 may be transmitted to the rotation shaft 241, which is a driving part first jaw rotation shaft, and the rotation shaft 242, which is a driving part second jaw rotation shaft, both provided in the driving part 200, through the first jaw plate 511 and the second jaw plate 521.

As power generated by the motor pack 500 according to the first embodiment of the present disclosure is transmitted to the driving part 200, a pitch motion, a yaw motion, and an actuation motion may be performed as described above.

Referring to FIG. 36, the power generated by the pitch driving motor 553 may be transmitted to the pitch plate 531, and as the pitch plate 531 rotates, the positions of the first jaw plate 511 and the second jaw plate 521 both arranged on the pitch plate 531 may be changed.

That is, the rotation shaft 241 and the rotation shaft 242 of the driving part 200, which are respectively connected to the first jaw plate 511 and the second jaw plate 521, may revolve around the rotation shaft 243, accordingly, the movement of the jaw wires on the side of the end tool according to the pitch motion may be compensated for, and thus, the pitch motion may be performed independently of the yaw/actuation motions. This has been described above, and thus, detailed descriptions thereof will be omitted.

Referring to FIG. 37, in a state in which the pitch plate 531 does not rotate, power generated by the second jaw driving motor 552 may be transmitted to the second jaw plate 512, to rotate the second jaw plate 512 in the direction of A2. The second jaw plate 512 may transmit power to the rotation shaft 242, which is a driving part second jaw rotation shaft, through the motor coupling part 252, and a yaw motion or an actuation motion may be performed by winding or unwinding the wires 302 and 306, which constitute the second jaw wire coupled to the rotation shaft 242.

Referring to FIG. 38, power generated by the pitch driving motor 553 of FIG. 36 rotates the pitch plate 531 in the direction of A3, and independently of this, power generated by the second jaw driving motor 552 may be transmitted to the second jaw plate 512 to rotate the second pitch plate 512 in the direction of A4.

Referring to FIG. 38, the pitch motion of the end tool 100 may be performed by driving the pitch driving motor 553, power may be transmitted to the rotation shaft 242, which is a driving part second jaw rotation shaft, through the motor coupling part 252 by driving the second jaw driving motor 552, and a yaw motion or an actuation motion may be performed by winding or unwinding the wires 302 and 306, which constitute the second jaw wire coupled to the rotation shaft 242.

Referring to FIGS. 31 to 38, in the motor pack 500 according to the first embodiment of the present disclosure, the first jaw plate 511 and the second jaw plate 521 are arranged inside the pitch plate 531, and when the pitch plate 531 receives power from the pitch driving motor 553 and rotates, the first jaw plate 511 and the second jaw plate 521 may revolve around the rotation center of the pitch plate 531.

In addition, the motor connection member 541 is coupled to the pitch plate 531 and changes in position as the pitch plate 531 rotates, and may revolve the first jaw driving motor 551 and the second jaw driving motor 552 both connected to the motor connection member 541, around the pitch driving motor 553.

Accordingly, power generated by the motor pack 500 may be transmitted to the driving part 200, and when the driving part pitch pulley rotates around the rotation shaft 243, the rotation shaft 241 and the rotation shaft 242 revolve around the rotation shaft 243 to change the path length of the jaw wire wound around the driving part jaw pulley. In addition, the change in the path length of the jaw wire compensates for the movement of the jaw wires on the side of the end tool due to the pitch motion, and as a result, the pitch motion may be independently performed.

### Mode for Invention

### <Driving part - second embodiment>

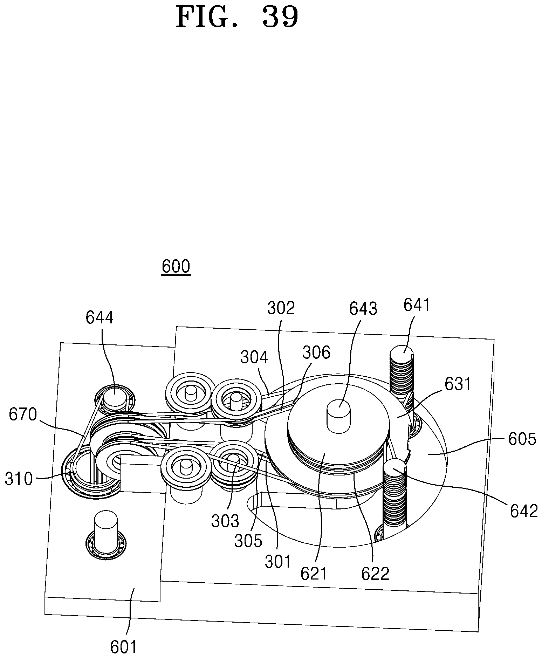

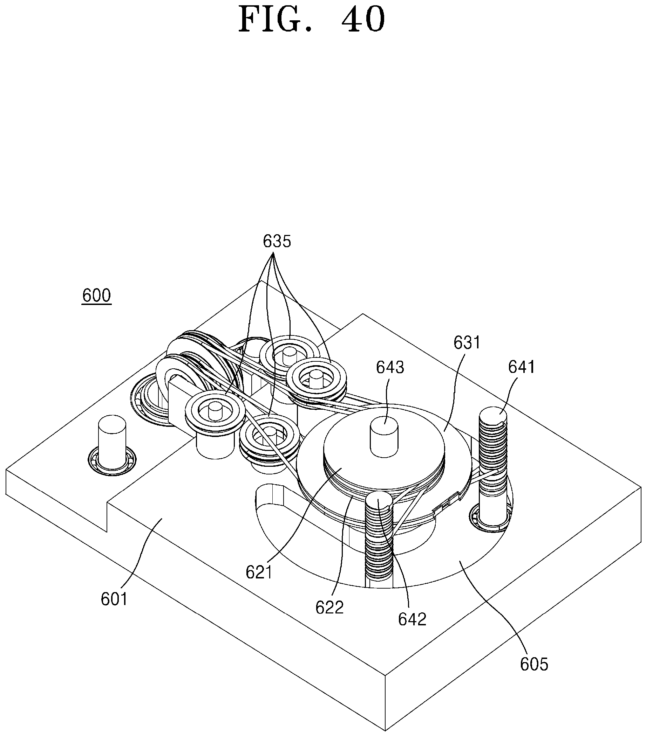

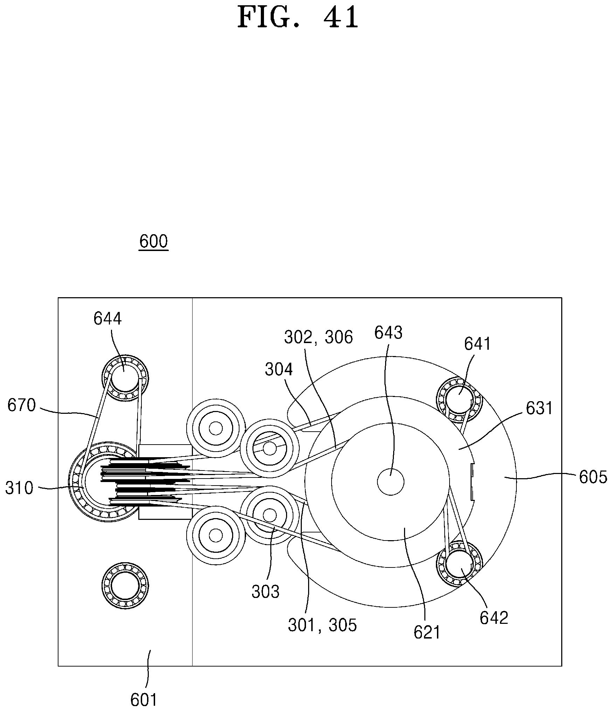

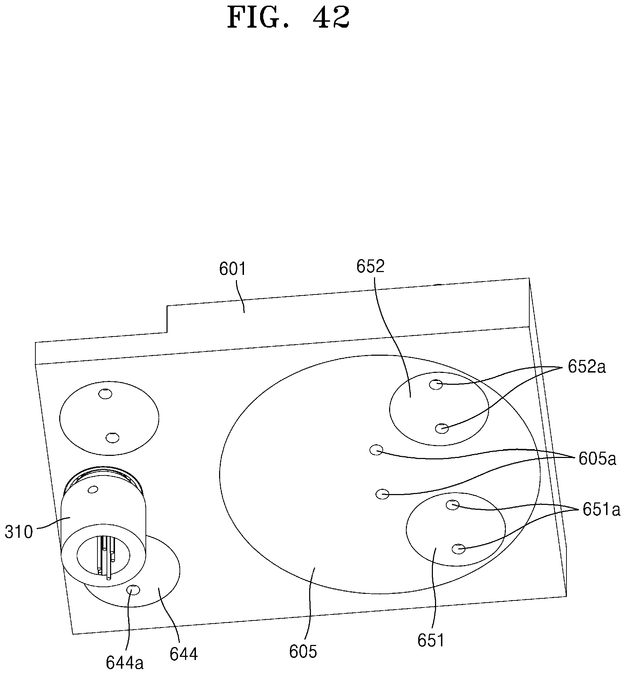

FIGS. 39 and 40 are perspective views illustrating a driving part according to another embodiment of the present disclosure. FIG. 41 is a plan view illustrating a driving part according to another embodiment of the present disclosure. FIG. 42 is a bottom perspective view illustrating a driving part according to another embodiment of the present disclosure.

A driving part 600 according to the second embodiment of the present disclosure is different from the first embodiment illustrated in FIGS. 15 to 28 in that the configuration of a roll rotating shaft 644 for a roll motion, and transmission of power generated from the roll rotating shaft 644 to the connection part 310 is different. The following description will focus on the difference from the first embodiment.

Referring to FIGS. 39 to 42, the rotation shaft 644, which is a roll rotation shaft, may be arranged in a case 601 of the driving part 600 according to the second embodiment of the present disclosure. The central rotation axis of the roll rotation shaft 644 may be arranged parallel to a rotation shaft 641, a rotation shaft 642, and a rotation shaft 643.

In addition, the central rotation axis of the roll rotation shaft 644 may be arranged parallel to the longitudinal central axis of the connection part 310.

Referring to FIGS. 39 to 42, the roll rotation shaft 644 may be connected to a roll driving motor (not shown) and may rotate by receiving power from the roll driving motor. A coupling hole 644a may be formed on one surface of the roll rotation shaft 644 and may be connected to a protrusion (not shown) formed on the roll driving motor.

Accordingly, the effect of transmitting power generated by the roll driving motor to the roll rotation shaft 644 may be obtained.

Referring to FIGS. 39 to 41, the roll rotation shaft 644 may transmit power to the connection part 310 through a wire 670. In the drawings, the roll rotation shaft 644 and the connection part 310 are connected to each other through the wire 670, but the present disclosure is not limited thereto, and various modifications are possible, such as connection to a belt.

The driving part 600 according to the second embodiment of the present disclosure has the same configuration as the driving part 200 according to the first embodiment, except that the roll rotation shaft 644 for transmitting rotational power to the connection part 310 is provided, and power generated by the roll driving motor is transmitted to the connection part 310 through the wire 670, and thus, redundant detailed descriptions thereof will be omitted.

### <Driving part - third embodiment>

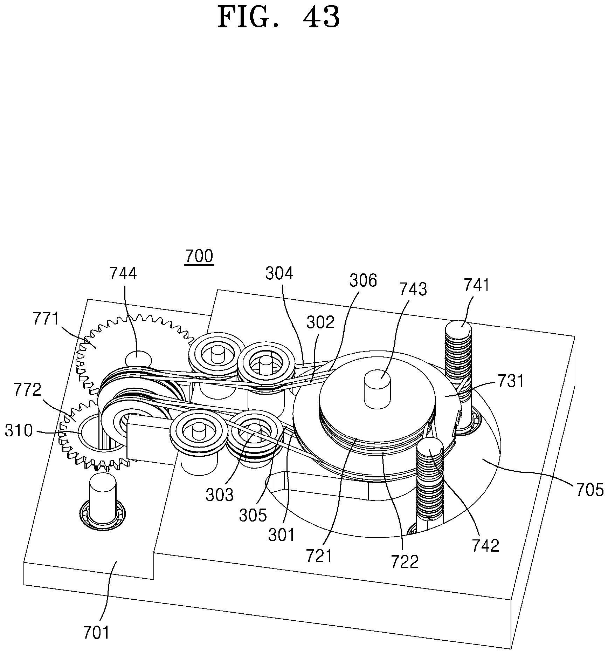

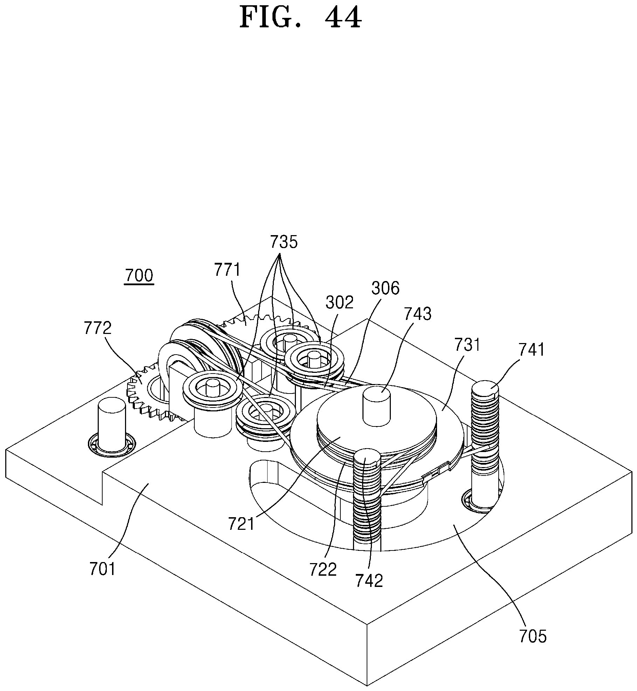

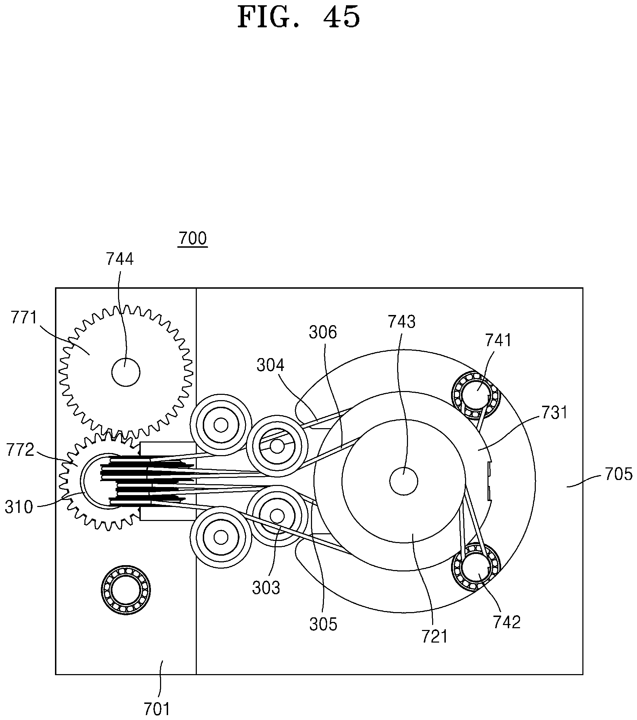

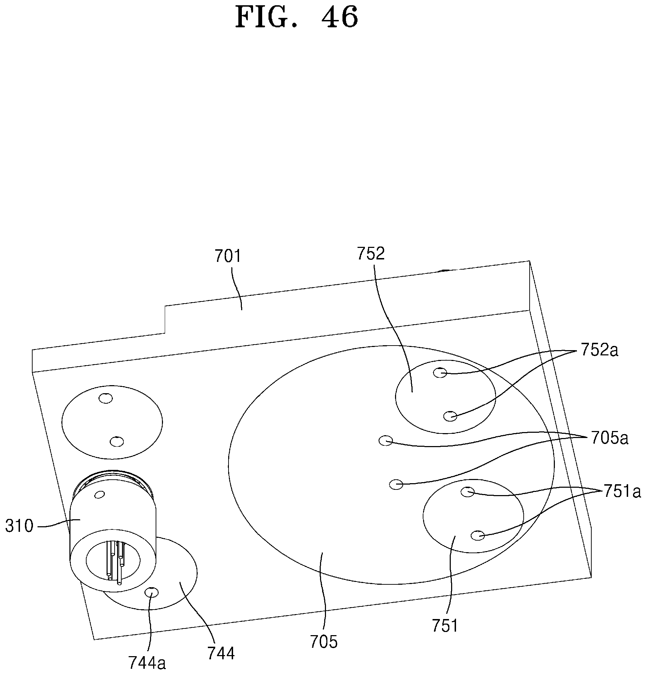

FIGS. 43 and 44 are perspective views illustrating a driving part according to another embodiment of the present disclosure. FIG. 45 is a plan view illustrating a driving part according to another embodiment of the present disclosure. FIG. 46 is a bottom perspective view illustrating a driving part according to another embodiment of the present disclosure.

A driving part 700 according to the third embodiment of the present disclosure is different from the driving part according to the second embodiment illustrated in FIGS. 39 to 42 in that the configuration of transmission of power generated from a roll rotating shaft 744 to the connection part 310 is different. The following description will focus on the difference from the second embodiment.

Referring to FIGS. 43 to 46, the rotation shaft 744, which is a roll rotation shaft, may be arranged in a case 701 of the driving part 700 according to the second embodiment of the present disclosure. The central rotation axis of the roll rotation shaft 744 may be arranged parallel to a rotation shaft 741, a rotation shaft 742, and a rotation shaft 743.

In addition, the central rotation axis of the roll rotation shaft 744 may be arranged parallel to the longitudinal central axis of the connection part 310.

Referring to FIGS. 43 to 46, the roll rotation shaft 744 may be connected to a roll driving motor (not shown) and may rotate by receiving power from the roll driving motor. A coupling hole 744a may be formed on one surface of the roll rotation shaft 744 and may be connected to a protrusion (not shown) formed on the roll driving motor.

Accordingly, the effect of transmitting power generated by the roll driving motor to the roll rotation shaft 744 may be obtained.

Referring to FIGS. 43 to 46, the roll rotation shaft 744 may transmit power to the connection part 310 through a plurality of wires. In detail, the gears may include a gear 771 for roll motion and a gear 772 for roll motion, and as the gears 771 and 772 engage each other, power generated from the roll rotation shaft 744 may be transmitted to the connection part 310.

The gears 771 and 772 may have different diameters as illustrated in FIG. 45. However, the present disclosure is not limited thereto, and various modifications are possible, for example, they have the same diameter.

The driving part 700 according to the third embodiment of the present disclosure has the same configuration as the driving part 600 according to the second embodiment, except that the roll rotation shaft 744 for transmitting rotational power to the connection part 310 is provided, and power generated by the roll driving motor is transmitted to the connection part 310 through a plurality of gears for roll motion including the gear 771 and the gear 772, and thus, redundant detailed descriptions thereof will be omitted.

### <Motor pack - second embodiment>

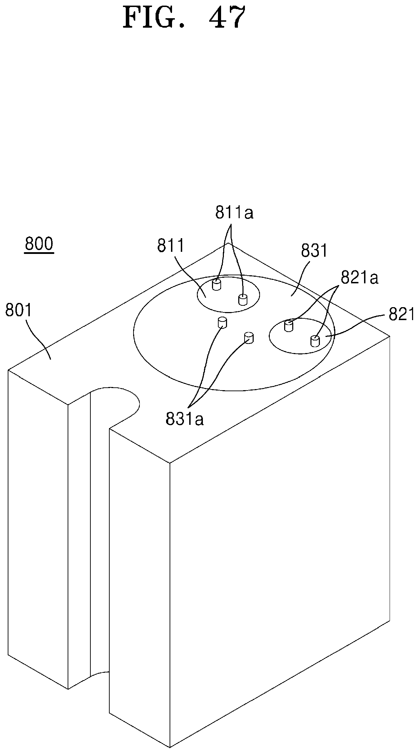

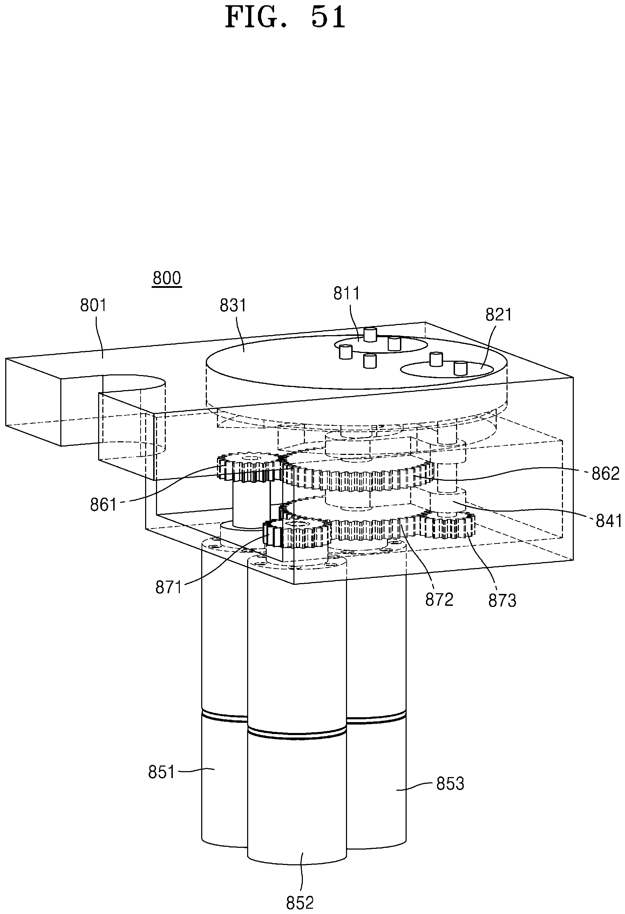

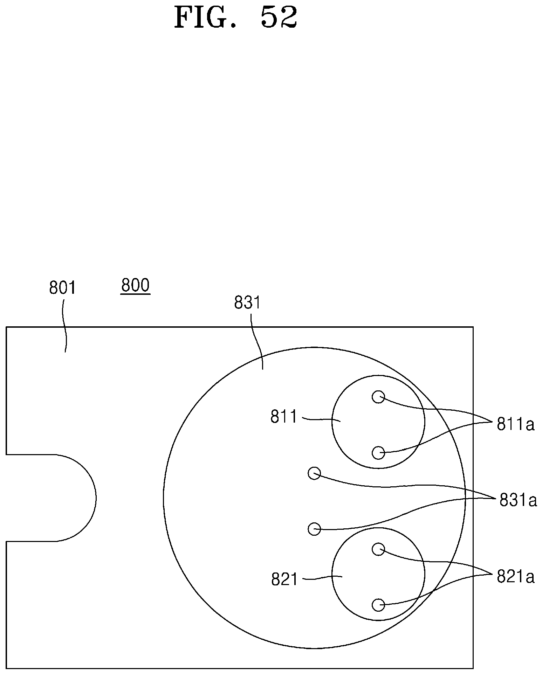

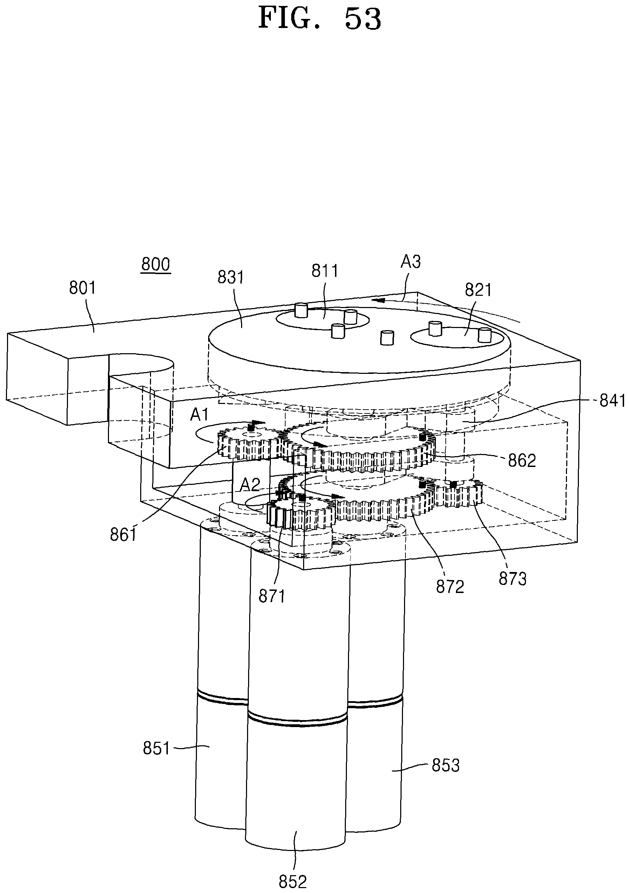

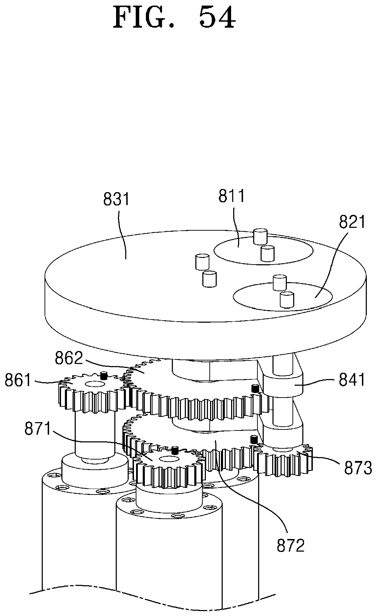

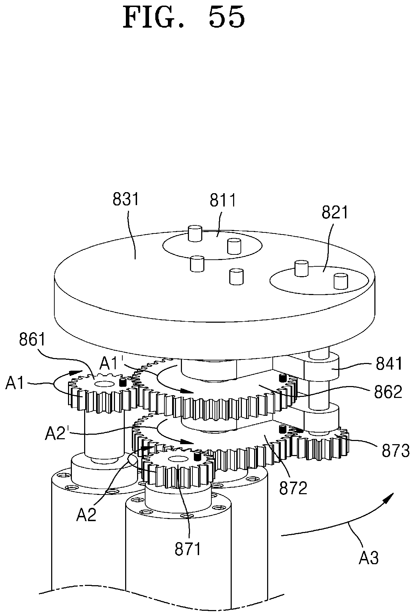

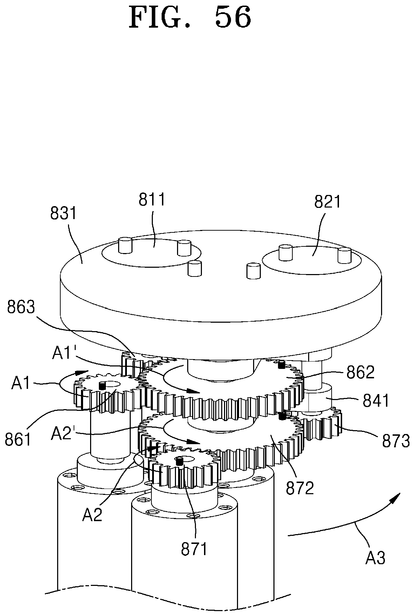

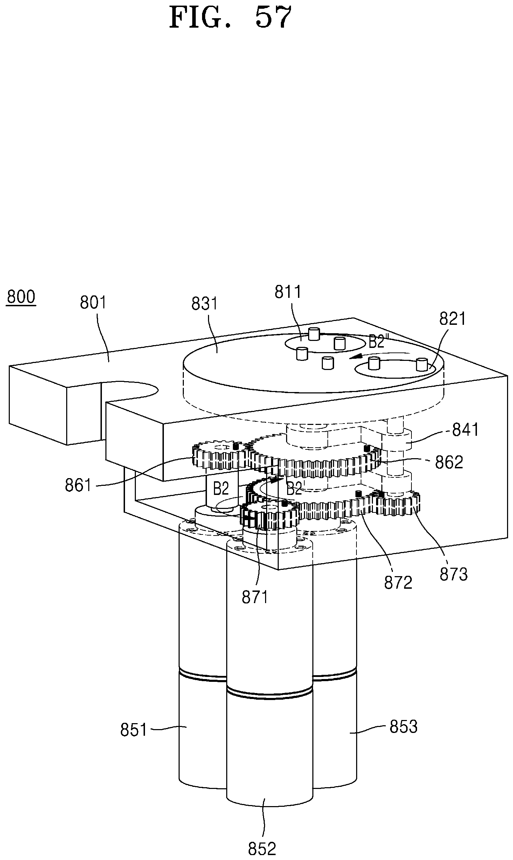

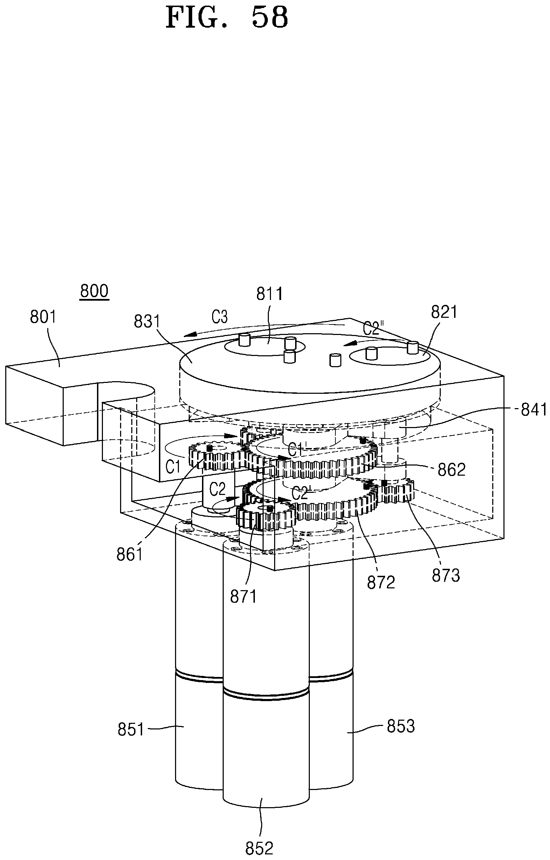

FIGS. 47 to 51 are perspective views illustrating a motor pack according to another embodiment of the present disclosure. FIG. 52 is a plan view illustrating a motor pack according to another embodiment of the present disclosure. FIG. 53 is a diagram illustrating rotation by a pitch driving motor in FIG. 51. FIGS. 54 to 56 are enlarged views sequentially illustrating rotation by a pitch driving motor. FIG. 57 is a diagram illustrating rotation by a second jaw driving motor in FIG. 51. FIG. 58 is a diagram illustrating rotation by a pitch driving motor and a second jaw driving motor in FIG. 51.

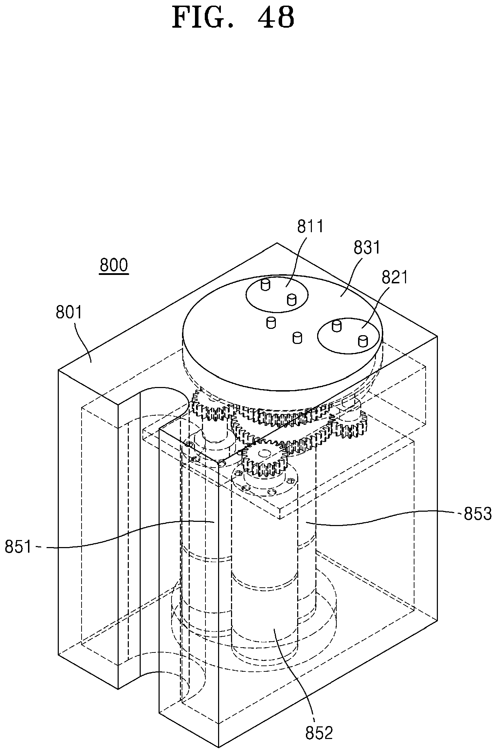

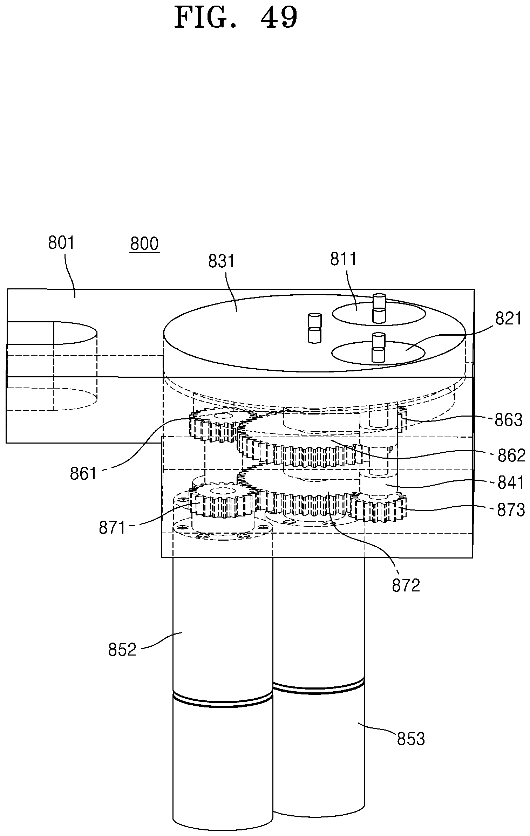

Referring to FIGS. 48 and 49, a motor pack 800 of the second embodiment of the present disclosure includes a first jaw driving motor 851, a second jaw driving motor 852, and a pitch driving motor 853. Meanwhile, a first jaw intermediate gear 862 and a second jaw intermediate gear 872, which will be described below, are rotatably coupled to a rotation shaft (not shown) of the pitch driving motor 853.

Referring to FIGS. 47 to 49, the motor pack 800 may further include a case 801. The case 801 has a hollow interior and may accommodate the first jaw driving motor 851, the second jaw driving motor 852, and the pitch driving motor 853.

A first jaw driving gear 861 is coupled to the rotation shaft of the first jaw driving motor 851, and when the rotation shaft of the first jaw driving motor 851 rotates, the first jaw driving gear 861 rotates together.

In addition, the first jaw intermediate gear 862 is arranged on one side of the first jaw driving gear 861 to engage the first jaw driving gear 861. In addition, a first jaw rotation gear 863 is arranged on one side of the first jaw intermediate gear 862 to engage the first jaw intermediate gear 862. In addition, a rotation shaft of the first jaw rotation gear 863 is coupled to a first jaw plate 811, such that, when the first jaw rotation gear 863 rotates, the first jaw plate 811 rotates together. Accordingly, when the first jaw driving motor 851 rotates, the driving force passes through the first jaw driving gear 861, the first jaw intermediate gear 862, and the first jaw rotation gear 863, and thus, the first jaw plate 811 rotates.

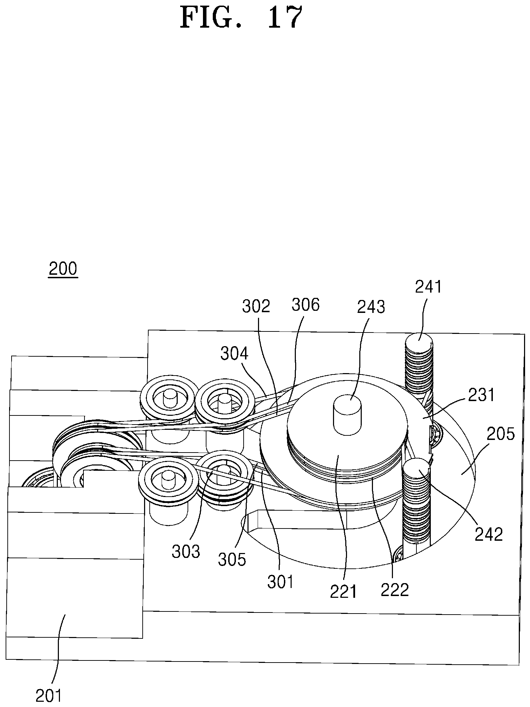

As the first jaw plate 811 rotates, a rotation shaft (see 241 of FIG. 17), which is a driving part first jaw rotation shaft connected to the first jaw plate 811, may rotate, and the wire 301 and the wire 305, which constitute the first jaw wire coupled to the first jaw rotation shaft 241, may be wound or unwound.

A second jaw driving gear 871 is coupled to the rotation shaft of the second jaw driving motor 852, and when the rotation shaft of the second jaw driving motor 852 rotates, the second jaw driving gear 871 rotates together. In addition, the second jaw intermediate gear 872 is arranged on one side of the second jaw driving gear 871 to engage the second jaw driving gear 871. In addition, a second jaw rotation gear 873 is arranged on one side of the second jaw intermediate gear 872 to engage the second jaw intermediate gear 872. In addition, a rotation shaft of the second jaw rotation gear 873 is coupled to a second jaw plate 821, such that, when the second jaw rotation gear 873 rotates, the second jaw plate 812 rotates together. Accordingly, when the second jaw driving motor 852 rotates, the driving force passes through the second jaw driving gear 871, the second jaw intermediate gear 872, and the second jaw rotation gear 873, and thus, the second jaw plate 821 rotates.

As the second jaw plate 812 rotates, a rotation shaft (see 242 of FIG. 17), which is a driving part second jaw rotation shaft connected to the second jaw plate 812, may rotate, and the wire 302 and the wire 306, which constitute the second jaw wire coupled to the second jaw rotation shaft 242, may be wound or unwound.

Referring to FIG. 52, a pitch plate 831 may be rotatably arranged on the case 801. The pitch plate 831 is connected to the pitch driving motor 853 and may receive power from the pitch driving motor 853. In detail, a rotation shaft (not shown) is formed in the pitch driving motor 853, and the pitch plate 831 is coupled to the rotation shaft (not shown). Thus, when the rotation shaft (not shown) of the pitch driving motor 853 rotates, the pitch plate 831 rotates together.

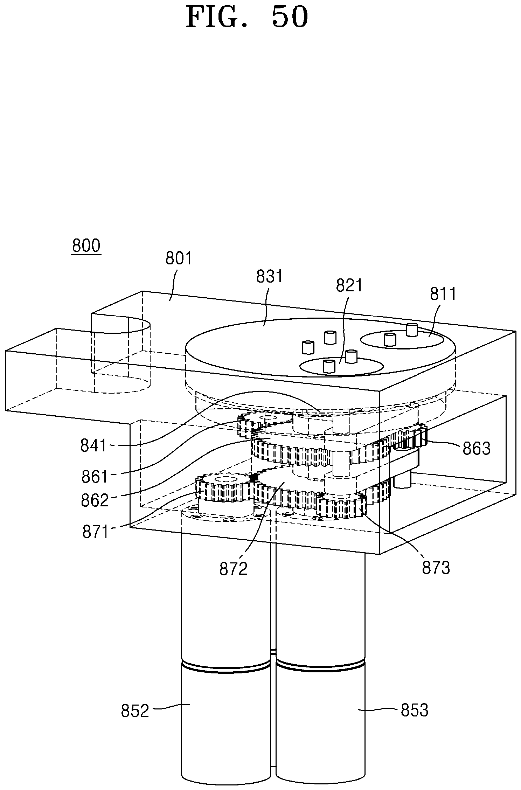

The pitch driving motor 853 may be connected to one surface of the pitch plate 831 (the lower surface in FIG. 50), and protrusions 831a may be formed on the opposite surface (the upper surface in FIG. 50) to protrude toward the driving part 200.

One or more protrusions 831a may be formed and may be inserted into the coupling hole 205a formed on one surface of the driving part 200 facing the protrusions 831a. Accordingly, the driving part 200 and the motor pack 800 may be connected to each other, and power generated by the motor pack 800, specifically the pitch driving motor 853, may be transmitted to the driving part 200.

Referring to FIGS. 47 to 52, the first jaw plate 811 and the second jaw plate 821 may be arranged inside the pitch plate 831. Holes may be formed on the pitch plate 831, and the first jaw plate 811 and the second jaw plate 821 may be arranged in these holes, respectively.

The first jaw plate 811 and the second jaw plate 821 may be arranged to be rotatable on the pitch plate 831. The first jaw plate 811 and the second jaw plate 821 may be connected to the first jaw driving motor 851 and the second jaw driving motor 852, respectively.

One surface of the first jaw plate 811 (the lower surface in FIG. 50) may be connected to the first jaw driving motor 851, and the other side (the upper surface in FIG. 50) may be connected to the driving part 200, specifically the motor coupling part 251.

Referring to FIG. 35, protrusions 811a may be formed on the first jaw plate 811 and may be connected to the coupling hole 251a formed in the motor coupling part 251. Accordingly, the motor coupling part 251 may rotate by receiving power generated by the first jaw driving motor 851, and as the motor coupling part 251 rotates, the rotation shaft 241 of the motor coupling part 251 rotates, and the wires 301 and 305, which constitute the first jaw wire connected to the rotation shaft 241, may be wound or unwound.

One surface of the second jaw plate 821 (the lower surface in FIG. 50) may be connected to the second jaw driving motor 852, and the other side (the upper surface in FIG. 50) may be connected to the driving part 200, specifically the motor coupling part 252.

Referring to FIG. 52, protrusions 821a may be formed on the second jaw plate 821 and may be connected to the coupling hole 252a formed in the motor coupling part 252. Accordingly, the motor coupling part 252 may rotate by receiving power generated by the second jaw driving motor 852, and as the motor coupling part 252 rotates, the rotation shaft 242 of the motor coupling part 252 rotates, and the wires 302 and 306, which constitute the second jaw wire connected to the rotation shaft 242, may be wound or unwound.

Referring to FIGS. 49 to 51, the first jaw intermediate gear 862 and the second jaw intermediate gear 872 share a central rotation axis with the pitch plate 831, and are rotatable independently of rotation of the pitch plate 831.

Although not illustrated in the drawings, bearings may be arranged between the central rotation axis (reference numeral not provided) of the pitch plate 831, the first jaw intermediate gear 862, and the second jaw intermediate gear 872, and the first jaw intermediate gear 862 and the second jaw intermediate gear 872 may be arranged to be rotatable independently of the central rotation axis of the pitch plate 831.

Referring to FIGS. 49 to 51, the first jaw intermediate gear 862 and the second jaw intermediate gear 872 have the same central rotation axis and may be arranged at different heights to be spaced apart from each other at regular intervals.

Accordingly, the first jaw driving gear 861 and the second jaw driving gear 871 may be arranged at different heights to be spaced apart from each other, and the first jaw rotation gear 863 and the second jaw rotation gear 873 may also be arranged at different heights to be spaced apart from each other.

Accordingly, the effect of preventing interference between the first jaw driving gear 861, the first jaw intermediate gear 862, the first jaw rotation gear 863, the second jaw driving gear 871, the second jaw intermediate gear 872, and the second jaw rotation gear 873 may be obtained.

Referring to FIGS. 49 to 51, the motor pack 800 may further include a motor connection member 841. The motor connection member 841 is coupled to the pitch plate 831 and may be connected to the central rotation axis of the first jaw plate 811 and the central rotation axis of the second jaw plate 821.

Accordingly, power generated by the first jaw driving motor 851 and the second jaw driving motor 852 may be transmitted to the first jaw plate 811 and the second jaw plate 821, while maintaining the balance of the central rotation axis of each of the first jaw plate 811 and the second jaw plate 821.

That is, the first jaw plate 811 and the second jaw plate 821 are coupled to the pitch plate 831, and thus, when the pitch plate 831 rotates, the first jaw plate 811 and the second jaw plate 821 revolve around a rotation shaft (not shown) of the pitch driving motor 853. This will be explained in more detail in the description of a pitch motion below.

### (Pitch motion)

FIGS. 54 to 56 are enlarged views sequentially illustrating rotation by a pitch driving motor. When the pitch driving motor 853 rotates, the pitch plate 831 rotates around a pitch rotation shaft (not shown), and the first jaw plate 811 and the second jaw plate 821 both arranged on the pitch plate 831 also revolve around the pitch rotation shaft.

At this time, in order to compensate for the rotation of the first jaw plate 811 and the second jaw plate 821, the first jaw driving motor 851 and the second jaw driving motor 852 rotate.

In detail, referring to FIGS. 54 to 56, the pitch driving motor 853 and the pitch plate 831 rotate in the direction of an arrow A3 for a pitch motion. Then, by the rotation of the pitch plate 831, the first jaw plate 811 and the second jaw plate 821 also revolve in the direction of the arrow A3 around the rotation shaft of the pitch driving motor 853.

In order to compensate for the rotation (revolution) of the first jaw plate 811 by the pitch driving motor 853 and the pitch plate 831, the first jaw driving motor 851 and the first jaw driving gear 861 coupled thereto rotate in the direction of an arrow A1. Then, the first jaw intermediate gear 862 engaging the first jaw driving gear 861 rotates in the direction of an arrow A1'. At this time, the first jaw rotation gear 863 revolves around the pitch rotation shaft by the first jaw plate 811.

As such, the first jaw intermediate gear 862 rotates around the pitch rotation shaft by the first jaw driving gear 861, and at the same time, the first jaw rotation gear 863 revolves around the pitch rotation shaft, and thus, during the rotation and revolution, a particular tooth of the first jaw intermediate gear 862 and a tooth of the first jaw rotation gear 863 are continuously in contact with each other. That is, the first jaw rotation gear 863 does not gear-rotate with respect to the first jaw intermediate gear 862.

As a result, while a pitch motion is performed, the relative positions of the first jaw intermediate gear 862 and the first jaw rotation gear 863 are kept constant. In order words, it may also be described that the first jaw intermediate gear 862 and the first jaw rotation gear 863 rotate around the pitch rotation shaft as one body.

As such, when the pitch driving motor 853 rotates to perform a pitch motion, the revolution of the first jaw plate 811 and the second jaw plate 812, which occurs incidentally, may be compensated for by rotation of the first jaw driving motor 851 and the second jaw driving motor 852, and accordingly, the pitch motion may not be affected by the movement of a jaw.

### (Second jaw rotation motion)

As illustrated in FIG. 57, in order for the second jaw (see 102 of FIG. 7) to operate, the second jaw driving motor 852 needs to rotate. When the rotation shaft of the second jaw driving motor 852 and the second jaw driving gear 871 coupled thereto rotate in the direction of an arrow B2, the second jaw intermediate gear 872 engaging the second jaw driving gear 871 rotates in the direction of B2'. In addition, the second jaw rotation gear 873 engaging the second jaw intermediate gear 872, and the second jaw plate 821 rotate in the direction of an arrow B2". At this time, the pitch plate 811 does not rotate, and maintains its position, and only the second jaw plate 821 arranged on the pitch plate 811 rotates.

### (Simultaneously performing a pitch motion and a second jaw rotation motion)

As illustrated in FIG. 58, in order for the second jaw to rotate at the same time as a pitch motion is performed, the pitch driving motor 853 and the second jaw driving motor 852 may each rotate.

In detail, referring to FIG. 58, the pitch driving motor 853 and the pitch plate 831 rotate in the direction of an arrow C3 for a pitch motion. Then, by the rotation of the pitch plate 831, the first jaw plate 811 and the second jaw plate 821 also revolve in the direction of the arrow C3 around the rotation shaft of the pitch driving motor 853.

In order to compensate for the rotation (revolution) of the first jaw plate 811 by the pitch driving motor 853 and the pitch plate 831, the first jaw driving motor 851 and the first jaw driving gear 861 coupled thereto rotate in the direction of an arrow C1. Then, the first jaw intermediate gear 862 engaging the first jaw driving gear 861 rotates in the direction of an arrow C1'. At this time, the first jaw rotation gear 863 revolves around the pitch rotation shaft by the first jaw plate 811.

Meanwhile, in order for the second jaw to rotate at the same time as a pitch motion is performed, the second jaw driving motor 852 and the second jaw driving gear 871 coupled thereto rotate in the direction of an arrow C2. Then, the second jaw intermediate gear 872 engaging the second jaw driving gear 871 rotates in the direction of an arrow C2', and the second jaw rotation gear 873 engaging the second jaw intermediate gear 872 rotates in the direction of an arrow C".

At this time, when the pitch driving motor 853 and the second jaw driving motor 852 rotate together, the rotation amount for compensating for the rotation of the pitch driving motor 853 when the second jaw driving motor 852 rotates needs to be considered. At this time, the drawing illustrates that, because the second jaw driving gear 871 is formed to be relatively smaller than the second jaw intermediate gear 872, assuming that the pitch rotates by 45° and the second jaw also rotates by 45°, the second jaw driving motor 852 rotates clockwise. However, this may vary depending on the rotation ratio of each gear.

In the motor pack 800 of the second embodiment of the present disclosure, the positions of the first jaw driving motor 851 and the second jaw driving motor 852 may be maintained without changing, unlike the motor pack 500 of the first embodiment in which the first jaw driving motor 551 and the second jaw driving motor 552 rotate together as the pitch driving motor 553 rotates.

In addition, the central rotation axis of the first jaw driving motor 851 is arranged spaced apart from the central rotation axis of the first jaw plate 811, and the central rotation axis of the second jaw driving motor 852 is arranged spaced apart from the central rotation axis of the second jaw plate 821, unlike the motor pack 500 of the first embodiment in which the central rotation axis of the first jaw driving motor 551 is the same as the central rotation axis of the first jaw plate 511 when rotational power is generated, and the central rotation axis of the second jaw driving motor 552 is the same as the central rotation axis of the second jaw plate 521 when rotational power is generated.

Instead, power generated by the first jaw driving motor 851 may be transmitted to the first jaw plate 811 through the first jaw driving gear 861, the first jaw intermediate gear 862, and the first jaw rotation gear 863.

Similarly, power generated by the second jaw driving motor 852 may be transmitted to the second jaw plate 821 through the second jaw driving gear 871, the first jaw intermediate gear 872, and the first jaw rotation gear 873.

Except for the above, the configuration and effect of transmitting rotational power of the pitch plate 831, the first jaw plate 811, and the second jaw plate 821 to the driving part 200 are the same as those of the motor pack 500 according to the first embodiment. and thus, redundant detailed descriptions thereof will be omitted.

As such, the present disclosure has been described with reference to one embodiment shown in the drawings, but it will be understood that this is merely exemplary, and those of ordinary skill in the art will understand that various modifications and variations of the embodiments are possible therefrom. Accordingly, the true technical protection scope of the present disclosure should be defined by the technical spirit of the appended claims.

### Industrial Applicability

The present disclosure relates to a surgical instrument and a surgical robot including the same, and more particularly, to a surgical instrument that may be operated manually or automatically for use in laparoscopic surgery or various other surgeries, and may perform a pitch motion and a yaw motion/actuation motion independently of each other in a seamless manner, and a surgical robot including the surgical instrument.

## Claims

1. A surgical instrument comprising:
an end tool comprising one or more jaws and an end tool jaw pulley, which is coupled to the jaw, and formed to be rotatable together with the one or
more jaws around a first shaft, the end tool being formed to be able to perform at least pitch rotation and yaw rotation;
a jaw wire that is coupled to the end tool jaw pulley and moves according to rotation of the end tool jaw pulley;
a connection part extending in one direction, through which the jaw wire passes, and having one end to which the end tool is coupled;
a driving part that is coupled to another end of the connection part and is configured to control the pitch rotation and the yaw rotation of the end tool, wherein the driving part comprises:
a driving part jaw pulley that is formed to be rotatable around a second shaft, and is formed to wind at least a portion of the jaw wire; and
a driving part jaw rotation shaft that is formed to be rotatable around a third shaft different from the second shaft, is relatively movable while maintaining a preset distance from a center of the driving part jaw pulley, revolves around the center of the driving part jaw pulley, and is coupled to the jaw wire,
the jaw wire moves through rotation of the driving part jaw rotation shaft to cause the end tool jaw pulley and the jaw to rotate,
two strands of the jaw wire, which emerge while being wound around the driving part jaw pulley, extend toward the end tool after being sequentially wound around the driving part jaw pulley, the driving part jaw rotation shaft, and the driving part jaw pulley, and
when the driving part jaw rotation shaft moves relative to the driving part jaw pulley, the end tool performs the pitch rotation while an overall length of the jaw wire within the driving part is changed.

2. The surgical instrument of claim 1, further comprising a driving part pitch pulley arranged adjacent to the driving part jaw pulley and formed to be rotatable around the second shaft,
wherein the driving part jaw rotation shaft is formed to be movable relative to the driving part pitch pulley, such that, when the driving part pitch pulley rotates, a relative position of the driving part jaw rotation shaft with respect to the second shaft is changed.

3. The surgical instrument of claim 2, wherein a relative distance between a rotation center of the driving part pitch pulley and a rotation center of the driving part jaw rotation shaft is kept constant.

4. The surgical instrument of claim 2, wherein, when the driving part pitch pulley rotates, the driving part jaw rotation shaft moves in conjunction with the driving part pitch pulley.

5. The surgical instrument of claim 2, wherein, when the driving part pitch pulley rotates around the second shaft, the driving part jaw rotation shaft moves relative to the driving part pitch pulley such that the overall length of the jaw wire within the driving part is changed.

6. The surgical instrument of claim 5, wherein, as the overall length of the jaw wire within the driving part is changed due to rotation of the driving part pitch pulley, an overall length of the jaw wire within the end tool is also changed.

7. The surgical instrument of claim 5, wherein, even when the overall length of the jaw wire within the driving part is changed due to rotation of the driving part pitch pulley, an overall length of the jaw wire is kept constant.

8. The surgical instrument of claim 2, further comprising an end tool jaw pitch main pulley formed adjacent to the end tool jaw pulley and formed to be rotatable around a fourth shaft different from the first shaft, and an end tool jaw pitch sub-pulley formed adjacent to the end tool jaw pitch main pulley and formed to be rotatable around a fifth shaft different from the first shaft.

9. The surgical instrument of claim 8, wherein, during the pitch rotation of the end tool, the two strands of the jaw wire, which emerge while being wound around the end tool jaw pulley and pass through the end tool jaw pitch main pulley and the end tool jaw pitch sub-pulley, simultaneously move in the same direction.

10. The surgical instrument of claim 8, wherein, with respect to one plane perpendicular to the first shaft and comprising the fourth shaft, the two strands of the jaw wire, which emerge while being wound around the end tool jaw pulley, are arranged on the same side with respect to the one plane.

11. The surgical instrument of claim 8, wherein the jaw comprises a first jaw
and a second jaw,
the end tool jaw pulley comprises an end tool first jaw pulley coupled to the first jaw, and an end tool second jaw pulley coupled to the second jaw, and
the jaw wire comprises a first jaw wire coupled to the end tool first jaw pulley and a second jaw wire coupled to the end tool second jaw pulley.

12. The surgical instrument of claim 11, wherein, with respect to a plane perpendicular to the first shaft and comprising the fourth shaft, two strands of the first jaw wire, which emerge while being wound around the end tool first jaw pulley, are arranged on one side with respect to the plane, and two strands of the second jaw wire, which emerge while being wound around the end tool second jaw pulley, are arranged on another side with respect to the plane.

13. The surgical instrument of claim 8, wherein the jaw wire is formed to be sequentially in contact with the end tool jaw pulley, the end tool jaw pitch main pulley, and the end tool jaw pitch sub-pulley.

14. The surgical instrument of claim 8, further comprising:
an end tool pitch pulley arranged adjacent to the end tool jaw pulley and formed to be rotatable around the fourth shaft or the fifth shaft; and
a pitch wire coupled to each of the end tool pitch pulley and the driving part pitch pulley to connect the end tool pitch pulley to the driving part pitch pulley.

15. The surgical instrument of claim 14, wherein a rotation amount of the driving part pitch pulley and a rotation amount of the end tool pitch pulley are substantially equal to each other.

16. The surgical instrument of claim 14, wherein, when the driving part pitch pulley rotates by a first angle, the driving part jaw rotation shaft revolves by the first angle, and
when the driving part pitch pulley rotates by the first angle, the end tool pitch pulley and the end tool jaw pitch main pulley rotate by a second angle.

17. The surgical instrument of claim 2, further comprising at least one driving part sub-relay pulley that is arranged adjacent to the driving part jaw pulley and transfers the jaw wire from the end tool to the driving part jaw pulley, wherein the jaw wire sequentially passes through the driving part sub-relay pulley, the driving part jaw pulley, and the driving part jaw rotation shaft.

18. The surgical instrument of claim 17, wherein, when the driving part pitch pulley rotates, the driving part jaw rotation shaft rotates together such that a path length of the jaw wire, from an entry point to the driving part sub-relay pulley, through the driving part jaw pulley, and to an exit point from the driving part jaw rotation shaft, is changed.

19. The surgical instrument of claim 17, wherein, when the driving part pitch pulley rotates, a path length of the jaw wire, from a point at which the jaw wire first contacts the driving part jaw pulley, to a point at which the jaw wire last contacts the driving part jaw pulley, on an arrangement path of the jaw wire connecting the end tool jaw pulley to the driving part jaw rotation shaft, is changed.

20. The surgical instrument of claim 2, wherein the driving part jaw pulley is formed to be rotatable around the second shaft, and
the driving part jaw rotation shaft is formed to be revolvable around the second shaft.

21. The surgical instrument of claim 20, wherein, when the driving part pitch pulley rotates around the second shaft, the driving part jaw rotation shaft connected to the driving part pitch pulley revolves around the second shaft, such that the overall length of the jaw wire within the driving part is changed.

22. The surgical instrument of claim 20, wherein, when the driving part pitch pulley rotates around the second shaft, the driving part jaw rotation shaft rotates around the second shaft while maintaining a certain distance from the second shaft, in a state in which the driving part jaw rotation shaft is spaced apart from the second shaft by a certain extent.

23. The surgical instrument of claim 20, further comprising a base plate formed to rotate together with the driving part pitch pulley around the second shaft,
wherein the driving part jaw rotation shaft is formed on the base plate.

24. The surgical instrument of claim 23, wherein two or more holes are formed on the base plate, and
the driving part jaw rotation shaft is arranged in at least one of the holes.

25. The surgical instrument of claim 23, wherein the base plate rigidly connects the driving part pitch pulley to the driving part jaw rotation shaft, such that, when the driving part pitch pulley rotates around the second shaft, the driving part jaw rotation shaft revolves around the second shaft.

26. The articulated surgical device of claim 20, wherein, when the driving part jaw rotation shaft rotates around the second shaft, a length of the jaw wire by which the jaw wire is wound around the driving part jaw pulley is changed.

27. The surgical instrument of claim 1, wherein, even when the driving part jaw pulley rotates, the overall length of the jaw wire within the driving part is kept constant.

28. The surgical instrument of claim 1, wherein the jaw wire is combined with each of the end tool jaw pulley and the driving part jaw pulley to form a closed loop as a whole.

29. The surgical instrument of claim 1, wherein the yaw rotation is a motion in which the end tool jaw pulley rotates around the first shaft, and
the pitch rotation is a motion in which the end tool jaw pulley revolves around a fourth shaft different from the first shaft.

30. A surgical robot comprising:
one or more robotic arm units configured to perform a motion by handle manipulation by an operator; and
a surgical instrument coupled to the robotic arm unit,
wherein the surgical instrument comprises:
an end tool comprising one or more jaws and an end tool jaw pulley, which is coupled to the jaw, and formed to be rotatable together with the jaw around a first shaft, the end tool being formed to be able to perform at least pitch rotation and yaw rotation;
a jaw wire that is coupled to the end tool jaw pulley and moves according to rotation of the end tool jaw pulley;
a connection part extending in one direction, through which the jaw wire passes, and having one end to which the end tool is coupled; and
a driving part that is coupled to another end of the connection part and is configured to control the pitch rotation and the yaw rotation of the end tool,
wherein
the driving part comprises:
a driving part jaw pulley that is formed to be rotatable around a second shaft, and is formed to wind at least a portion of the jaw wire; and
a driving part jaw rotation shaft that is formed to be rotatable around a third shaft different from the second shaft, is relatively movable while maintaining a preset distance from a center of the driving part jaw pulley, revolves around the center of the driving part jaw pulley, and is coupled to the jaw wire,
the jaw wire moves through rotation of the driving part jaw rotation shaft to cause the end tool jaw pulley and the jaw to rotate,
two strands of the jaw wire, which emerge while being wound around the driving part jaw pulley, extend toward the end tool after being sequentially wound around the driving part jaw pulley, the driving part jaw rotation shaft, and the driving part jaw pulley, and
when the driving part jaw rotation shaft moves relative to the driving part jaw pulley, the end tool performs the pitch rotation while an overall length of the jaw wire within the driving part is changed.
